# EUROPEAN PATENT SPECIFICATION

(11) **EP 1 280 821 B1**
(45) Date of publication and mention of the grant of the patent: **18.10.2006**
(21) Application number: 01924404.5
(22) Date of filing: 28.03.2001
(51) Int. Cl.: C07K 14/47, C07K 14/705, C12N 5/10, C12N 15/63, C12N 15/64, G01N 33/50

(54) **DNA MOLECULES ENCODING LIGAND GATED ION CHANNELS FROM DERMACENTOR VARIABILIS**
LIGANDENGESTEUERTE IONENKANÄLE AUS DERMACENTOR VARIABILIS CODIERENDE DNS-MOLEKÜLE
MOLECULES D'ADN CODANT POUR DES CANAUX IONIQUES SENSIBLES A DES LIGANDS ISSUS DE DERMACENTOR VARIABILIS

(30) Priority: 31.03.2000 US 193935 P
(43) Date of publication of application: 05.02.2003
(73) Proprietor: Merial Limited, Duluth, GA 30096 (US)
(72) Inventor: CULLY, Doris, F., Rahway, NJ 07065-0907 (US); ZHENG, Yingcong, Rahway, NJ 07065-0907 (US)
(74) Representative: Harding, Charles Thomas
(86) International application number: PCT/US2001/009956
(87) International publication number: WO 2001/074899

(56) References cited:
- WO-A-01/74884
- WO-A-99/07828
- NIKOLIC Z ET AL: "The human glycine receptor subunit alpha3/GLRA3 GENE STRUCTURE,CHROMOSOMAL LOCALIZATION,AND FUNCTIONAL CHARACTERIZATION OF ALTERNATIVE TRANSCRIPTS*" JOURNAL OF BIOLOGICAL CHEMISTRY, AMERICAN SOCIETY OF BIOLOGICAL CHEMISTS, BALTIMORE, MD, US, [Online] vol. 273, no. 31, 31 July 1998 (1998-07-31), pages 19708-19714, XP002954893 ISSN: 0021-9258 -& DATABASE EMBL [Online] 15 December 1998 (1998-12-15) Database accession no. O75311 XP002274508
- KUHSE J ET AL: "IDENTIFICATION AND FUNCTIONAL EXPRESSION OF A NOVEL LIGAND BINDING SUBUNIT OF THE INHIBITORY GLYCINE RECEPTOR" JOURNAL OF BIOLOGICAL CHEMISTRY, [Online] vol. 265, no. 36, 1990, pages 22317-22320, XP002274507 ISSN: 0021-9258 -& DATABASE EMBL [Online] 1 March 1992 (1992-03-01) Database accession no. P24524 XP002274509
- DATABASE EMBL [Online] 1 November 1998 (1998-11-01) SEMENOV, E.P. & PAK, W.L.: "Diversification of Drosophila chloride channel gene by multiple posttranscriptoinal mRNA modifications" retrieved from EBML, accession no. Uniprot Database accession no. O77295 XP002274510
- CULLY D F ET AL: "IDENTIFICATION OF A DROSOPHILA MELANOGASTER GLUTAMATE-GATED CHLORIDE CHANNEL SENSITIVE TO THE ANTIPARASITIC AGENT AVERMECTIN" JOURNAL OF BIOLOGICAL CHEMISTRY, AMERICAN SOCIETY OF BIOLOGICAL CHEMISTS, BALTIMORE, MD, US, vol. 271, no. 33, 16 August 1996 (1996-08-16), pages 20187-20191, XP002911645 ISSN: 0021-9258
- ABBOTT ET AL.: 'MiRP1 forms IKr potassium channels with HERG and is associated with cardiac arrhythmia' CELL vol. 97, December 1999, pages 175 - 187, XP002944234

## Description

### FIELD OF THE INVENTION

The present invention relates in part to isolated nucleic acid molecules (polynucleotides) which encode *Dermacentor variabilis* (American dog tick) ligand-gated chloride channels. The present invention also relates to recombinant vectors and recombinant hosts which contain a DNA fragment encoding *D. variabilis* ligand-gated chloride channels, substantially purified forms of associated *D. variabilis* ligand-gated chloride channels and recombinant membrane fractions comprising these proteins, associated mutant proteins, and methods associated with identifying compounds which modulate associated *Dermacentor variabilis* ligand-gated chloride channels, which will be useful as insecticides and acaricides.

### BACKGROUND OF THE INVENTION

Glutamate-gated chloride channels, or H-receptors, have been identified in arthropod nerve and muscle (Lingle et al, 1981, *Brain Res.* 212: 481-488; Horseman et al., 1988, *Neurosci. Lett.* 85: 65-70; Wafford and Sattelle, 1989, *J. Exp. Bio.* 144: 449-462; Lea and Usherwood, 1973, *Comp. Gen. Parmacol.* 4: 333-350; and Cull-Candy, 1976, *J. Physiol.* 255: 449-464).

Invertebrate glutamate-gated chloride channels are important targets for the widely used avermectin class of anthelmintic and insecticidal compounds. The avermectins are a family of macrocyclic lactones originally isolated from the actinomycete *Streptomyces avermitilis.* The semisynthetic avermectin derivative, ivermectin (22,23-dihydro-avermectin B₁ₐ), is used throughout the world to treat parasitic helminths and insect pests of man and animals. The avermectins remain the most potent broad spectrum endectocides exhibiting low toxicity to the host. After many years of use in the field, there remains little resistance to avermectin in the insect population. The combination of good therapeutic index and low resistance strongly suggests that the ligand-gated ion channels, and especially glutamate-gated chloride (LGIC/GluCl) channels remain good targets for insecticide development.

Glutamate-gated chloride channels have been cloned from the soil nematode *Caenorhabditis elegans* (Cully et al., 1994, *Nature* 371: 707-711; see also U.S. Patent No. 5,527,703 and Arena et al., 1992, *Molecular Brain Research.* 15: 339-348) and *Ctenocephalides felis* (flea; see WO 99/07828).

In addition, a gene encoding a glutamate-gated chloride channel from *Drosophila melanogaster* was previously identified (Cully et al., 1996, *J. Biol. Chem.* 271: 20187-20191; see also U.S. Patent No. 5,693,492).

*Dermacentor variabilis* (American dog tick) is indegenous to the majority of the U.S. with known common hosts of livestock, deer, dogs, humans and small mammals. This tick is associated with various diseases, including Rocky Mountain spotted fever, babesiosis, tick paralysis, anaplasmosis, tularemia and cytauxzoonosis.

Despite the identification of the aforementioned cDNA clones encoding non-tick LGIC/GluCl channels, it would be advantageous to identify additional genes which encode *D. variabilis* LGIC/GluCl channels in order to allow for improved screening to identify novel LGIC/GluCl channel modulators that may have insecticidal, acaricidal, and/or nematocidal activity for animal health, especially as related to treatment of tick infestations in livestock and domesticated animals, such as dogs and cats. The present invention addresses and meets these needs by disclosing novel genes which encode *D. variabilis* LGIC/GluCl proteins and when expressed in *Xenopus* oocytes result in formation of functional LGIC/GluCl channels. Heterologous expression of a LGIC/GluCl channel of the present invention will allow the pharmacological analysis of compounds active against parasitic invertebrate species relevant to animal and human health, especially in the treatment of tick infestations directly related to *Dermacentor variabilis.* Heterologous cell lines expressing an active LGIC/GluCl channel can be used to establish functional or binding assays to identify novel LGIC/GluCl channel modulators that may be useful in control of the aforementioned species groups.

### SUMMARY OF THE INVENTION

The present invention relates to an isolated or purified nucleic acid molecule (polynucleotide) which encodes a novel *Dermacentor variabilis* (American dog tick) invertebrate LGIC channel protein, including but not necessarily limited to a *D. variabilis* LGIC/GluCl channel protein. The DNA molecules disclosed herein may be transfected into a host cell of choice wherein the transfeted host cell provides a source for substantial levels of an expressed functional single, homomultimer or heteromultimer LGIC. Such functional ligand-gated ion channels may possibly respond to other known ligands which will in turn provide for additional screening targets to identify modulators of these channels, modulators which may act as effective insecticidal, acaricidal, mitacidal and/or nematocidal treatments for use in animal and human health and/or crop protection.

The present invention further relates to an isolated nucleic acid molecule (polynucleotide) which encodes mRNA which expresses a novel *Dermacentor variabilis* LGIC/GluCl channel protein, this DNA molecule comprising the nucleotide sequence disclosed herein as SEQ ID NO:1, SEQ ID NO:3, SEQ ID NO:4 and SEQ ID NO:6.

The present invention also relates to biologically active fragments or mutants of SEQ ID NOs:1, 3, 4 and 6 which encodes mRNA expressing a novel *Dennacentor variabilis* invertebrate LGIC/GluCl channel protein. Any such biologically active fragment and/or mutant will encode either a protein or protein fragment which at least substantially mimics the pharmacological properties of a *D. variabilis* LGIC/GluCl channel protein, including but not limited to the *D. variabilis* LGIC/GluCl channel proteins as set forth in SEQ ID NO:2, SEQ ID NO:5 and SEQ ID NO:7. Any such polynucleotide includes but is not necessarily limited to nucleotide substitutions, deletions, additions, amino-terminal truncations and carboxy-terminal truncations such that these mutations encode mRNA which express a functional *D. variabilis* LGIC/GluCl channel in a eukaryotic cell, such as *Xenopus* oocytes, so as to be useful for screening for agonists and/or antagonists of *D. variabilis* LGIC/GluCl activity.

A preferred aspect of this portion of the present invention is disclosed in Figure 1 (SEQ ID NO:1; designated DvLGIC/GluCl 1), Figure 3 (SEQ ID NO:3; designated DvLGIC/GluCl 11), Figure 4 (SEQ ID NO:4; designated DvLGIC/GluCl 7-1) and Figure 6 (SEQ ID NO:6, designated DvLGIC/GluCl 10-2) which encode novel forms of *Dermacentor variabilis* LGIC/GluCl channel proteins.

The isolated nucleic acid molecules of the present invention may include a deoxyribonucleic acid molecule (DNA), such as genomic DNA and complementary DNA (cDNA), which may be single (coding or noncoding strand) or double stranded, as well as synthetic DNA, such as a synthesized, single stranded polynucleotide. The isolated nucleic acid molecule of the present invention may also include a ribonucleic acid molecule (RNA).

The present invention also relates to recombinant vectors and recombinant host cells, both prokaryotic and eukaryotic, which contain the substantially purified nucleic acid molecules disclosed throughout this specification.

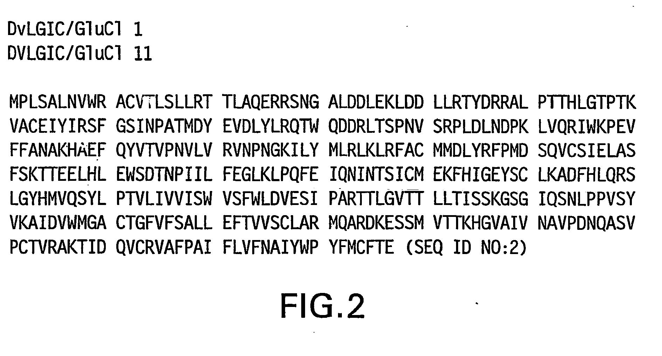

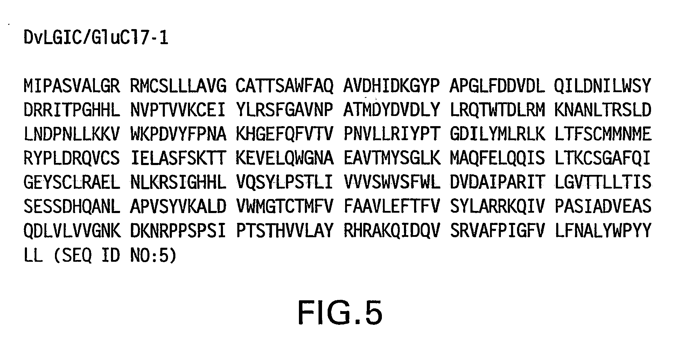

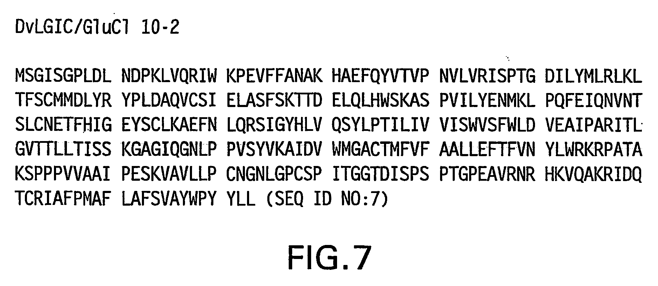

The present invention also relates in part to a substantially purified form of a *D. variabilis* LGIC/GluCl channel protein, which comprises the amino acid sequence disclosed in Figure 2 (SEQ ID NO:2), Figure 5 (SEQ ID NO:5) and Figure 7 (SEQ ID NO:7).

A preferred aspect of this portion of the present invention is a *D. variabilis* LGIC/GluCl channel protein which consists of the amino acid sequence disclosed in Figure 2 (SEQ ID NO:2), Figure 5 (SEQ ID NO:5) and Figure 7 (SEQ ID NO:7).

Another preferred aspect of the present invention relates to a substantially purified, fully processed (including any proteolytic processing, glycosylation and/or phosphorylation) mature LGIC/GluCl channel protein obtained from a recombinant host cell containing a DNA expression vector comprising a nucleotide sequence as set forth in SEQ ID NOs: 1, 3, 4 and/or 6 and expresses the DvLGIC/GluCl precursor or mature form of the respective protein. It is especially preferred that the recombinant host cell be a eukaryotic host cell, including but not limited to a mammalian cell line, an insect cell line such as S2 cells, or *Xenopus* oocytes.

Another preferred aspect of the present invention relates to a substantially purified membrane preparation, partially purified membrane preparations or cell lysate which has been obtained from a recombinant host cell transformed or transfected with a DNA expression vector which comprises and appropriately expresses a complete open reading frame as set forth in SEQ ID NOs: 1, 3, 4 and/or 6, resulting in a functional form of the respective DvLGIC/GluCl channel. The subcellular membrane fractions and/or membrane-containing cell lysates from the recombinant host cells (both prokaryotic and eukaryotic as well as both stably and transiently transformed/transfected cells) contain the functional and processed proteins encoded by the nucleic acids of the present invention. This recombinant-based membrane preparation may comprise a *D. variabilis* LGIC/GluCl channel and is essentially free from contaminating proteins, including but not limited to other *D. variabilis* source proteins or host proteins from a recombinant cell which expresses the LGIC/GluCl 1 (SEQ ID NO:2), LGIC/GluCl 11 (also SEQ ID NO:2) LGIC/GluCl 7-1 (SEQ ID NO:5) and/or the LGIC/GluCl 10-2 (SEQ ID NO:7) LGIC/GluCl channel protein. Therefore, a preferred aspect of the invention is a membrane preparation which contains a *D*. *variabilis* LGIC/GluCl channel comprising a LGIC/GluCl protein comprising the functional form of the LGIC/GluCl channel proteins as disclosed in Figure 2 (SEQ ID NO:2; LGIC/GluCl 1 and LGIC/GluCl 11), Figure 5 (SEQ ID NO:5, LGIC/GluCl 7-1) and/or Figure 7 (SEQ ID NO:7; LGIC/GluCl 10-2). These subcellular membrane fractions will comprise either wild-type or mutant variations which are biologically functional forms of the *D. variabilis* LGIC/GluCl channels. Any functional single channel, homomultimer or heteromultimer combination of the DvLGIC/GluCl proteins disclosed herein is contemplated at levels substantially above endogenous levels and hence will be useful in various assays described throughout this specification. It is also possible that the disclosed channel proteins may, alone or in combination, form functional heteromultimeric channels with as yet identified channel proteins. A preferred eukaryotic host cell of choice to express the glutamate-gated channels of the present invention is a mammalian cell line, an insect-based cell line such as S2 cells, or *Xenopus* oocytes.

The present invention also relates to biologically active fragments and/or mutants of a *D. variabilis* LGIC/GluCl channel protein, comprising the amino acid sequence as set forth in SEQ ID NOs:2, 5, and/or 7, including but not necessarily limited to amino acid substitutions, deletions, additions, amino terminal truncations and carboxy-terminal truncations such that these mutations provide for proteins or protein fragments of diagnostic, therapeutic or prophylactic use and would be useful for screening for selective modulators, including but not limited to agonists and/or antagonists for *D. variabilis* LGIC/GluCl channel pharmacology.

A prefened aspect of the present invention is disclosed in Figure 2 (SEQ ID NO:2), Figure 5 (SEQ ID NO:5) and Figure 7 (SEQ ID NO:7), amino acid sequences which comprise the *D. variabilis* LGIC/GluCl proteins of the present invention, respectively. Characterization of one or more of these channel proteins allows for screening methods to identify novel LGIC/GluCl channel modulators that may have insecticidal, acaricidal and/or nematocidal activity for animal health, human health and/or crop protection. As noted above, heterologous expression of a functional single channel, homomultimeric or heteromultimeric channel which is comprised of one or a combination of the DvLGIC/GluCl proteins disclosed herein is comtemplated at levels substantially above endogenous levels and will allow the pharmacological analysis of compounds active against parasitic invertebrate species relevant to animal and human health in general as well as possible DvLGIC/GluCl specific modulators which, may be useful to control various parasitic infestations. Heterologous cell lines expressing a functional DvLGIC/GluCl channel (e.g., functional forms of SEQ ID NOs:2, 5, and/or 7) can be used to establish functional or binding assays to identify novel LGIC/GluCl channel modulators that may be useful in control of the aforementioned species groups.

The present invention also relates to polyclonal and monoclonal antibodies raised in response to the disclosed forms of DvLGIC/GluCl, or a biologically active fragment thereof.

The present invention also relates to DvLGIC/GluCl fusion constructs, including but not limited to fusion constructs which express a portion of the DvLGIC/GluCl linked to various markers, including but in no way limited to GFP (Green fluorescent protein), the MYC epitope, GST, and Fc. Any such fusion constructs may be expressed in the cell line of interest and used to screen for modulators of one or more of the DvLGIC/GluCl proteins disclosed herein.

The present invention relates to methods of expressing *D. variabilis* LGIC/GluCl channel proteins and biological equivalents disclosed herein, assays employing these gene products, recombinant host cells which comprise DNA constructs which express these proteins, and compounds identified through these assays which act as agonists or antagonists of LGIC/GluCl channel activity.

It is an object of the present invention to provide an isolated nucleic acid molecule (e.g., SEQ ID NOs:1, 3, 4, and 6) which encodes a novel form of *D. variabilis* LGIC/GluCl, or fragments, mutants or derivatives of DvLGIC/GluCl, these proteins as set forth in SEQ ID NOs:2, 5 and 7, respectively. Any such polynucleotide includes but is not necessarily limited to nucleotide substitutions, deletions, additions, amino-terminal truncations and carboxy-terminal truncations such that these mutations encode mRNA which express a protein or protein fragment of diagnostic, therapeutic or prophylactic use and would be useful for screening for selective modulators for invertebrate LGIC/GluCl pharmacology.

It is a further object of the present invention to provide the *D. variabilis* LGIC/GluCl proteins or protein fragments encoded by the nucleic acid molecules referred to in the preceding paragraph.

It is a further object of the present invention to provide recombinant vectors and recombinant host cells which comprise a nucleic acid sequence encoding *D. variabilis* LGIC/GluCl proteins or a biological equivalent thereof.

It is an object of the present invention to provide a substantially purified form of *D. variabilis* LGIC/GluCl proteins, respectively, as set forth in SEQ ID NOs:2, 5, and 7.

It is another object of the present invention to provide a substantially purified recombinant form of a *D. variabilis* LGIC/GluCl protein which has been obtained from a recombinant host cell transformed or transfected with a DNA expression vector which comprises and appropriately expresses a complete open reading frame as set forth in SEQ ID NOs: 1, 3, 4, and 6, resulting in a functional form of the respective DyLGIC/GluCl channel. It is especially preferred that the recombinant host cell be a eukaryotic host cell, such as a mammalian cell line.

It is an object of the present invention to provide for biologically active fragments and/or mutants of *D. variabilis* LGIC/GluCl proteins, respectively, such as set forth in SEQ ID NOs: 2, 5, and 7, including but not necessarily limited to amino acid substitutions, deletions, additions, amino terminal truncations and carboxy-terminal truncations such that these mutations provide for proteins or protein fragments of diagnostic, therapeutic and/or prophylactic use.

It is further an object of the present invention to provide for substantially purified subcellular membrane preparations, partially purified subcellular membrane preparations, or crude lysates from recombinant cells which comprise pharmacologically active *D. variabilis* LGIC/GluCl channels, respectively, especially subcellular fractions obtained from a host cell transfected or transformed with a DNA vector comprising a nucleotide sequence which encodes a protein which comprises the amino acid as set forth in Figure 2 (SEQ ID NO:2), Figure 5 (SEQ ID NO:5), and/or Figure 7 (SEQ ID NO:7).

It is another object of the present invention to provide a substantially purified membrane preparation, partially purified subcellular membrane preparations, or crude lysates obtained from a recombinant host cell transformed or transfected with a DNA expression vector which comprises and appropriately expresses a complete open reading frame as set forth in SEQ ID NOs: 1, 3, 4, and/or 6, resulting in a functional, processed form of the respective DvLGIC/GluCl channel. It is especially preferred is that the recombinant host cell be a eukaryotic host cell, including but not limited to a mammalian cell line, an insect cell line such as S2 cells, or *Xenopus* oocytes.

It is also an object of the present invention to use *D. variabilis* LGIC/GluCl proteins or membrane preparations containing *D. variabilis* LGIC/GluCl proteins or a biological equivalent to screen for modulators, preferably selective modulators of *D. variabilis* LGIC/GluCl channel activity and/or an invertebrate LGIC/GluCl channel. Any such protein or membrane associated protein may be useful in screening for and selecting these modulators active against parasitic invertebrate species relevant to animal and human health. Such species include, in addition to the American dog tick channels disclosed herein, worms, fleas, other tick species, and lice. These membrane preparations may be generated from heterologous cell lines expressing these LGIC/GluCls and may constitute full length protein, biologically active fragments of the full length protein or may rely on fusion proteins expressed from various fusion constructs which may be constructed with materials available in the art.

As used herein, "substantially free from other nucleic acids" means at least 90%, preferably 95%, more preferably 99%, and even more preferably 99.9%, free of other nucleic acids. As used interchangeably with the terms "substantially free from other nucleic acids" or "substantially purified" or "isolated nucleic acid" or "purified nucleic acid" also refer to a DNA molecules which comprises a coding region for a *D. variabilis* LGIC/GluCl protein that has been purified away from other cellular components. Thus, a *D. variabilis* LGIC/GluCl DNA preparation that is substantially free from other nucleic acids will contain, as a percent of its total nucleic acid, no more than 10%, preferably no more than 5%, more preferably no more than 1%, and even more preferably no more than 0.1%, of non-*D*. *variabilis* LGIC/GluCl nucleic acids. Whether a given *D. variabilis* LGIC/GluCl DNA preparation is substantially free from other nucleic acids can be determined by such conventional techniques of assessing nucleic acid purity as, e.g., agarose gel electrophoresis combined with appropriate staining methods, e.g., ethidium bromide staining, or by sequencing.

As used herein, "substantially free from other proteins" or "substantially purified" means at least 90%, preferably 95%, more preferably 99%, and even more preferably 99.9%, free of other proteins. Thus, a *D. variabilis* LGIC/GluCl protein preparation that is substantially free from other proteins will contain, as a percent of its total protein, no more than 10%, preferably no more than 5%, more preferably no more than 1%, and even more preferably no more than 0.1 %, of non-*D. variabilis* LGIC/GluCl proteins. Whether a given *D. variabilis* LGIC/GluCl protein preparation is substantially free from other proteins can be determined by such conventional techniques of assessing protein purity as, e.g., sodium dodecyl sulfate polyacrylamide gel electrophoresis (SDS-PAGE) combined with appropriate detection methods, e.g., silver staining or immunoblotting. As used interchangeably with the terms "substantially free from other proteins" or "substantially purified", the terms "isolated *D. variabilis* LGIC/GluCl protein" or "purified *D. variabilis* LGIC/GluCl protein" also refer to *D. variabilis* LGIC/GluCl protein that has been isolated from a natural source. Use of the term "isolated" or "purified" indicates that *D. variabilis* LGIC/GluCl protein has been removed from its normal cellular environment. Thus, an isolated *D. variabilis* LGIC/GluCl protein may be in a cell-free solution or placed in a different cellular environment from that in which it occurs naturally. The term isolated does not imply that an isolated *D. variabilis* LGIC/GluCl protein is the only protein present, but instead means that an isolated *D. variabilis* LGIC/GluCl protein is substantially free of other proteins and non-amino acid material (e.g., nucleic acids, lipids, carbohydrates) naturally associated with the *D. variabilis* LGIC/GluCl protein *in vivo.* Thus, a *D*. *variabilis* LGIC/GluCl protein that is recombinantly expressed in a prokaryotic or eukaryotic cell and substantially purified from this host cell which does not naturally (*i.e.*, without intervention) express this LGIC/GluCl protein is of course "isolated *D. variabilis* LGIC/GluCl protein" under any circumstances referred to herein. As noted above, a *D*. *variabilis* LGIC/GluCl protein preparation that is an isolated or purified *D. variabilis* LGIC/GluCl protein will be substantially free from other proteins will contain, as a percent of its total protein, no more than 10%, preferably no more than 5%, more preferably no more than 1%, and even more preferably no more than 0.1%, of non-*D. variabilis* LGIC/GluCl proteins.

As used interchangeably herein, "functional equivalent" or "biologically active equivalent" means a protein which does not have exactly the same amino acid sequence as naturally occurring *D. variabilis* LGIC/GluCl, due to alternative splicing, deletions, mutations, substitutions, or additions, but retains substantially the same biological activity as *D. variabilis* LGIC/GluCl. Such functional equivalents will have significant amino acid sequence identity with naturally occurring *D. variabilis* LGIC/GluCl and genes and cDNA encoding such functional equivalents can be detected by reduced stringency hybridization with a DNA sequence encoding naturally occurring *D. variabilis* LGIC/GluCl. For example, a naturally occurring *D. variabilis* LGIC/GluCl protein disclosed herein comprises the amino acid sequence shown as SEQ ID NO:2 and is encoded by SEQ ID NO:1. A nucleic acid encoding a functional equivalent has at least about 50% identity at the nucleotide level to SEQ ID NO:1.

As used herein, "a conservative amino acid substitution" refers to the replacement of one amino acid residue by another, chemically similar, amino acid residue. Examples of such conservative substitutions are: substitution of one hydrophobic residue (isoleucine, leucine, valine, or methionine) for another; substitution of one polar residue for another polar residue of the same charge (e.g., arginine for lysine; glutamic acid for aspartic acid).

As used herein, "LGIC" refers to a --ligand-gated ion channel --.

As used herein, "GluCl" refers to -- L-glutamate gated chloride channel--.

As used herein, "LGIC/GluCl" refers to -- ligand gated ion channel /L-glutamate gated chloride channel --.

As used herein, "DvLGIC/GluCl" refers to -- *Dennacentor variabilis* ligand gated channel / L-glutamate gated chloride channel -.

As used herein, the term "mammalian" will refer to any mammal, including a human being.

### BRIEF DESCRIPTION OF THE DRAWINGS

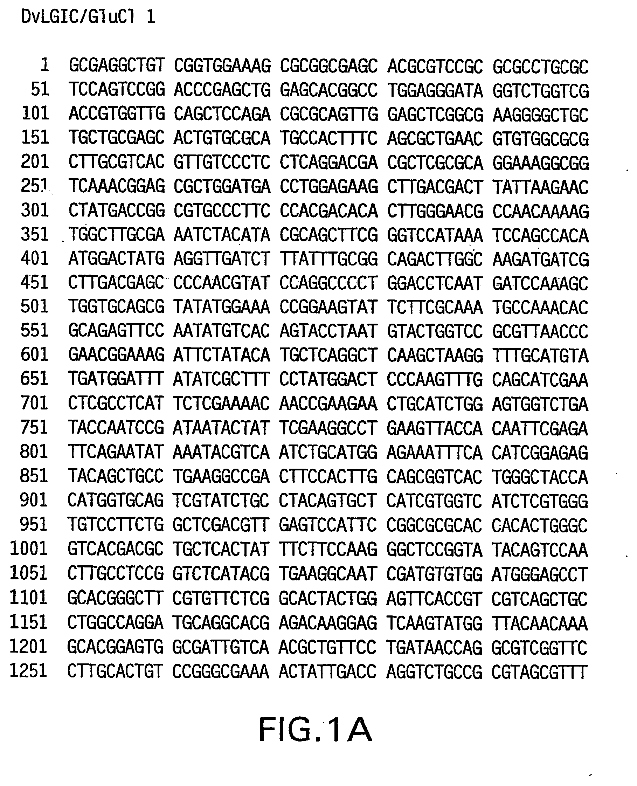

Figure 1A-C shows the nucleotide sequence of the *D. variabilis* LGIC/GluCl cDNA clone, DvLOIC/GluCl 1, set forth in SEQ ID NO:1.
Figure 2 shows the amino acid sequence of the *D. variabilis* LGIC/GluCl protein, DvLGIC/GluCl1 and DvLGIC/GluCl 11, as set forth in SEQ ID NO:2.

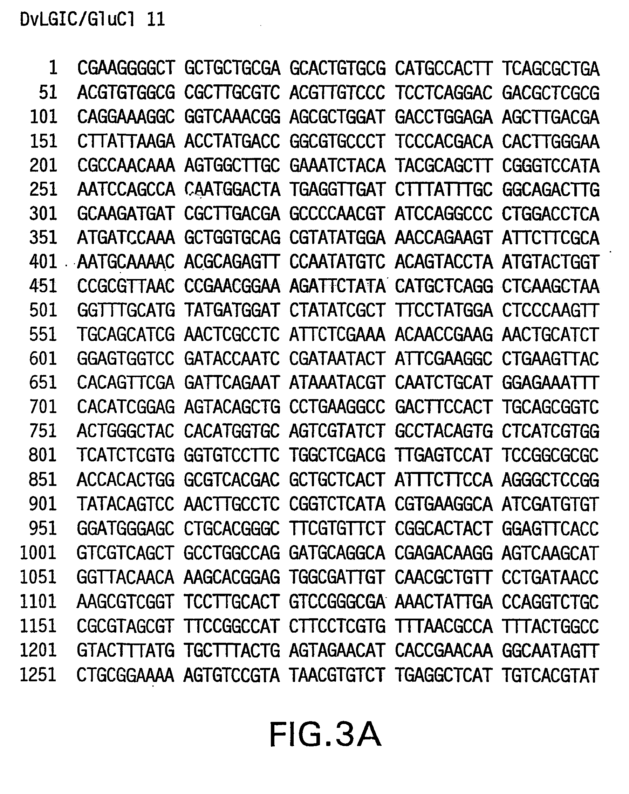

Figure 3A-C shows the nucleotide sequence of the *D. variabilis* LGIC/GluCl cDNA clone, DvLGIC/GluCl 11, as set forth in SEQ ID NO:3.

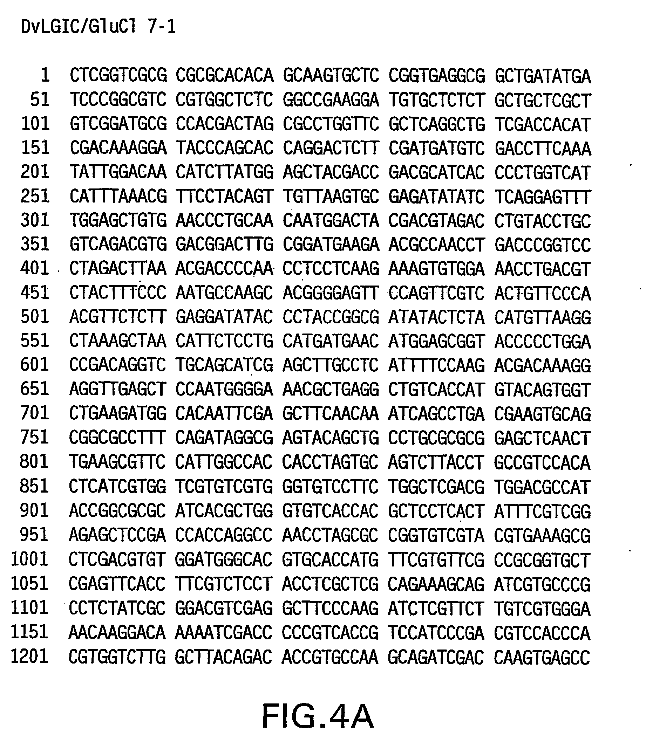

Figure 4A-B shows the nucleotide sequence of the *D. variabilis* LGIC/GluCl cDNA clone, DvLGIC/GluCl 7-1, as set forth in SEQ ID NO:4.
Figure 5 shows the amino acid sequence of the *D. variabilis* LGIC/GluCl protein, DvLGIC/GluCl 7-1, as set forth in SEQ ID NO:5.

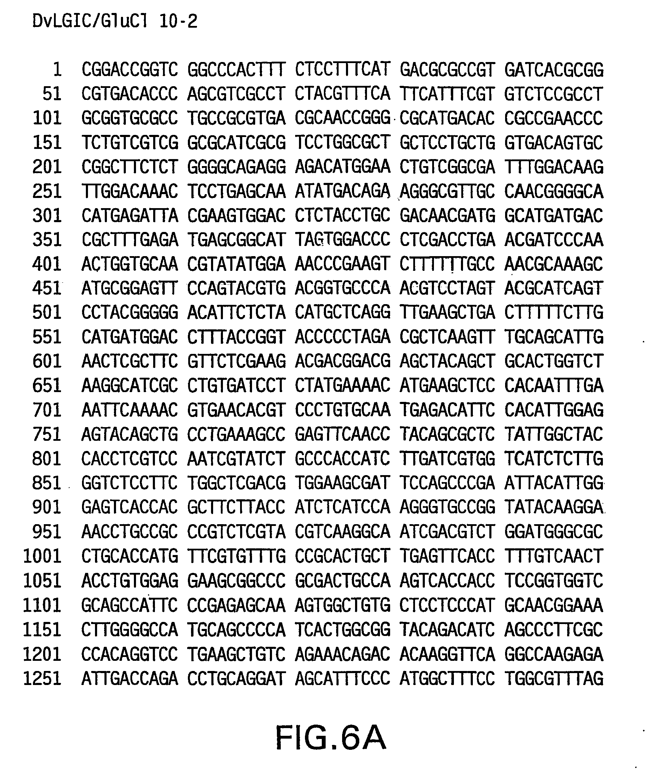

Figure 6A-C shows the nucleotide sequence of the *D. variabilis* LGIC/GluCl cDNA clone, DvLGIC/GluCl 10-2, as set forth in SEQ ID NO:6.
Figure 7 shows the amino acid sequence of the *D. variabilis* LGIC/GluCl protein, DvLGIC/GluCl 10-2, as set forth in SEQ ID NO;7.

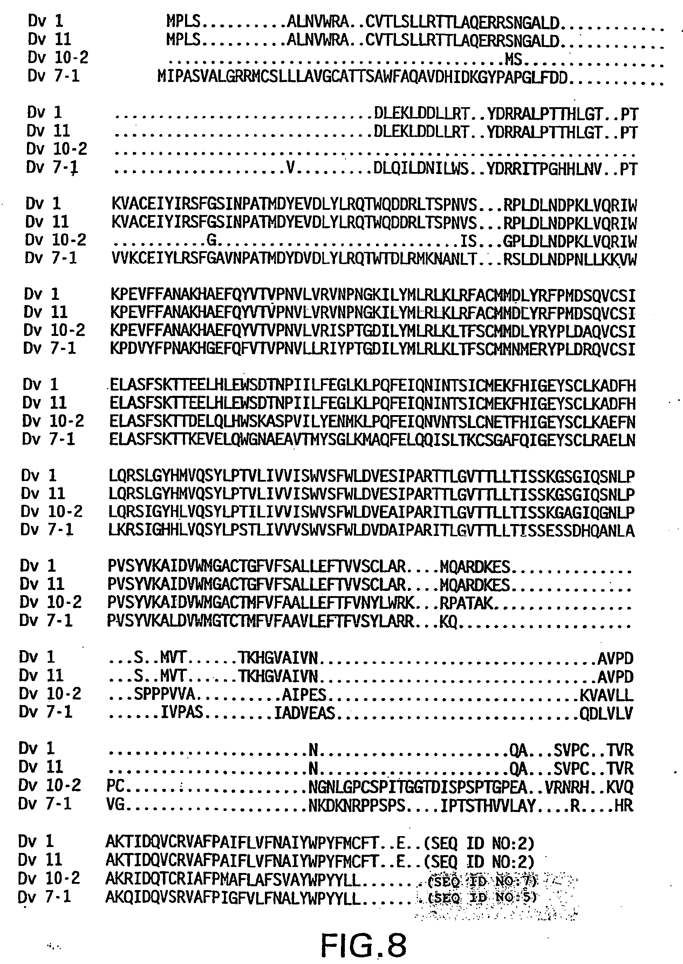

Figure 8 shows the amino acid sequence comparison for DvLGrC/GluCl 1 (SEQ ID: NO:2 , DvLGIC/GluCl 11 (SEQ ID NO:2), DvLGIC/GluCl 7-1 (SEQ ID NO:5) and DvLGIC/GluCl 10-2 (SEQ ID NO:7) proteins.

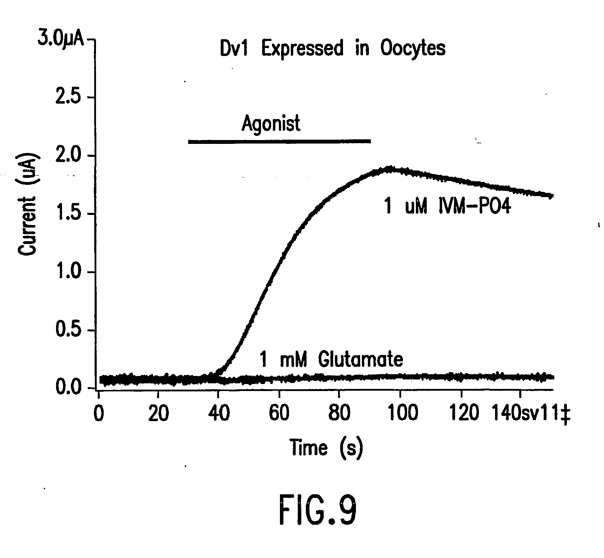

Figure 9 shows current activation in *Xenopus* oocytes injected with DvLGIC/GluCl 1 mRNA. Current activation was maximal with 1µM ivermectin-phosphate.

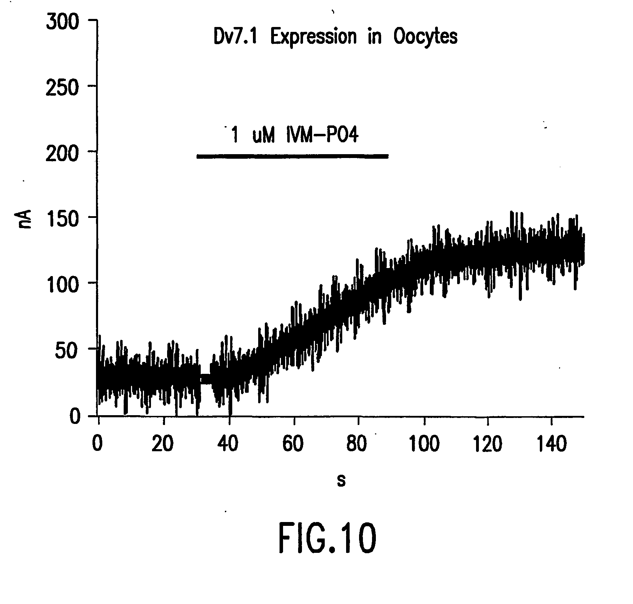

Figure 10 shows activation by ivermectin with DvLGIC/GluCl 7-1 expressed in *Xenopus* oocytes. Current activation was maximal with -1 µM ivermectin phosphate.

### DETAILED DESCRIPTION OF THE INVENTION

The present invention relates to an isolated nucleic acid molecule (polynucleotide) which encodes a *Dermacentor variabilis* invertebrate LGIC/GluCl channel protein. The isolated or purified nucleic acid molecules of the present invention are substantially free from other nucleic acids. For most cloning purposes, DNA is a preferred nucleic acid. As noted above, the DNA molecules disclosed herein may be transfected into a host cell of choice wherein the recombinant host cell provides a source for substantial levels of an expressed functional single, homomultimeric or heteromultimeric LGIC. Such functional ligand-gated ion channels may possibly respond to other known ligands which will in turn provide for additional screening targets to identify modulators of these channels, modulators which may act as effective insecticidal, mitacidal and/or nematocidal treatment for use in animal and human health and/or crop protection. It is shown herein that DvLGIC/GluCl 1,11 and 7-1 expressed in *Xenopus* oocytes exhibit a current in response to the addition of ivermectin phosphate. In contrast, DvLGIC/GluC1 10-2 was not responsive to ivermectin phosphate or glutamate. However, it should be noted that the GABA-A subunit gamma does not express a functional homomultimer. Therefore, the expressed proteins of the present invention may function *in vivo* as a component of a wild type ligand-gated ion channel which contains a number of accessory and/or channel proteins, including the channel proteins disclosed herein. However, the LGIC proteins of the present invention need not directly mimic the wild type channel in order to be useful to the skilled artisan. Instead, the ability to form a functional, single, membrane associated channel within a recombinant host cell renders these proteins amenable to the screening methodology known in the art and described in part within this specification. Therefore, as noted within this specification, the disclosed Dv channel proteins of the present invention are useful as single functional channels, as a homomultimeric channel or as a heteromultimeric channel with various proteins disclosed herein with or without additional Dv channel subunit proteins or accessory proteins which may contribute to the full, functional LGIC channel.

The present invention relates to an isolated nucleic acid molecule (polynucleotide) which encodes mRNA which expresses a novel *Dermacentor variabilis* invertebrate LGIC/GluCl channel protein, this DNA molecule comprising the nucleotide sequence disclosed herein as SEQ ID NO:1, SEQ ID NO:3, SEQ ID NO:4, and SEQ ID NO:6.

The isolation and characterization of the DvLGIC/GluCl nucleic acid molecules of the present invention were identified as described in detail in Example Section 1. These cDNA molecules, as discussed herein, are especially useful to establish novel insecticide screens, validate potential lead compounds with insecticidal activity, especially for use in treating parasite infestations in human and animals, such as livestock, dogs and cats or that may kill other arachnids. These cDNAs, or portions thereof, are also useful as hybridization probes to isolate related genes from other organisms to establish additional pesticide drug screens. The DvLGIC/GluCl encoding cDNAs of the present invention were isolated from the American dog tick species *Dermacentor variabilis.* The DNA sequence predicts proteins that share common features with the class of chloride channels sensitive to glutamate and ivermectin. When the DvLGIC/GluCl cDNAs are expressed in *Xenopus* oocytes, a glutamate and/or ivermectin-sensitive channel is observed. The pharmacology of compounds that act at these channels would likely be different between these species. By screening on the arachnid channel it will be more likely to discover arachnid-specific compounds. Therefore, the cDNAs of the present invention can be expressed in cell lines or other expression systems and used for competition binding experiments or for functional chloride channel assays to screen for compounds that activate, block or modulate the channel.

Invertebrate glutamate-gated chloride channels (LGIC/GluCls) are related to the glycine- and GABA-gated chloride channels and are distinct from the excitatory glutamate receptors (e.g. NMDA or AMPA receptors). The first two members of the LGIC/GluCl family were identified in the nematode *C*. *elegans,* following a functional screen for the receptor of the anthelmintic drug ivermectin. Several additional LGIC/GluCls have now been cloned in other invertebrate species. However, there is no evidence yet for LGIC/GluCl counterparts in vertebrates; because of this, LGIC/GluCls are excellent targets for anthelmintics, insecticides, acaricides, etc. Specific LGIC/GluCl modulators, such as nodulisporic acid and its derivatives have an ideal safety profile because they lack mechanism-based toxicity in vertebrates. The present invention relates in part to four novel *D. variabilis* LGIC/GluCl clones, DvLGIC/GluCl1, DvLGIC/GluCl11, DvLGIC/GluCl 7-1 and DvLGIC/GluCl 10-2. The DvLGIC/GluCl cDNAs were isolated from a *Dermacentor* tick cDNA library. Two hybridization probes were utilized. First a probe from the tick *Rhipicephalus sanguineus* GluCl (RsGluCl) gene was PCR amplified to generate a fragment that runs from nucleotide 448 through 1645 of the RsGluCl 1 open reading frame. A second probe from the tick *Rhipicephalus sanguineus* GluC12 clone (RsGluC12) gene was PCR amplified to generate a fragment that runs from nucleotide 166 through 1315 of the Rs GluC12 open reading frame. Eight positive clones, including DvLGIC/GluCl1, DvLGIC/GluCl11, DvLGIC/GluC17-1 and DvLGIC/GluCl 10-2 were identified in the original screen. DvLGIC/GluCl1, DvLGIC/GluCl 11, and DvLGIC/GluCl 7-1 were dentified by both probes while DvLGIC/GluCl 10-2 was recognized only by RsLGIC/GluC12 probe.

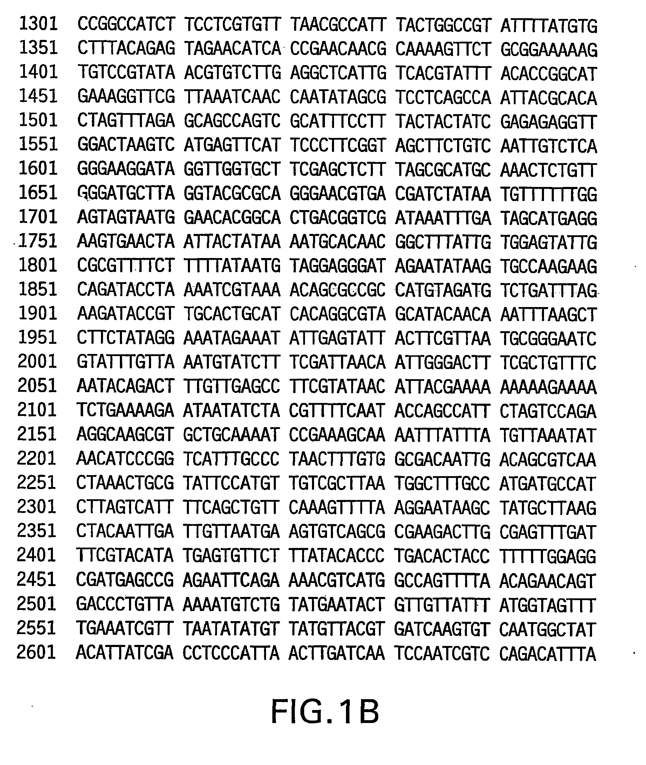

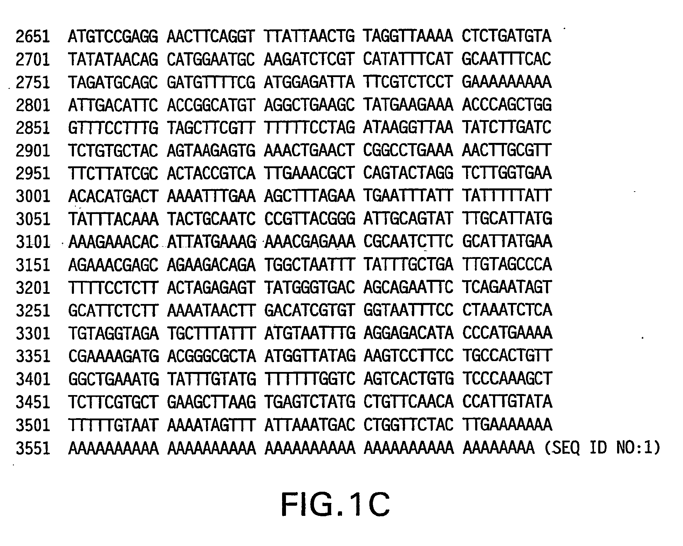

The present invention relates to the isolated or purified DNA molecule described in Figure 1 (DvLGIC/GluCl 1) and set forth as SEQ ID NO:1, which encodes the *D. variabilis* LGIC/GluCl protein described in Figure 2 and set forth as SEQ ID NO:2, the nucleotide sequence of DvLGIC/GluCl 1 is as follows:

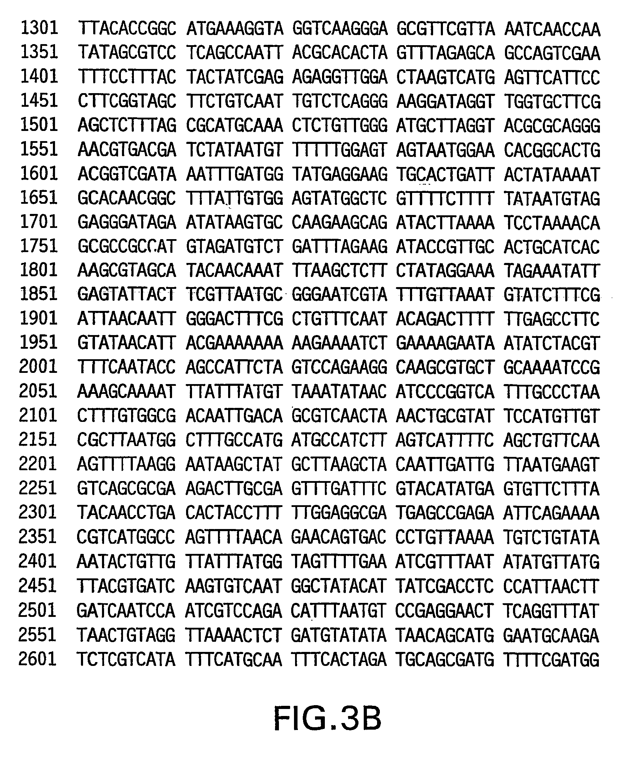

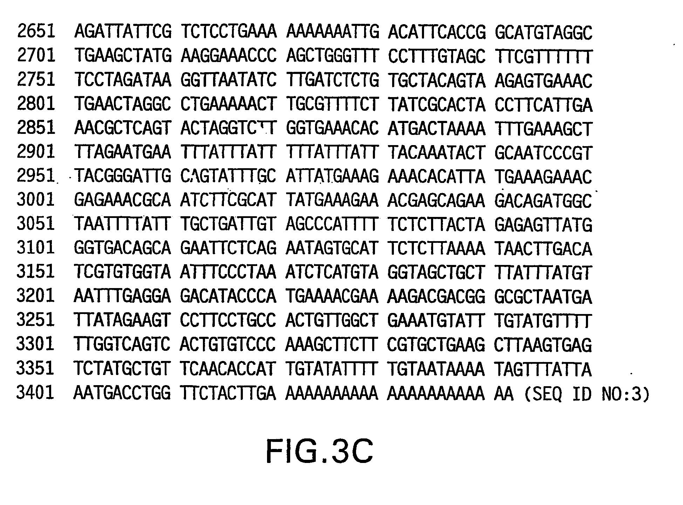

The present invention also relates to the isolated or purified DNA molecule described in Figure 3 (DvLGIC/GluCl 11) and set forth as SEQ ID NO:3, which encodes the *D. variabilis* LGIC/GluCl protein described in Figure 2 and set forth as SEQ ID NO:2, the nucleotide sequence of DvLGIC/GluCl is as follows:

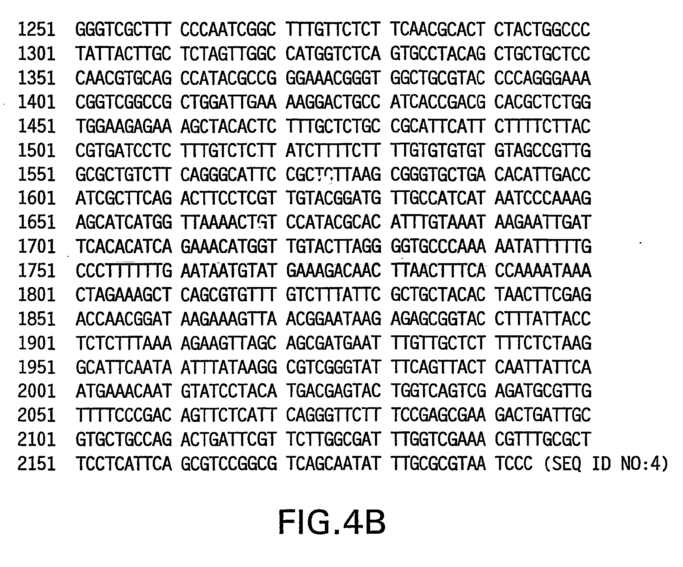

The present invention also relates to the isolated or purified DNA molecule described in Figure 4 (DvLGIC/GluCl 7-1) and set forth as SEQ ID NO:4, which encodes the *D. variabilis* LGIC/GluCl protein described in Figure 5 and set forth as SEQ ID NO:5, the nucleotide sequence of DvLGIC/GluCl 7-1 is as follows:

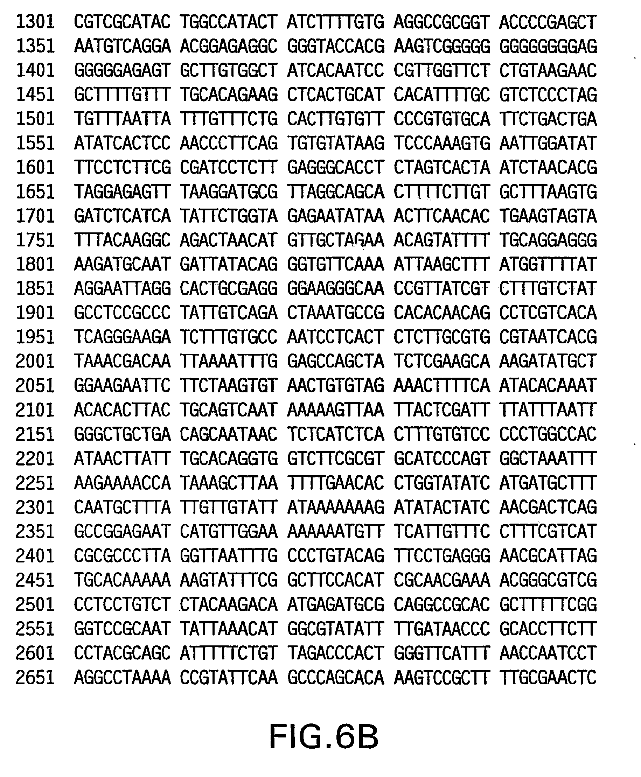

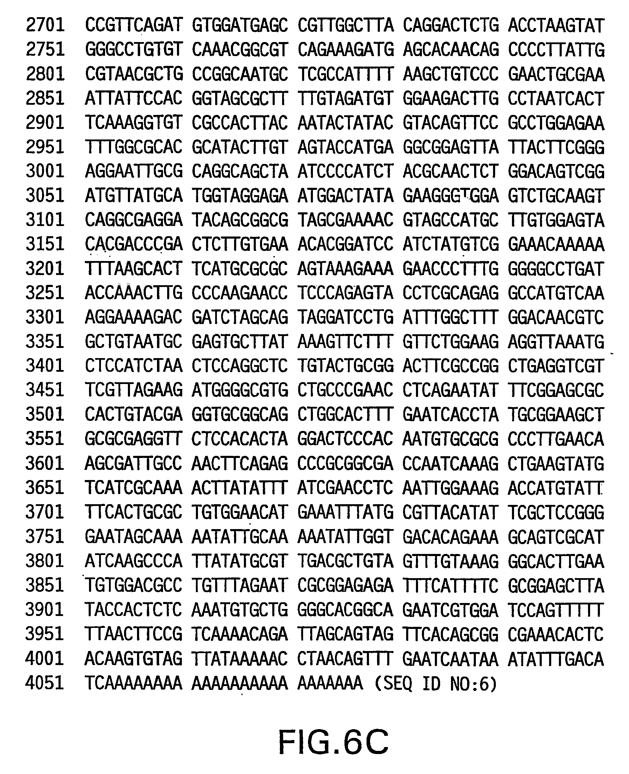

The present invention also relates to an isolated or purified DNA molecule described in Figure 6 (DvLGIC/GluCl 10-2) and set forth as SEQ ID NO:6, which encodes the *D. variabilis* LGIC/GluCl protein described in Figure 7 and set forth as SEQ ID NO:7, the nucleotide sequence of DvLGIC/GluCl 10-2 is as follows:

The above-exemplified isolated DNA molecules, shown in Figure 1, 3 4, and 6, respectively, comprise the following characteristics:
DvLGIC/GluCl 1 (SEQ ID NO:1):
   3598 nuc.:initiating Met (nuc. 170-172) and "TAG" term. codon (nuc.1361-1363), the open reading frame resulting in an expressed protein of 397 amino acids, as set forth in SEQ ID NO:2.
DvLGIC/GluCl 11 (SEQ ID NO:3):
   3442 nuc.:initiating Met (nuc. 32-34) and "TAG" term. codon (nuc.1223-1225), the open reading frame resulting in an expressed protein of 397 amino acids, as set forth in SEQ ID NO:2. The DvLGIC/GluCl 11 protein, as with DvLGIC/GluCl 1, comprises the amino acid sequence as set forth in SEQ ID NO:2. The nucleotide sequences within the open reading frame of SEQ ID NO:3 and SEQ ID NO: 1 show 9 nucleotide substitutions. Three of the substitutions are A-G changes possibly resulting from RNA editing events, while the remainder of changes most likely are a result of allelic differences within the tick population.
DvLGIC/GluCl 7-1 (SEQ ID NO:4):
   2194 nuc.:initiating Met (nuc. 47-49) and "TGA" term. codon (nuc. 1313-1315), the open reading frame resulting in an expressed protein of 422 amino acids, as set forth in SEQ ID NO:5.
DvLGIC/GluCl 10-2 (SEQ ID NO:6):
   4177 nuc.:initiating Met (nuc. 360-362) and "TGA" term. codon (nuc. 1329-1331), the open reading frame resulting in an expressed protein of 323 amino acids, as set forth in SEQ ID NO:7.

The percent identity at the nucleotide level for various exemplified cDNA molecules of the present invention were generated using the GCG-Best fit-Smith and Waterman algorithm. Comparative percent identities are shown below:
Drosophila LGIC/GluClα1 (US Pat. No.5,693,492) and DvLGIC/GluCl 1 - 54.869%;
Drosophila GluClα1 and DvLGIC/GluCl 7-1 - 58.029%;
Drosophila GluClα1 and DvLGIC/GluCl 10-2 - 54.938%;
DvLGIC/GluCl 1 and DvLGIC/GluCl 7-1-66.555%;
DvLGIC/GluCl 1 and DvLGIC/GluCl 10-2 - 75.000%;
DvLGIC/GluCl 1 and DvLGIC/GluCl 11 - 99.246%; and,
DvLGIC/GluCl 7-1 and DvLGIC/GluCl 10-2 - 69.103%.
To this end, the present invention relates a purified nucleic acid molecule encoding a *D. variabilis* LGIC/GluCl channel protein where the nucleic acid molecule comprises (a) a nucleic acid molecule which encodes an amino acid sequence selected from the group consisting of SEQ ID NOs 2, 5 and 7; or, (b) a nucleic acid molecule which hybridizes under conditions of moderate stringency to the complement of a second nucleic acid molecule which encodes SEQ ID NOs 2, 5 and 7; or, (c) a nucleic acid molecule which hybridizes under conditions of moderate to high stringency to the complement of a second nucleic acid molecule as set forth in SEQ ID NOs 1, 3, 4 and 6 and this nucleic acid molecule has at least about a 65% identity at the nucleotide level within the open reading frame to at least one of the second nucleic acid molecules as set forth in SEQ ID NOs 1, 3, 4 and 6.

The present invention also relates to biologically active fragments or mutants of SEQ ID NOs:1, 3, 4 and 6 which encodes mRNA expressing a novel *Dermacentor variabilis* invertebrate LGIC/GluCl channel protein, respectively. Any such biologically active fragment and/or mutant will encode either a protein or protein fragment which at least substantially mimics the pharmacological properties of a *D. variabilis* LGIC/GluC1 channel protein, including but not limited to the *D*. *variabilis* LGIC/GluCl channel proteins as set forth in SEQ ID NO:2, SEQ ID NO:5, and SEQ ID NO:7. Any such polynucleotide includes but is not necessarily limited to nucleotide substitutions, deletions, additions, amino-terminal truncations and carboxy-terminal truncations such that these mutations encode mRNA which express a functional *D. variabilis* LGIC/GluCl channel in a eukaryotic cell, such as *Xenopus* oocytes, so as to be useful for screening for agonists and/or antagonists of *D. variabilis* LGIC/GluCl activity.

A preferred aspect of this portion of the present invention is disclosed in Figure 1 (SEQ ID NO:1; designated DvLGIC/GluCl 1), Figure 3 (SEQ ID NO:3; designated DvLGIC/GluCl 11), Figure 4 (SEQ ID NO:4; designated DvLGIC/GluCl 7-1) and Figure 6 (SEQ ID NO:6, designated DvLGIC/GluCl 10-2) encoding a novel *Dermacentor variabilis* LGIC/GluCl protein.

The present invention also relates to isolated nucleic acid molecules which are fusion constructions expressing fusion proteins useful in assays to identify compounds which modulate wild-type DvLGIC/GluCl activity, as well as generating antibodies against DvLGIC/GluCl. One aspect of this portion of the invention includes, but is not limited to, glutathione S-transferase (GST)-DvLGIC/GluCl fusion constructs. Recombinant GST-DvLGIC/GluCl fusion proteins may be expressed in various expression systems, including *Spodoptera frugiperda* (Sf21) insect cells (Invitrogen) using a baculovirus expression vector (pAcG2T, Pharmingen). Another aspect involves DvLGIC/GluCl fusion constructs linked to various markers, including but not limited to GFP (Green fluorescent protein), the MYC epitope, and GST. Again, any such fusion constructs may be expressed in the cell line of interest and used to screen for modulators of one or more of the DvLGIC/GluCl proteins disclosed herein.

The isolated nucleic acid molecules of the present invention may include a deoxyribonucleic acid molecule (DNA), such as genomic DNA and complementary DNA (cDNA), which may be single (coding or noncoding strand) or double stranded, as well as synthetic DNA, such as a synthesized, single stranded polynucleotide. The isolated nucleic acid molecule of the present invention may also include a ribonucleic acid molecule (RNA).

The degeneracy of the genetic code is such that, for all but two amino acids, more than a single codon encodes a particular amino acid. This allows for the construction of synthetic DNA that encodes the DvLGIC/GluCl protein where the nucleotide sequence of the synthetic DNA differs significantly from the nucleotide sequence of SEQ ID NOs:1, 3, 4, and 6 but still encodes the same DvLGIC/GluCl protein as SEQ ID NO:1, 3, 4 and 6. Such synthetic DNAs are intended to be within the scope of the present invention. If it is desired to express such synthetic DNAs in a particular host cell or organism, the codon usage of such synthetic DNAs can be adjusted to reflect the codon usage of that particular host, thus leading to higher levels of expression of the DvLGIC/GluCl channel protein in the host. In other words, this redundancy in the various codons which code for specific amino acids is within the scope of the present invention. Therefore, this invention is also directed to those DNA sequences which encode RNA comprising alternative codons which code for the eventual translation of the identical amino acid, as shown below:
A=Ala=Alanine: codons GCA, GCC, GCG, GCU
C=Cys=Cysteine: codons UGC, UGU
D=Asp=Aspartic acid: codons GAC, GAU
E=Glu=Glutamic acid: codons GAA, GAG
F=Phe=Phenylalanine: codons UUC, UUU
G=Gly=Glycine: codons GGA, GGC, GGG, GGU
H=His =Histidine: codons CAC, CAU
I=Ile =Isoleucine: codons AUA, AUC, AUU
K=Lys=Lysine: codons AAA, AAG
L=Leu=Leucine: codons UUA, UUG, CUA, CUC, CUG, CUU
M=Met=Methionine: codon AUG
N=Asp=Asparagine: codons AAC, AAU
P=Pro=Proline: codons CCA, CCC, CCG, CCU
Q=Gln=Glutamine: codons CAA, CAG
R=Arg=Arginine: codons AGA, AGG, CGA, CGC, CGG, CGU
S=Ser=Serine: codons AGC, AGU, UCA, UCC, UCG, UCU
T=Thr=Threonine: codons ACA, ACC, ACG, ACU
V=Val=Valine: codons GUA, GUC, GUG, GUU
W=Trp=Tryptophan: codon UGG
Y=Tyr-Tyrosine: codons UAC, UAU
Therefore, the present invention discloses codon redundancy which may result in differing DNA molecules expressing an identical protein. For purposes of this specification, a sequence bearing one or more replaced codons will be defined as a degenerate variation. Another source of sequence variation may occur through RNA editing, as discussed *infra.* Such RNA editing may result in another form of codon redundancy, wherein a change in the open reading frame does not result in an altered amino acid residue in the expressed protein. Also included within the scope of this invention are mutations either in the DNA sequence or the translated protein which do not substantially alter the ultimate physical properties of the expressed protein. For example, substitution of valine for leucine, arginine for lysine, or asparagine for glutamine may not cause a change in functionality of the polypeptide.

It is known that DNA sequences coding for a peptide may be altered so as to code for a peptide having properties that are different than those of the naturally occurring peptide. Methods of altering the DNA sequences include but are not limited to site directed mutagenesis. Examples of altered properties include but are not limited to changes in the affinity of an enzyme for a substrate or a receptor for a ligand.

Included in the present invention are DNA sequences that hybridize to SEQ ID NOs:1, 3, 4 and 6 under moderate to highly stringent conditions. By way of example, and not limitation, a procedure using conditions of high stringency is as follows: Prehybridization of filters containing DNA is carried out for 2 hours to overnight at 65°C in buffer composed of 6X SSC, 5X Denhardt's solution, and 100 *µ*g/ml denatured salmon sperm DNA. Filters are hybridized for 12 to 48 hrs at 65°C in prehybridization mixture containing 100 *µ*g/ml denatured salmon sperm DNA and 5-20 X 10⁶ cpm of ³²P-labeled probe. Washing of filters is done at 37°C for 1 hr in a solution containing 2X SSC, 0.1% SDS. This is followed by a wash in 0.1X SSC, 0.1 % SDS at 50°C for 45 min. before autoradiography. Other procedures using conditions of high stringency would include either a hybridization step carried out in 5X SSC, 5X Denhardt's solution, 50% formamide at 42°C for 12 to 48 hours or a washing step carried out in 0.2X SSPE, 0.2% SDS at 65°C for 30 to 60 minutes. Reagents mentioned in the foregoing procedures for carrying out high stringency hybridization are well known in the art. Details of the composition of these reagents can be found in, e.g., Sambrook et al., 1989, *Molecular Cloning: A Laboratory Manual;* Cold Spring Harbor Laboratory, Cold Spring Harbor, New York. In addition to the foregoing, other conditions of high stringency which may be used are well known in the art.

"Identity" is a measure of the identity of nucleotide sequences or amino acid sequences. In general, the sequences are aligned so that the highest order match is obtained. "Identity" per se has an art-recognized meaning and can be calculated using published techniques. See, e.g.,: (Computational Molecular Biology, Lesk, A. M., ed. Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds.. Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). While there exists a number of methods to measure identity between two polynucleotide or polypeptide sequences, the term "identity" is well known to skilled artisans (Carillo and Lipton, 1988, *SIAM J Applied Math* 48:1073). Methods commonly employed to determine identity or similarity between two sequences include, but are not limited to, those disclosed in Guide to Huge Computers, Martin J. Bishop, ed., Academic Press, San Diego, 1994, and Carillo and Lipton, 1988, *SIAM J Applied Math* 48:1073. Methods to determine identity and similarity are codified in computer programs. Preferred computer program methods to determine identity and similarity between two sequences include, but are not limited to, GCG program package (Devereux, et al, 1984, *Nucleic Acids Research* 12(1):387), BLASTN, FASTA (Altschul, et al., 1990, *J Mol. Biol.* 215:403).

As an illustration, by a polynucleotide having a nucleotide sequence having at least, for example, 95% "identity" to a reference nucleotide sequence of SEQ ID NO:1 is intended that the nucleotide sequence of the polynucleotide is identical to the reference sequence except that the polynucleotide sequence may include up to five point mutations or alternative nucleotides per each 100 nucleotides of the reference nucleotide sequence of SEQ ID NO:1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations or alternative nucleotide substitutions of the reference sequence may occur at the 5' or 3' terminal positions of the reference nucleotide sequence or anywhere between those terminal positions, interspersed either individually among nucleotides in the reference sequence or in one or more contiguous groups within the reference sequence. One source of such a "mutation" or change which results in a less than 100% identity may occur through RNA editing. The process of RNA editing results in modification of an mRNA molecule such that use of that modified mRNA as a template to generate a cloned cDNA may result in one or more nucleotide changes, which may or may not result in a codon change. This RNA editing is known to be catalyzed by an RNA editase. Such an RNA editase is RNA adenosine deaminase, which converts an adenosine residue to an inosine residue, which tends to mimic a cytosine residue. To this end, conversion of an mRNA residue from A to I will result in A to G transitions in the coding and noncoding regions of a cloned cDNA (e.g., see Hanrahan et al, 1999, *Annals New York Acad. Sci.* 868: 51-66); for a review see Bass (1997, *TIBS* 22: 157-162).

Similarly, by a polypeptide having an amino acid sequence having at least, for example, 95% identity to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO:2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence of anywhere between those terminal positions, interspersed either individually among residues in the reference sequence or in one or more contiguous groups within the reference sequence. Again, as noted above, RNA editing may result in a codon change which will result in an expressed protein which differs in "identity" from other proteins expressed from "non-RNA edited" transcripts, which correspond directly to the open reading frame of the genomic sequence.

The present invention also relates to recombinant vectors and recombinant hosts, both prokaryotic and eukaryotic, which contain the substantially purified nucleic acid molecules disclosed throughout this specification. The nucleic acid molecules of the present invention encoding a DvLGIC/GluCl channel protein, in whole or in part, can be linked with other DNA molecules, i.e, DNA molecules to which the DvLGIC/GluCl coding sequence are not naturally linked, to form "recombinant DNA molecules" which encode a respective DvLGIC/GluCl channel protein. The novel DNA sequences of the present invention can be inserted into vectors which comprise nucleic acids encoding DvLGIC/GluCl or a functional equivalent. These vectors may be comprised of DNA or RNA; for most cloning purposes DNA vectors are preferred. Typical vectors include plasmids, modified viruses, bacteriophage, cosmids, yeast artificial chromosomes, and other forms of episomal or integrated DNA that can encode a DvLGIC/GluCl channel protein. It is well within the purview of the skilled artisan to determine an appropriate vector for a particular gene transfer or other use.

The present invention also relates to a substantially purified form of a respective DvLGIC/GluCl channel protein, which comprise the amino acid sequence disclosed in Figure 2, Figure 5 and Figure 7, and as set forth in SEQ ID NOs :2, 5, and 7, respectively. The disclosed DvLGIC/GluCl proteins contain an open reading frame of 397 amino acids (DvLGIC/GluCl 1 and DvLGIC/GluCl 11, SEQ ID NO:2), 422 amino acids (DvLGIC/GluCl 7-1, SEQ ID NO: 5) and 323 amino acids (DvLGIC/GluCl 10-2, SEQ ID NO:7) in length, as shown in Figures 2, 5, and 7, and as follows: and,

Figure 8 shows the amino acid sequence comparison for DvLGIC/GluCI 1 and 11 (SEQ ID NO:2), DvLGIC/GluCl 7-1 (SEQ ID NO:5) and DvLGIC/GluCl 10-2 (SEQ ID NO:7) proteins.

The present invention also relates to biologically active fragments and/or mutants of the DvLGIC/GluCl proteins comprising the amino acid sequence as set forth in SEQ ID NOs:2, 5, and 7, including but not necessarily limited to amino acid substitutions, deletions, additions, amino terminal truncations and carboxy-terminal truncations such that these mutations provide for proteins or protein fragments of diagnostic, therapeutic or prophylactic use and would be useful for screening for agonists and/or antagonists of DvLGIC/GluCl function.

Another preferred aspect of the present invention relates to a substantially purified, fully processed LGIC/GluCl channel protein obtained from a recombinant host cell containing a DNA expression vector comprises a nucleotide sequence as set forth in SEQ ID NOs: 1, 3, 4, and/or 6, and expresses the respective DvLGIC/GluCl precursor protein. It is especially preferred that the recombinant host cell be a eukaryotic host cell, including but not limited to a mammalian cell line, an insect cell line such as S2 cells, or *Xenopus* oocytes, as noted above.

As with many proteins, it is possible to modify many of the amino acids of DvLGIC/GluCl channel protein and still retain substantially the same biological activity as the wild type protein. Thus this invention includes modified DvLGIC/GluCl polypeptides which have amino acid deletions, additions, or substitutions but that still retain substantially the same biological activity as a respective, corresponding DvLGIC/GluCl. It is generally accepted that single amino acid substitutions do not usually alter the biological activity of a protein (see, e.g., *Molecular Biology of the Gene,* Watson *et al.,* 1987, Fourth Ed., The Benjamin/Cummings Publishing Co., Inc., page 226; and Cunningham & Wells, 1989, *Science* 244:1081-1085). Accordingly, the present invention includes polypeptides where one amino acid substitution has been made in SEQ ID NO:2, 5, and/or 7, wherein the polypeptides still retain substantially the same biological activity as a corresponding DvLGIC/GluCl protein. The present invention also includes polypeptides where two or more amino acid substitutions have been made in SEQ ID NO:2, 5, and.7, wherein the polypeptides still retain substantially the same biological activity as a corresponding DvLGIC/GluCl protein. In particular, the present invention includes embodiments where the above-described substitutions are conservative substitutions.

One skilled in the art would also recognize that polypeptides that are functional equivalents of DvLGIC/GluCl and have changes from the DvLGIC/GluCl amino acid sequence that are small deletions or insertions of amino acids could also be produced by following the same guidelines, (i.e, minimizing the differences in amino acid sequence between DvLGIC/GluCl and related proteins). Small deletions or insertions are generally in the range of about 1 to 5 amino acids. The effect of such small deletions or insertions on the biological activity of the modified DvLGIC/GluCl polypeptide can easily be assayed by producing the polypeptide synthetically or by making the required changes in DNA encoding DvLGIC/GluCl and then expressing the DNA recombinantly and assaying the protein produced by such recombinant expression.

The present invention also includes truncated forms of DvLGIC/GluCl which contain the region comprising the active site of the enzyme. Such truncated proteins are useful in various assays described herein, for crystallization studies, and for structure-activity-relationship studies.

The present invention also relates to membrane-containing crude lysates, partially purified or substantially purified subcellular membrane fractions from the recombinant host cells (both prokaryotic and eukaryotic as well as both stably and transiently transformed/transfected cells) which contain the nucleic acid molecules of the present invention. These recombinant host cells express DvLGIC/GluCl or a functional equivalent, which becomes post translationally associated with the cell membrane in a biologically active fashion. These subcellular membrane fractions will comprise either wild-type or mutant forms of DvLGIC/GluCl at levels substantially above endogenous levels and hence will be useful in assays to select modulators of DvLGIC/GluCl proteins or channels. In other words, a specific use for such subcellular membranes involves expression of DvLGIC/GluCl within the recombinant cell followed by isolation and substantial purification of the membranes away from other cellular components and subsequent use in assays to select for modulators, such as agonist or antagonists of the protein or biologically active channel comprising one or more of the proteins disclosed herein. Alternatively, the lysed cells, containing the membranes, may be used directly in assays to select for modulators of the recombinantly expressed protein(s) disclosed herein. Therefore, another preferred aspect of the present invention relates to a substantially purified membrane preparation or lysed recombinant cell components which include membranes, which has been obtained from a recombinant host cell transformed or transfected with a DNA expression vector which comprises and appropriately expresses a complete open reading frame as set forth in SEQ ID NOs: 1, 3, 4, and/or 6, resulting in a functional form of the respective DvLGIC/GluCl channel. It is especially preferred that the recombinant host cell be a eukaryotic host cell, including but not limited to a mammalian cell line such as an insect cell line such as S2 cells, or *Xenopus* oocytes, as noted above.

Any of a variety of procedures may be used to clone DvLGIC/GluCl. These methods include, but are not limited to, (1) a RACE PCR cloning technique (Frohman, et al., 1988, *Proc. Natl. Acad. Sci. USA* 85: 8998-9002). 5' and/or 3' RACE may be performed to generate a full-length cDNA sequence. This strategy involves using gene-specific oligonucleotide primers for PCR amplification of DvLGIC/GluCl cDNA. These gene-specific primers are designed through identification of an expressed sequence tag (EST) nucleotide sequence which has been identified by searching any number of publicly available nucleic acid and protein databases; (2) direct functional expression of the DvLGIC/GluCl cDNA following the construction of a DvLGIC/GluCl-containing cDNA library in an appropriate expression vector system; (3) screening a DvLGIC/GluCl-containing cDNA library constructed in a bacteriophage or plasmid shuttle vector with a labeled degenerate oligonucleotide probe designed from the amino acid sequence of the DvLGIC/GluCl protein; (4) screening a DvLGIC/GluCl-containing cDNA library constructed in a bacteriophage or plasmid shuttle vector with a partial cDNA encoding the DvLGIC/GluCl protein. This partial cDNA is obtained by the specific PCR amplification of DvLGIC/GluCl DNA fragments through the design of degenerate oligonucleotide primers from the amino acid sequence known for other ion channel subunits which are related to the DvLGIC/GluCl protein; (5) screening a DvLGIC/GluCl-containing cDNA library constructed in a bacteriophage or plasmid shuttle vector with a partial cDNA or oligonucleotide with homology to a DvLGIC/GluCl protein. This strategy may also involve using gene-specific oligonucleotide primers for PCR amplification of DvLGIC/GluCl cDNA identified as an EST as described above; or (6) designing 5' and 3' gene specific oligonucleotides using SEQ ID NO: 1, 3, 4 and/or 6 as a template so that either the full-length cDNA may be generated by known RACE techniques, or a portion of the coding region may be generated by these same known RACE techniques to generate and isolate a portion of the coding region to use as a probe to screen one of numerous types of cDNA and/or genomic libraries in order to isolate a full-length version of the nucleotide sequence encoding DvLGIC/GluCl. Alternatively, the DvLGIC/GluCl1 (1, 11 and 7-1) and DvLGIC/GluCl2 (10-2) cDNAs of the present invention may be cloned as described in Example Section 1.

It is readily apparent to those skilled in the art that other types of libraries, as well as libraries constructed from other cell types-or species types, may be useful for isolating a DvLGIC/GluCl-encoding DNA or a DvLGIC/GluCl homologue. Other types of libraries include, but are not limited to, cDNA libraries derived from other American dog tick cell types.

It is readily apparent to those skilled in the art that suitable cDNA libraries may be prepared from cells or cell lines which have DvLGIC/GluCl activity. The selection of cells or cell lines for use in preparing a cDNA library to isolate a cDNA encoding DvLGIC/GluCl may be done by first measuring cell-associated DvLGIC/GluCl activity using any known assay available for such a purpose.

Preparation of cDNA libraries can be performed by standard techniques well known in the art. Well known cDNA library construction techniques can be found for example, in Sambrook et al., 1989, *Molecular Cloning: A Laboratory Manual;* Cold Spring Harbor Laboratory, Cold Spring Harbor, New York. Complementary DNA libraries may also be obtained from numerous commercial sources, including but not limited to Clontech Laboratories, Inc. and Stratagene.

It is also readily apparent to those skilled in the art that DNA encoding DvLGIC/GluCl may also be isolated from a suitable genomic DNA library. Construction of genomic DNA libraries can be performed by standard techniques well known in the art. Well known genomic DNA library construction techniques can be found in Sambrook, et al., *supra.* One may prepare genomic libraries, especially in P1 artificial chromosome vectors, from which genomic clones containing the DvLGIC/GluCl can be isolated, using probes based upon the DvLGIC/GluCl nucleotide sequences disclosed herein. Methods of preparing such libraries are known in the art (Ioannou et al., 1994, *Nature Genet.* 6:84-89).

In order to clone a DvLGIC/GluCl gene by one of the preferred methods, the amino acid sequence or DNA sequence of a DvLGIC/GluCl or a homologous protein may be necessary. To accomplish this, a respective DvLGIC/GluCl channel protein may be purified and the partial amino acid sequence determined by automated sequenators. It is not necessary to determine the entire amino acid sequence, but the linear sequence of two regions of 6 to 8 amino acids can be determined for the PCR amplification of a partial DvLGIC/GluCl DNA fragment. Once suitable amino acid sequences have been identified, the DNA sequences capable of encoding them are synthesized. Because the genetic code is degenerate, more than one codon may be used to encode a particular amino acid, and therefore, the amino acid sequence can be encoded by any of a set of similar DNA oligonucleotides. Only one member of the set will be identical to the DvLGIC/GluCl sequence but others in the set will be capable of hybridizing to DvLGIC/GluCl DNA even in the presence of DNA oligonucleotides with mismatches. The mismatched DNA oligonucleotides may still sufficiently hybridize to the DvLGIC/GluCl DNA to permit identification and isolation of DvLGIC/GluCl encoding DNA. Alternatively, the nucleotide sequence of a region of an expressed sequence may be identified by searching one or more available genomic databases. Gene-specific primers may be used to perform PCR amplification of a cDNA of interest from either a cDNA library or a population of cDNAs. As noted above, the appropriate nucleotide sequence for use in a PCR-based method may be obtained from SEQ ID NO: 1, 3, 4, or 6 either for the purpose of isolating overlapping 5' and 3' RACE products for generation of a full-length sequence coding for DvLGIC/GluCl, or to isolate a portion of the nucleotide sequence coding for DvLGIC/GluCl for use as a probe to screen one or more cDNA- or genomic-based libraries to isolate a full-length sequence encoding DvLGIC/GluCl or DvLGIC/GluCl-like proteins.

This invention also includes vectors containing a DvLGIC/GluCl gene, host cells containing the vectors, and methods of making substantially pure DvLGIC/GluCl protein comprising the steps of introducing the DvLGIC/GluCl gene into a host cell, and cultivating the host cell under appropriate conditions such that DvLGIC/GluCl is produced. The DvLGIC/GluCl so produced may be harvested from the host cells in conventional ways. Therefore, the present invention also relates to methods of expressing the DvLGIC/GluCl protein and biological equivalents disclosed herein, assays employing these gene products, recombinant host cells which comprise DNA constructs which express these proteins, and compounds identified through these assays which act as agonists or antagonists of DvLGIC/GluCl activity.

The cloned DvLGIC/GluCl cDNA obtained through the methods described above may be recombinantly expressed by molecular cloning into an expression vector (such as pcDNA3.neo, pcDNA3.1, pCR2.1, pBlueBacHis2 or pLITMUS28, as well as other examples, listed *infra*) containing a suitable promoter and other appropriate transcription regulatory elements, and transferred into prokaryotic or eukaryotic host cells to produce recombinant DvLGIC/GluCl. Expression vectors are defined herein as DNA sequences that are required for the transcription of cloned DNA and the translation of their mRNAs in an appropriate host. Such vectors can be used to express eukaryotic DNA in a variety of hosts such as bacteria, blue green algae, plant cells, insect cells and mammalian cells (e.g., HEL human cells). Specifically designed vectors allow the shuttling of DNA between hosts such as bacteria-yeast or bacteria-mammalian cells. An appropriately constructed expression vector should contain: an origin of replication for autonomous replication in host cells, selectable markers, a limited number of useful restriction enzyme sites, a potential for high copy number, and active promoters. A promoter is defined as a DNA sequence that directs RNA polymerase to bind to DNA and initiate RNA synthesis. A strong promoter is one which causes mRNAs to be initiated at high frequency. To determine the DvLGIC/GluCl cDNA sequence(s) that yields optimal levels of DvLGIC/GluCl, cDNA molecules including but not limited to the following can be constructed: a cDNA fragment containing the full-length open reading frame for DvLGIC/GluCl as well as various constructs containing portions of the cDNA encoding only specific domains of the protein or rearranged domains of the protein. All constructs can be designed to contain none, all or portions of the 5' and/or 3' untranslated region of a DvLGIC/GluCl cDNA. The expression levels and activity of DvLGIC/GluCl can be determined following the introduction, both singly and in combination, of these constructs into appropriate host cells. Following determination of the DvLGIC/GluCl cDNA cassette yielding optimal expression in transient assays, this DvLGIC/GluCl cDNA construct is transferred to a variety of expression vectors (including recombinant viruses), including but not limited to those for mammalian cells, plant cells, insect cells, oocytes, bacteria, and yeast cells. Techniques for such manipulations can be found described in Sambrook, et al., *supra,* are well known and available to the artisan of ordinary skill in the art. Therefore, another aspect of the present invention includes host cells that have been engineered to contain and/or express DNA sequences encoding the DvLGIC/GluCl. An expression vector containing DNA encoding a DvLGIC/GluCl-like protein may be used for expression of DvLGIC/GluCl in a recombinant host cell. Such recombinant host cells can be cultured under suitable conditions to produce DvLGIC/GluCl or a biologically equivalent form. Expression vectors may include, but are not limited to, cloning vectors, modified cloning vectors, specifically designed plasmids or viruses. Commercially available mammalian expression vectors which may be suitable for recombinant DvLGIC/GluCl expression, include but are not limited to, pcDNA3.neo (Invitrogen), pcDNA3.1 (Invitrogen), pCI-neo (Promega), pLITMUS28, pLITMUS29, pLITMUS38 and pLITMUS39 (New England Bioloabs), pcDNAI, pcDNAIamp (Invitrogen), pcDNA3 (Invitrogen), pMClneo (Stratagene), pXT1 (Stratagene), pSG5 (Stratagene), EBO-pSV2-neo (ATCC 37593) pBPV-1(8-2) (ATCC 37110), pdBPV-MMTneo(342-12) (ATCC 37224), pRSVgpt (ATCC 37199), pRSVneo (ATCC 37198), pSV2-dhfr (ATCC 37146), pUCTag (ATCC 37460), and IZD35 (ATCC 37565). Also, a variety of bacterial expression vectors may be used to express recombinant DvLGIC/GluCl in bacterial cells. Commercially available bacterial expression vectors which may be suitable for recombinant DvLGIC/GluCl expression include, but are not limited to pCR2.1 (Invitrogen), pET11a (Novagen), lambda gt11 (Invitrogen), and pKK223-3 (Pharmacia). In addition, a variety of fungal cell expression vectors may be used to express recombinant DvLGIC/GluCl in fungal cells. Commercially available fungal cell expression vectors which may be suitable for recombinant DvLGIC/GluCl expression include but are not limited to pYES2 (Invitrogen) and *Pichia* expression vector (Invitrogen). Also, a variety of insect cell expression vectors may be used to express recombinant protein in insect cells. Commercially available insect cell expression vectors which may be suitable for recombinant expression of DvLGIC/GluCl include but are not limited to pBlueBacIII and pBlueBacHis2 (Invitrogen), and pAcG2T (Pharmingen).

Recombinant host cells may be prokaryotic or eukaryotic, including but not limited to, bacteria such as *E. coli,* fungal cells such as yeast, mammalian cells including, but not limited to, cell lines of bovine, porcine, monkey and rodent origin; and insect cells including but not limited to *D. variabilis* and silkworm derived cell lines. For instance, one insect expression system utilizes *Spodoptera frugiperda* (Sf21) insect cells (Invitrogen) in tandem with a baculovirus expression vector (pAcG2T, Pharmingen). Also, mammalian species which may be suitable and which are commercially available, include but are not limited to, L cells L-M(TK⁻) (ATCC CCL 1.3), L cells L-M (ATCC CCL 1.2), Saos-2 (ATCC HTB-85), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171) and CPAE (ATCC CCL 209).

A preferred aspect for screening for modulators of DvLGIC/GluCl channel activity is an expression system for electrophysiologically-based assays for measuring ligand gated channel activity (such as GluCl channel activity) comprising injecting the DNA or RNA molecules of the present invention into *Xenopus laevis* oocytes. The general use of *Xenopus* oocytes in the study of ion channel activity is known in the art (Dascal, 1987, *Crit. Rev. Biochem.* 22: 317-317; Lester, 1988, *Science* 241: 1057-1063; see also *Methods of Enzymology,* Vol. 207, 1992, Ch. 14-25, Rudy and Iverson, ed., Academic Press, Inc., New York). The *Xenopus* oocytes are injected with nucleic acid material, including but not limited to DNA, mRNA or cRNA which encode a ligand gated-channel, whereafter channel activity may be measured as well as response of the channel to various modulators.

The specificity of binding of compounds showing affinity for LGIC/GluCl is shown by measuring the affinity of the compounds for recombinant cells expressing the cloned receptor or for membranes from these cells, which form a functional homomultimeric or heteromultimeric channel. Expression of the cloned receptor and screening for compounds that bind to LGIC/GluCl or that inhibit the binding of a known ligand of LGIC/GluCl to these cells, or membranes prepared from these cells, provides an effective method for the rapid selection of compounds with high affinity for LGIC/GluCl.. Compounds identified by the above method are likely to be agonists or antagonists of LGIC/GluCl and may be peptides, proteins or non-proteinaceous organic or inorganic molecules.

Accordingly, the present invention is directed to methods for screening for compounds which modulate the expression of DNA or RNA encoding a LGIC/GluCl protein as well as compounds which effect the function of the LGIC/GluCl protein. Methods for identifying agonists and antagonists of other receptors are well known in the art and can be adapted to identify agonists and antagonists of a LGIC/GluCl channel. For example, Cascieri et al. (1992, *Molec. Pharmacol.* 41:1096-1099) describe a method for identifying substances that inhibit agonist binding to rat neurokinin receptors and thus are potential agonists or antagonists of neurokinin receptors. The method involves transfecting COS cells with expression vectors containing rat neurokinin receptors, allowing the transfected cells to grow for a time sufficient to allow the neurokinin receptors to be expressed, harvesting the transfected cells and resuspending the cells in assay buffer containing a known radioactively labeled agonist of the neurokinin receptors either in the presence or the absence of the substance, and then measuring the binding of the radioactively labeled known agonist of the neurokinin receptor to the neurokinin receptor. If the amount of binding of the known agonist is less in the presence of the substance than in the absence of the substance, then the substance is a potential ligand of the neurokinin receptor. Where binding of the substance such as an agonist or antagonist to LGIC/GluCl is measured, such binding can be measured by employing a labeled ligand. The ligand can be labeled in any convenient manner known to the art, e.g., radioactively, fluorescently, enzymatically.

Therefore, the present invention is directed to methods for screening for compounds which modulate the expression of DNA or RNA encoding a DvLGIC/GluCl protein. Compounds which modulate these activities may be DNA, RNA, peptides, proteins, or non-proteinaceous organic or inorganic molecules. Compounds may modulate by increasing or attenuating the expression of DNA or RNA encoding DvLGIC/GluCl, or the function of the DvLGIC/GluCl-based channels. Compounds that modulate the expression of DNA or RNA encoding DvLGIC/GluCl or the biological function thereof may be detected by a variety of assays. The assay may be a simple "yes/no" assay to determine whether there is a change in expression or function. The assay may be made quantitative by comparing the expression or function of a test sample with the levels of expression or function in a standard sample. Kits containing DvLGIC/GluCl, antibodies to DvLGIC/GluCl, or modified DvLGIC/GluCl may be prepared by known methods for such uses.

To this end, the present invention relates in part to methods of identifying a substance which modulates LGIC/GluCl receptor activity, which involves:
(a) adding a test substance in the presence and absence of a LGIC/GluCl receptor protein wherein said LGIC/GluCl receptor protein comprises the amino acid sequence as set forth in SEQ ID NOs: 2, 6 and/or 7; and,
(b) measuring and comparing the effect of the test substance in the presence and absence of the LGIC/GluCl receptor protein or respective functional channel.

In addition, several specific embodiments are disclosed herein to show the diverse types of screening or selection assays which the skilled artisan may utilize in tandem with an expression vector directing the expression of the LGIC/GluCl receptor protein. Methods for identifying ligands of other receptors are well known in the art and can be adapted to ligands of LGIC/GluCl. Therefore, these embodiments are presented as examples and not as limitations. To this end, the present invention includes assays by which LGIC/GluCl modulators (such as agonists and antagonists) may be identified. Accordingly, the present invention includes a method for determining whether a substance is a potential agonist or antagonist of LGIC/GluCl that comprises:
(a) transfecting or transforming cells with an expression vector that directs expression of LGIC/GluCl in the cells, resulting in test cells;
(b) allowing the test cells to grow for a time sufficient to allow LGIC/GluCl to be expressed and for a functional channel to be generated;
(c) exposing the cells to a labeled ligand of LGIC/GluCl in the presence and in the absence of the substance;
(d) measuring the binding of the labeled ligand to the LGIC/GluCl channel; where if the amount of binding of the labeled ligand is less in the presence of the substance than in the absence of the substance, then the substance is a potential ligand of LGIC/GluCl.

The conditions under which step (c) of the method is practiced are conditions that are typically used in the art for the study of protein-ligand interactions: e.g., physiological pH; salt conditions such as those represented by such commonly used buffers as PBS or in tissue culture media; a temperature of about 4°C to about 55°C. The test cells may be harvested and resuspended in the presence of the substance and the labeled ligand. In a modification of the above-described method, step (c) is modified in that the cells are not harvested and resuspended but rather the radioactively labeled known agonist and the substance are contacted with the cells while the cells are attached to a substratum, e.g., tissue culture plates.

The present invention also includes a method for determining whether a substance is capable of binding to LGIC/GluCl, i.e., whether the substance is a potential modulator of LGIC/GluCl channel activation, where the method comprises:
(a) transfecting or transforming cells with an expression vector that directs the expression of LGIC/GluCl in the cells, resulting in test cells;
(b) exposing the test cells to the substance;
(c) measuring the amount of binding of the substance to LGIC/GluCl;
(d) comparing the amount of binding of the substance to LGIC/GluCl in the test cells with the amount of binding of the substance to control cells that have not been transfected with LGIC/GluCl;
wherein if the amount of binding of the substance is greater in the test cells as compared to the control cells, the substance is capable of binding to LGIC/GluCl. Determining whether the substance is actually an agonist or antagonist can then be accomplished by the use of functional assays, such as an electrophysiological assay described herein.

The conditions under which step (b) of the method is practiced are conditions that are typically used in the art for the study of protein-ligand interactions: e.g., physiological pH; salt conditions such as those represented by such commonly used buffers as PBS or in tissue culture media; a temperature of about 4°C to about 55°C. The test cells are harvested and resuspended in the presence of the substance.

The above described assays may be functional assays, where electrophysiological assays (e.g., see Example 2) may be carried out in transfected mammalian cell lines, an insect cell line, or *Xenopus* oocytes to measure the various effects test compounds may have on the ability of a known ligand (such as glutamate) to activate the channel, or for a test compound to modulate activity in and of itself (similar to the effect of ivermectin on known GluCl channels). Therefore, the skilled artisan will be comfortable adapting the cDNA clones of the present invention to known methodology for both initial and secondary screens to select for compounds that bind and/or activate the functional LGIC/GluCl channels of the present invention.

A preferred method of identifying a modulator of a LGIC/GluCl channel protein comprise firstly contacting a test compound with a *D*. *variabilis* LGIC/GluCl-channel protein selected from the group consisting of SEQ ID NO:2, SEQ ID NO:5; and SEQ ID NO:7; and, secondly measuring the effect of the test compound on the LGIC/GluCl channel protein. A preferred aspect involves using a *D. variabilis* LGIC/GluCl protein which is a product of a DNA expression vector contained within a recombinant host cell.

Another preferred method of identifying a compound that modulates LGIC/GluCl glutamate-gated channel protein activity comprises firstly injecting into a host cell a population of nucleic acid molecules, at least a portion of which encodes a *D. variabilis.* GluCl channel protein selected from the group consisting of SEQ ID NO:2, SEQ ID NO:5, and SEQ ID NO:7, such that expression of said portion of nucleic acid molecules results in an active ligand-gated channel, secondly measuring host cell membrane current in the presence and absense of a test compound. Numerous templates may be used, including but not limited to complementary DNA, poly A⁺ messenger RNA and complementary RNA.

The DNA molecules, RNA molecules, recombinant protein and antibodies of the present invention may be used to screen and measure levels of DvLGIC/GluCl. The recombinant proteins, DNA molecules, RNA molecules and antibodies lend; themselves to the formulation of kits suitable for the detection and typing of DvLGIC/GluCl. Such a kit would comprise a compartmentalized carrier suitable to hold in close confinement at least one container. The carrier would further comprise reagents such as recombinant DvLGIC/GluCl or anti-DvLGIC/GluCl antibodies suitable for detecting DvLGIC/GluCl. The carrier may also contain a means for detection such as labeled antigen or enzyme substrates or the like.

The assays described herein can be carried out with cells that have been transiently or stably transfected with DvLGIC/GluCl. The expression vector may be introduced into host cells via any one of a number of techniques including but not limited to transformation, transfection, protoplast fusion, and electroporation. Transfection is meant to include any method known in the art for introducing DvLGIC/GluCl into the test cells. For example, transfection includes calcium phosphate or calcium chloride mediated transfection, lipofection, infection with a retroviral construct containing DvLGIC/GluCl, and electroporation. The expression vector-containing cells are individually analyzed to determine whether they produce DvLGIC/GluCl protein. Identification of DvLGIC/GluCl expressing cells may be done by several means, including but not limited to immunological reactivity with anti-DvLGIC/GluCl antibodies, labeled ligand binding, or the presence of functional, non-endogenous DvLGIC/GluCl activity.

The specificity of binding of compounds showing affinity for DvLGIC/GluCl is shown by measuring the affinity of the compounds for recombinant cells expressing the cloned receptor or for membranes from these cells. Expression of the cloned receptor and screening for compounds that bind to DvLGIC/GluCl or that inhibit the binding of a known, ligand of DvLGIC/GluCl to these cells, or membranes prepared from these cells, provides an effective method for the rapid selection of compounds with high affinity for DvLGIC/GluCl. Such ligands need not necessarily be radiolabeled but can also be nonisotopic compounds that can be used to displace bound radioactively, fluorescently or enzymatically labeled compounds or that can be used as activators in functional assays. Compounds identified by the above method are likely to be agonists or antagonists of DvLGIC/GluCl.

Therefore, the specificity of binding of compounds having affinity for DvLGIC/GluCl is shown by measuring the affinity of the compounds for recombinant cells expressing the cloned receptor or for membranes from these cells. Expression of the cloned receptor and screening for compounds that bind to DvLGIC/GluCl or that inhibit the binding of a known, radiolabeled ligand of DvLGIC/GluCl (such as glutamate, ivermectin or nodulisporic acid) to these cells, or membranes prepared from these cells, provides an effective method for the rapid selection of compounds with high affinity for DvLGIC/GluCl. Such ligands need not necessarily be radiolabeled but can also be nonisotopic compounds that can be used to displace bound radioactively, fluorescently or enzymatically labeled compounds or that can be used as activators in functional assays. Compounds identified by the above method again are likely to be agonists or antagonists of DvLGIC/GluCl. As noted elsewhere in this specification, compounds may modulate by increasing or attenuating the expression of DNA or RNA encoding DvLGIC/GluCl, or by acting as an agonist or antagonist of the DvLGIC/GluCl receptor protein. Again, these compounds that modulate the expression of DNA or RNA encoding DvLGIC/GluCl or the biological function thereof may be detected by a variety of assays. The assay may be a simple "yes/no" assay to determine whether there is a change in expression or function. The assay may be made quantitative by comparing the expression or function of a test sample with the levels of expression or function in a standard sample.

Expression of DvLGIC/GluCl DNA may also be performed using *in vitro* produced synthetic mRNA. Synthetic mRNA can be efficiently translated in various cell-free systems, including but not limited to wheat germ extracts and reticulocyte extracts, as well as efficiently translated in cell based systems, including but not limited to microinjection into frog oocytes, with microinjection into frog oocytes being preferred.

Following expression of DvLGIC/GluCl in a host cell, DvLGIC/GluCl protein may be recovered to provide DvLGIC/GluCl protein in active form. Several DvLGIC/GluCl protein purification procedures are available and suitable for use. Recombinant DvLGIC/GluCl protein may be purified from cell lysates and extracts by various combinations of, or individual application of salt fractionation, ion exchange chromatography, size exclusion chromatography, hydroxylapatite adsorption chromatography and hydrophobic interaction chromatography. In addition, recombinant DvLGIC/GluCl protein can be separated from other cellular proteins by use of an immunoaffinity column made with monoclonal or polyclonal antibodies specific for full-length DvLGIC/GluCl protein, or polypeptide fragments of DvLGIC/GluCl protein.

*D. variabilis* channel functional assays measure one or more ligand-gated chloride channel activities where the channel is made up in whole, or in part, by the DvLGIC/GluCl channel. DvLGIC/GluCl channel activity can be measured using the channel described herein by itself; or as a subunit in combination with one or more additional ligand-gated chloride channel subunits (preferably one or more DvLGIC/GluCl), where the subunits combine together to provide functional channel activity. Assays measuring DvLGIC/GluCl-gated chloride channel activity include functional screening using ³⁶Cl, functional screening using patch clamp electrophysiology and functional screening using fluorescent dyes. Techniques for carrying out such assays in general are well known in the art. (See, for example, Smith et al., 1998, *European Journal of Pharmacology* 159:261-269; González and Tsien, 1997, *Chemistry & Biology* 4:269-277; Millar et al., 1994, *Proc. R. Soc. Lond. B.* 258:307-314; Rauh et al., 1990 *TiPS* 11:325-329, and Tsien et al., U.S. Patent No. 5,661,035.) Functional assays can be performed using individual compounds or preparations containing different compounds. A preparation containing different compounds where one or more compounds affect DvLGIC/GluCl channel activity can be divided into smaller groups of compounds to identify the compound(s) affecting DvLGIC/GluCl channel activity. In an embodiment of the present invention a test preparation containing at least 10 compounds is used in a functional assay. Recombinantly produced DvLGIC/GluCl channels present in different environments can be used in a functional assay. Suitable environments include live cells and purified cell extracts containing the DvLGIC/GluCl channel and an appropriate membrane for activity; and the use of a purified DvLGIC/GluCl channel produced by recombinant means that is introduced into a different environment suitable for measuring DvLGIC/GluCl channel activity. DvLGIC/GluCl derivatives can be used to assay for compounds active at the channel and to obtain information concerning different regions of the channel. For example, DvLGIC/GluCl channel derivatives can be produced where amino acid regions in the native channel are altered and the effect of the alteration on channel activity can be measured to obtain information regarding different channel regions.

Polyclonal or monoclonal antibodies may be raised against DvLGIC/GluCl or a synthetic peptide (usually from about 9 to about 25 amino acids in length) from a portion of DvLGIC/GluCl 1 (i.e., 1, 11 or 7-1) or DvLGIC/GluCl2 (10-2) as disclosed in SEQ ID NOs:2, 5and/or 7. Monospecific antibodies to DvLGIC/GluCl arc purified from mammalian antisera containing antibodies reactive against DvLGIC/GluCl or are prepared as monoclonal antibodies reactive with DvLGIC/GluCl using the technique of Kohler and Milstein (1975, *Nature* 256: 495-497). Monospecific antibody as used herein is defined as a single antibody species or multiple antibody species with homogenous binding characteristics for DvLGIC/GluCl. Homogenous binding as used herein refers to the ability of the antibody species to bind to a specific antigen or epitope, such as those associated with DvLGIC/GluCl, as described above. Human DvLGIC/GluCl-specific antibodies are raised by immunizing animals such as mice, rats, guinea pigs, rabbits, goats, horses and the like, with an appropriate concentration of DvLGIC/GluCl protein or a synthetic peptide generated from a portion of DvLGIC/GluCl with or without an immune adjuvant.

Preimmune serum is collected prior to the first immunization. Each animal receives between about 0.1 mg and about 1000 mg of DvLGIC/GluCl protein associated with an acceptable immune adjuvant. Such acceptable adjuvants include, but are not limited to, Freund's complete, Freund's incomplete, alum-precipitate, water in oil emulsion containing *Corynebacterium parvum* and tRNA. The initial immunization consists of DvLGIC/GluCl protein or peptide fragment thereof in, preferably, Freund's complete adjuvant at multiple sites either subcutaneously (SC), intraperitoneally (IP) or both. Each animal is bled at regular intervals, preferably weekly, to determine antibody titer. The animals may or may not receive booster injections following the initial immunization. Those animals receiving booster injections are generally given an equal amount of DvLGIC/GluCl in Freund's incomplete adjuvant by the same route. Booster injections are given at about three week intervals until maximal titers are obtained. At about 7 days after each booster immunization or about weekly after a single immunization, the animals are bled, the serum collected, and aliquots are stored at about -20°C.

Monoclonal antibodies (mAb) reactive with DvLGIC/GluCl are prepared by immunizing inbred mice, preferably Balb/c, with DvLGIC/GluCl protein. The mice are immunized by the IP or SC route with about 1 mg to about 100 mg, preferably about 10 mg, of DvLGIC/GluCl protein in about 0.5 ml buffer or saline incorporated in an equal volume of an acceptable adjuvant, as discussed above. Freund's complete adjuvant is preferred. The mice receive an initial immunization on day 0 and are rested for about 3 to about 30 weeks. Immunized mice are given one or more booster immunizations of about 1 to about 100 mg of DvLGIC/GluCl in a buffer solution such as phosphate buffered saline by the intravenous (IV) route. Lymphocytes, from antibody positive mice, preferably splenic lymphocytes, are obtained by removing spleens from immunized mice by standard procedures known in the art. Hybridoma cells are produced by mixing the splenic lymphocytes with an appropriate fusion partner, preferably myeloma cells, under conditions which will allow the formation of stable hybridomas. Fusion partners may include, but are not limited to: mouse myelomas P3/NS1/Ag 4-1; MPC-11; S-194 and Sp 2/0, with Sp 2/0 being preferred. The antibody producing cells and myeloma cells are fused in polyethylene glycol, about 1000 mol. wt., at concentrations from about 30% to about 50%. Fused hybridoma cells are selected by growth in hypoxanthine, thymidine and aminopterin supplemented Dulbecco's Modified Eagles Medium (DMEM) by procedures known in the art. Supernatant fluids are collected form growth positive wells on about days 14, 18, and 21 and are screened for antibody production by an immunoassay such as solid phase immunoradioassay (SPIRA) using DvLGIC/GluCl as the antigen. The culture fluids are also tested in the Ouchterlony precipitation assay to determine the isotype of the mAb. Hybridoma cells from antibody positive wells are cloned by a technique such as the soft agar technique of MacPherson, 1973, Soft Agar Techniques, in *Tissue Culture Methods and Applications,* Kruse and Paterson, Eds., Academic Press.

Monoclonal antibodies are produced *in vivo* by injection of pristine primed Balb/c mice, approximately 0.5 ml per mouse, with about 2 x 10⁶ to about 6 x 10⁶ hybridoma cells about 4 days after priming. Ascites fluid is collected at approximately 8-12 days after cell transfer and the monoclonal antibodies are purified by techniques known in the art.

*In vitro* production of anti-DvLGIC/GluCl mAb is carried out by growing the hybridoma in DMEM containing about 2% fetal calf serum to obtain sufficient quantities of the specific mAb. The mAb are purified by techniques known in the art.

Antibody titers of ascites or hybridoma culture fluids are determined by various serological or immunological assays which include, but are not limited to, precipitation, passive agglutination, enzyme-linked immunosorbent antibody (ELISA) technique and radioimmunoassay (RIA) techniques. Similar assays are used to detect the presence of DvLGIC/GluCl in body fluids or tissue and cell extracts.

It is readily apparent to those skilled in the art that the above described methods for producing monospecific antibodies may be utilized to produce antibodies specific for DvLGIC/GluCl peptide fragments, or a respective full-length DvLGIC/GluCl.

DvLGIC/GluCl antibody affinity columns are made, for example, by adding the antibodies to Affigel-10 (Biorad), a gel support which is pre-activated with N-hydroxysuccinimide esters such that the antibodies form covalent linkages with the agarose gel bead support. The antibodies are then coupled to the gel via amide bonds with the spacer arm. The remaining activated esters are then quenched with 1M ethanolamine HCl (pH 8). The column is washed with water followed by 0.23 M glycine HCl (pH 2.6) to remove any non-conjugated antibody or extraneous protein. The column is then equilibrated in phosphate buffered saline (pH 7.3) and the cell culture supernatants or cell extracts containing full-length DvLGIC/GluCl or DvLGIC/GluCl protein fragments are slowly passed through the column. The column is then washed with phosphate buffered saline until the optical density (A₂₈₀) falls to background, then the protein is eluted with 0.23 M glycine-HCl (pH 2.6). The purified DvLGIC/GluC1 protein is then dialyzed against phosphate buffered saline.

The present invention also relates to a non-human transgenic animal which is useful for studying the ability of a variety of compounds to act as modulators of DvLGIC/GluCl, or any alternative functional DvLGIC/GluCl channel *in vivo* by providing cells for culture, *in vitro.* In reference to the transgenic animals of this invention, reference is made to transgenes and genes. As used herein, a transgene is a genetic construct including a gene. The transgene is integrated into one or more chromosomes in the cells in an animal by methods known in the art. Once integrated, the transgene is carried in at least one place in the chromosomes of a transgenic animal. Of course, a gene is a nucleotide sequence that encodes a protein, such as one or a combination of the cDNA clones described herein. The gene and/or transgene may also include genetic regulatory elements and/or structural elements known in the art. A type of target cell for transgene introduction is the embryonic stem cell (ES). ES cells can be obtained from pre-implantation embryos cultured *in vitro* and fused with embryos (Evans et al., 1981, *Nature* 292:154-156; Bradley et al., 1984, *Nature* 309:255-258; Gossler et al., 1986, *Proc. Natl. Acad. Sci.* USA 83:9065-9069; and Robertson et al., 1986 *Nature* 322:445-448). Transgenes can be efficiently introduced into the ES cells by a variety of standard techniques such as DNA transfection, microinjection, or by retrovirus-mediated transduction. The resultant transformed ES cells can thereafter be combined with blastocysts from a non-human animal. The introduced ES cells thereafter colonize the embryo and contribute to the germ line of the resulting chimeric animal (Jaenisch, 1988, *Science* 240: 1468-1474).

The present invention also relates to a non-human transgenic animal which is useful for studying the ability of a variety of compounds to act as modulators of DvLGIC/GluCl. In regard to transgenic animals of this invention, reference is made to transgenes and genes. As used herein, a transgene is a genetic construct including a gene. The transgene is integrated into one or more chromosomes in the cells in an animal by methods known in the art. Once integrated, the transgene is carried in at least one place in the chromosomes of a transgenic animal. Of course, a gene is a nucleotide sequence that encodes a protein, such as one or a combination of the cDNA clones described herein. The gene and/or transgene may also include genetic regulatory elements and/or structural elements known in the art. A type of target cell for transgene introduction is the embryonic stem cell (ES). ES cells can be obtained from pre-implantation embryos cultured *in vitro* and fused with embryos (Evans et al., 1981, *Nature* 292:154-156; Bradley et al., 1984, *Nature* 309:255-258; Gossler et al., 1986, *Proc. Natl. Acad. Sci.* USA 83:9065-9069; and Robertson et al., 1986 *Nature* 322:445-448). Transgenes can be efficiently introduced into the ES cells by a variety of standard techniques such as DNA transfection, microinjection, or by retrovirus-mediated transduction. The resultant transformed ES cells can thereafter be combined with blastocysts from a non-human animal. The introduced ES cells thereafter colonize the embryo and contribute to the germ line of the resulting chimeric animal (Jaenisch, 1988, *Science* 240: 1468-1474).

A naturally occuring DvLGIC/GluCl gene is referred to as the native gene, and if it is not mutant, it can also be referred to as wild-type. An altered DvLGIC/GluCl gene should not fully encode the same LGIC/GluCl as native to the host animal, and its expression product can be altered to a minor or greater degree, or absent altogether. In cases where it is useful to express a non-native DvLGIC/GluCl gene in a transgenic animal in the absence of a native LGIC/GluCl gene (such as within *C. elegans*), we prefer that the altered LGIC/GluCl gene induce a null knockout phenotype in the animal. However a more modestly modified LGIC/GluCl gene can also be useful and is within the scope of the present invention. The DvLGIC/GluCl mutation may be a targeted deletion mutation, a targeted substitution mutation and/or a targeted insertion mutation. However, the preferred mutation is a deletion mutation, and especially preferred is a deletion mutation which results in a deletion of most if not all of the DvLGIC/GluCl gene. Transgenic animals are generated which have an altered, or preferably, completely deleted LGIC/GluCl gene. LGIC/GluCl gene deletions, gene modifications and or gene insertions can render the native gene nonfunctional, producing a "knockout" transgenic animal, or can lead to a LGIC/GluCl with altered expression or activity. As noted above, a non-human transgenic animal without an activated DvLGIC/GluCl gene can be used to for testing/screening of modulators of DvLGIC/GluCl expression and/or activity (modulators such as small molecules or peptides) that may reverse the pathological phenotype which results from the overexpression or deletion of DvLGIC/GluCl.

A preferred deletion mutation may contain a deletion of anywhere from 1 nucleotide to deletion of the entire gene, including the open reading frame and associated *cis*-acting regulatory sequences associated with wild type DvLGIC/GluCl. A smaller deletion within the open reading frame is preferably not divisible by three, so as to result in a frameshift mutation resulting in a protein which most likely is non-functional. It is preferred that any such smaller deletion not divisible by three be targeted toward the 5' region of the open reading frame to increase the possibility of generating a non-functional truncated protein product. However, as noted above, it is preferable that the deletion mutation encompass most if not all of the DvLGIC/GluCl gene so as to insure prevention of expression of a functional DvLGIC/GluCl protein. Therefore, the DvLGIC/GluCl deficient animal cells, non-human transgenic embryos, non-human transgenic animals and non-human transgenic littermates of the present invention may be generated by any techniques known in the art, as sampled in the previous paragraph. It will also be within the purview of the skilled artisan to produce transgenic or knock-out invertebrate animals (e.g., *C. elegans)* which express the DvLGIC/GluCl transgene in a wild type *C. elegans* LGIC/GluCl background as well in *C. elegans* mutants deficient for one or more of the *C. elegans* LGIC/GluCl subunits.

Pharmaceutically useful compositions comprising modulators of DvLGIC/GluCl may be formulated according to known methods such as by the admixture of a pharmaceutically acceptable carrier. Examples of such carriers and methods of formulation may be found in Remington's Pharmaceutical Sciences. To form a pharmaceutically acceptable composition suitable for effective administration, such compositions will contain an effective amount of the protein, DNA, RNA, modified DvLGIC/GluCl, or either DvLGIC/GluCl agonists or antagonists including tyrosine kinase activators or inhibitors.

Therapeutic or diagnostic compositions of the invention are administered to an individual in amounts sufficient to treat or diagnose disorders. The effective amount may vary according to a variety of factors such as the individual's condition, weight, sex and age. Other factors include the mode of administration.

The pharmaceutical compositions may be provided to the individual by a variety of routes such as subcutaneous, topical, oral and intramuscular.

The term "chemical derivative" describes a molecule that contains additional chemical moieties which are not normally a part of the base molecule. Such moieties may improve the solubility, half-life, absorption, etc. of the base molecule. Alternatively the moieties may attenuate undesirable side effects of the base molecule or decrease the toxicity of the base molecule. Examples of such moieties are described in a variety of texts, such as Remington's Pharmaceutical Sciences.

Compounds identified according to the methods disclosed herein may be used alone at appropriate dosages. Alternatively, co-administration or sequential administration of other agents may be desirable.

The present invention also has the objective of providing suitable topical, oral, systemic and parenteral pharmaceutical formulations for use in the novel methods of treatment of disorders involving components of the present invention. The compositions containing compounds identified according to this invention as the active ingredient can be administered in a wide variety of therapeutic dosage forms in conventional vehicles for administration. For example, the compounds can be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

For combination treatment with more than one active agent, where the active agents are in separate dosage formulations, the active agents can be administered concurrently, or they each can be administered at separately staggered times.

The dosage regimen utilizing the compounds of the present invention is selected in accordance with a variety of factors including type, species, age, weight, sex and medical condition of the patient; the severity of the condition to be treated; the route of administration; the renal, hepatic and cardiovascular function of the patient; and the particular compound thereof employed. A physician or veterinarian of ordinary skill can readily determine and prescribe the effective amount of the drug required to prevent, counter or arrest the progress of the condition. Optimal precision in achieving concentrations of drug within the range that yields efficacy without toxicity requires a regimen based on the kinetics of the drug's availability to target sites. This involves a consideration of the distribution, equilibrium, and elimination of a drug.

The following examples are provided to illustrate the present invention without, however, limiting the same hereto.

### EXAMPLE 1

### Isolation and Expression of cDNAs Encoding DvLGIC/GluCl 1, DvLGIC/GluCl 11, DvLGIC/GluCl 7-1 (DvGluCl1) and DvLGIC/GluCl 10-2 (DvGluCl2)

### From a Tick Dermacentor cDNA library

*Generation of a tick Demacentor cDNA library* - PolyA⁺ RNAwas purified from whole *Dermacentor* ticks to generate an oligo(dT)-primed ZAP cDNA library cloned as 5' EcoRI-3' XhoI inserts. The library consisted of approximately 1.8x10⁶ independent clones prior to amplification. The ZAP Express cDNA Synthesis Kit and the ZAP Express^{™} cDNA GigapackIII Gold Cloning Kit were purchased from Stratagene (La Jolla, CA) and used according to the manufacturer's instructions.

*Library Screening and Isolation of Dermacentor LGIC*/*GluCl genes -* Two DNA probes were used.
1. A first probe is from the tick *Rhipicephalus sanguineus* LGIC/GluC11 (RsLGIC/GluCl1) gene and was PCR amplified using as primers i) sense strand 5' CGG ATA TTG GAC AGC ATC 3'(SEQ ID NO:8) and ii) antisense strand 5' CCA GTA GAC GAG GTT GAA GAG G-3' (SEQ ID NO:9), to generate a fragment that runs from nucleotide 448 through 1645 of the RsLGIC/GluCl 1 open reading frame. The nucleotide sequence of the RsLGIC/GluCl1 probe is as follows:
2. A second probe is from the tick *Rhipicephalus sanguineus* LGIC/GluCl2 clone (RsLGIC/GluCl2) gene which was PCR amplified using as primers i) sense strand 5' TGT GGT GGT GAT AGC TGC 3' (SEQ ID NO:11) and ii) antisense strand 5' GAG TTG ATC AAT CTG CTT GG 3' (SEQ ID NO:12), to, generate a fragment that runs from nucleotide 166 through 1315 of the Rs LGIC/GluCl 2 open reading frame. The nucleotide sequence of the RsLGIC/GluCl1 probe is as follows:

Vent DNA Polymerase for PCR was purchased from New England Biolabs (Boston MA). Each amplification cycle consisted of 1 min. at 95° C, 1 min. at 72° C, and 1 min. at 72° C. Following 35 cycles, there was a final 5 minute extension at 72°C. The PCR product was agarose gel purified, labeled with ³²P -dCTP using the Random Primer DNA Labeling System (GibcoBRL, Gaithersburg, MD), and the resulting RsLGIC/GluCl1 (SEQ ID NO:11) probe was first employed to screen approximately 5.5x10⁵ recombinants of the *Dermacentor* cDNA library.
Hybridization was performed in 6xSSPE, 0.1%SDS, 10x Denhardt's solution, salmon sperm DNA (200µg/ml), and 45% formamide at 42° C. The membranes were then washed twice in i) 2xSSC 0.5% SDS at room temperature for 15 min. and ii) 0.2xSSC 0.5% SDS at 42° C for 30 min., followed by a single wash in 0.2xSSC, 0.5% SDS at 55°C for 30 min. The RsLGIC/GluCl1 probe was removed from the membranes by i) incubating at ~ 1 hour in a 0.05M NaOH + 0.5M NaCl solution, then ii) incubating ~1 hour in a 0.5M Tris:Cl (pH7.4) solution, then iii) rinsing in 1XSSPE all at room temperature. Eight positive clones, including DvLGIC/GluCl1, DvLGIC/GluCl 11,DvLGIC/GluCl 7-1 and DvLGIC/GluCl 10-2 were identified in the original screen. DvLGIC/GluCl1, DvLGIC/GluCl 11, and DvLGIC/GluCl 7-1 were identified by both probes while DvLGIC/GluCl 10-2 was recognized only by RsLGIC/GluCl2 probe. All 6 inserts were excised from the phage, converted to pBK-CMV phagemid vectors using the manufacturer's protocol (Stratagene, La Jolla, CA), and sequenced on an ABI PRISM^{™} 377 DNA Sequencer (Perkin Elmer, Foster City; CA). The DvLGIC/GluCl1 cDNA insert is 3598 bp and is disclosed in Figure 1A-C and is disclosed as SEQ ID NO:1. The DvLGIC/GluCl11 cDNA insert is 3442 bp and is disclosed in Figure 3A-C and is disclosed as SEQ ID NO:3. The DvLGIC/GluCl 7-1 cDNA insert is 2194 bp and is disclosed in Figure 4A-B and is disclosed as SEQ ID NO:4. Finally, the DvLGIC/GluC110-2 cDNA insert is 4077 bp and is disclosed in Figure 6A-C and is disclosed as SEQ ID NO:6.

*Synthesis of in vitro transcribed capped RNA* - A PCR strategy was used to add the T7 promoter upstream of the initiating methionine (ATG) and a polyA⁺ tail following the stop codon (TAG) of the open reading frame (ORF) of clones DvLGIC/GluCl1, DvLGIC/GluCl 11,DvLGIC/GluCl 7-1 and DvLGIC/GluCl 10-2. Amplified ORFs which contained the flanking T7 promoter and polyA⁺ tail were used directly as templates in the *in vitro* transcription reaction (mMessage mMachine^{™}, Ambion, Austin, TX). After removal of DNA template, the volume was adjusted to 100 µl with nuclease free water, and RNA purified using a G-50 Sephadex Column (Boehringer Mannheim, Indianapolis, IN). The elutate was extracted with an equal volume of phenol/chloroform, followed with a second chloroform extraction, precipitated with isopropyl alcohol, and resuspended in nuclease-free water to a storage concentration of 1µg/µl.

### EXAMPLE 2

### Functional expression of DvLGIC/GluCl1 clones in Xenopus oocytes

*Xenopus laevis* oocytes were prepared and injected using standard methods previously described [Arena, J.P., Liu, K.K., Paress, P.S. & Cully, D.F. Mol. Pharmacol. 40, 368-374 (1991); Arena, J.P., Liu, K.K., Paress, P.S., Schaeffer, J.M. & Cully, D.F., Mol. Brain Res. 15, 339-348 (1992)]. Adult female *Xenopus laevis* were anesthetized with 0.17% tricaine methanesulfonate and the ovaries were surgically removed and placed in a solution consisting of (mM): NaCl 82.5, KCl 2, MgCl₂ 1, HEPES 5, NaPyruvate 2.5, Penicillin G. 100,000 units/L, Streptomycin Sulfate 1000 mg/L, pH 7.5 (Mod. OR-2). Ovarian lobes were broken open, rinsed several times in Mod. OR-2, and incubated in 0.2% collagenase (Sigma, Type1) in Mod. OR-2 at room temperature with gentle shaking. After 1 hour the collagenase solution was renewed and the oocytes were incubated for an additional 30-90 min until approximately 50% of the oocytes were released from the ovaries. Stage V and VI oocytes were selected and placed in media containing (mM): NaCl 96, KCl 2, MgCl₂1, CaCl₂ 1.8, HEPES 5, NaPyruvate 2.5, theophylline 0.5, gentamicin 50 mg/ml, pH 7.5 (ND-96) for 16-24 hours before injection. Oocytes were injected with 50 nl of DvLGIC/GluCl1 or DvLGIC/GluCl 7-1 RNA at a concentration of 0.2 mg/ml. Oocytes were incubated at 18°C for 1-6 days in ND-96 before recording.

Recordings were made at room temperature in modified ND-96 consisting of (mM): NaCl 96, MgCl₂ 1, CaCl₂ 0.1, BaCl₂ 3.5, HEPES 5, pH 7.5. Oocytes were voltage clamped using a Dagan CA1 two microelectrode amplifier (Dagan Corporation, Minneapolis, MN) interfaced to a Macintosh 7100/80 computer. The current passing electrode was filled with 0.7 M KCl, 1.7 M KCitrate, and the voltage recording electrode was filled with 1 M KCl. Throughout the experiment oocytes were superfused with modified ND-96 (control solution) or with ND-96 containing potential channel activators and blockers at a rate of approximately 3 ml/min. Data were acquired at 100 Hz and filtered at 33.3 Hz using Pulse software from HEKA Elektronik (Lambrecht, Germany). All recordings were performed from a holding potential of either 0 or -30 mV.

Oocytes expressing DvLGIC/GluCl 1 (Figure 9) or DvLGIC/GluCl 7-1 (Figure 10) exhibited a slowly activating current in response to application of 1 µM ivermectin phosphate. This current was irreversible upon wash-out of ivermectin phosphate. In contrast, application of 1 mM glutamate did not activate a current.

### EXAMPLE 3

### Functional expression of DvLGIC/GluCl Clones in Mammalian Cells

A DvLGIC/GluCl may be subcloned into a mammalian expression vector and used to transfect the mammalian cell line of choice. Stable cell clones are selected by growth in the presence of G418. Single G418 resistant clones are isolated and tested to confirm the presence of an intact DvLGIC/GluCl gene. Clones containing the DvLGIC/GluCls are then analyzed for expression using immunological techniques, such as immuneprecipitation, Western blot, and immunofluorescence using antibodies specific to the DvLGIC/GluCl proteins. Antibody is obtained from rabbits innoculated with peptides that are synthesized from the amino acid sequence predicted from the DvLGIC/GluCl sequences. Expression is also analyzed using patch clamp electrophysiological techniques and an anion flux assay.

Cells that are expressing DvLGIC/GluCl stably or transiently, are used to test for expression of active channel proteins. These cells are used to identify and examine compounds for their ability to modulate, inhibit or activate the respective channel.

Cassettes containing the DvLGIC/GluCl cDNA in the positive orientation with respect to the promoter are ligated into appropriate restriction sites 3' of the promoter and identified by restriction site mapping and/or sequencing. These cDNA expression vectors may be introduced into fibroblastic host cells, for example, COS-7 (ATCC# CRL1651), and CV-1 tat [Sackevitz et al.,1987, *Science* 238: 1575], 293, L (ATCC# CRL6362) by standard methods including but not limited to electroporation, or chemical procedures (cationic liposomes, DEAE dextran, calcium phosphate). Transfected cells and cell culture supernatants can be harvested and analyzed for DvLGIC/GluCl expression as described herein.

All of the vectors used for mammalian transient expression can be used to establish stable cell lines expressing DvLGIC/GluCl. Unaltered DvLGIC/GluCl cDNA constructs cloned into expression vectors are expected to program host cells to make DvLGIC/GluCl protein. The transfection host cells include, but are not limited to, CV-1-P [Sackevitz et al.,1987, *Science* 238: 1575], tk-L [Wigler, et al., 1977, *Cell* 11: 223], NS/0, and dHFr- CHO [Kaufman and Sharp, 1982, *J. Mol. Biol.* 159: 601].

Co-transfection of any vector containing a DvLGIC/GluCl cDNA with a drug selection plasmid including, but not limited to G418, aminoglycoside phosphotransferase; hygromycin, hygromycin-B phosphotransferase; APRT, xanthine-guanine phosphoribosyl-transferase, will allow for the selection of stably transfected clones. Levels of DvLGIC/GluCl are quantitated by the assays described herein. DvLGIC/GluCl cDNA constructs may also be ligated into vectors containing amplifiable drug-resistance markers for the production of mammalian cell clones synthesizing the highest possible levels of DvLGIC/GluCl. Following introduction of these constructs into cells, clones containing the plasmid are selected with the appropriate agent, and isolation of an over-expressing clone with a high copy number of plasmids is accomplished by selection with increasing doses of the agent. The expression of recombinant DvLGIC/GluCl is achieved by transfection of full-length DvLGIC/GluCl cDNA into a mammalian host cell.

### EXAMPLE 4

### Cloning of DvLGIC/GluCl cDNA into a Baculovirus Expression Vector for Expression in Insect Cells

Baculovirus vectors, which are derived from the genome of the AcNPV virus, are designed to provide high level expression of cDNA in the Sf9 line of insect cells (ATCC CRL# 1711). A recombinant baculoviruse expressing DvLGIC/GluCl cDNA is produced by the following standard methods (In Vitrogen Maxbac Manual): The DvLGIC/GluCl cDNA constructs are ligated into the polyhedrin gene in a variety of baculovirus transfer vectors, including the pAC360 and the BlueBac vector (InVitrogen). Recombinant baculoviruses are generated by homologous recombination following co-transfection of the baculovirus transfer vector and linearized AcNPV genomic DNA [Kitts, 1990, *Nuc. Acid. Res.* 18: 5667] into Sf9-cells. Recombinant pAC360 viruses are identified by the absence of inclusion bodies in infected cells and recombinant pBlueBac viruses are identified on the basis of b-galactosidase expression (Summers, M. D. and Smith, G. E., Texas Agriculture Exp. Station Bulletin No. 1555). Following plaque purification, DvLGIC/GluCl expression is measured by the assays described herein.

The cDNA encoding the entire open reading frame for DvLGIC/GluCl LGIC/GluCl is inserted into the BamHI site of pBlueBacII. Constructs in the positive orientation are identified by sequence analysis and used to transfect Sf9 cells in the presence of linear AcNPV mild type DNA.

### EXAMPLE 5

### Cloning of DvLGIC/GluCl cDNA into a Yeast Expression Vector

Recombinant DvLGIC/GluCl is produced in the yeast *S. cerevisiae* following the insertion of the optimal DvLGIC/GluCl cDNA cistron into expression vectors designed to direct the intracellular or extracellular expression of heterologous proteins. In the case of intracellular expression, vectors such as EmBLyex4 or the like are ligated to the DvLGIC/GluCl cistron [Rinas, et al., 1990, *Biotechnology 8*: 543-545; Horowitz B. et al., 1989, *J. Biol. Chem. 265:* 4189-4192]. For extracellular expression, the DvLGIC/GluCl LGIC/GluCl cistron is ligated into yeast expression vectors which fuse a secretion signal (a yeast or mammalian peptide) to the NH₂ terminus of the DvLGIC/GluCl protein [Jacobson, 1989, *Gene 85:* 511-516; Riett and Bellon , 1989, *Biochem. 28:* 2941-2949].

These vectors include, but are not limited to pAVE1-6, which fuses the human serum albumin signal to the expressed cDNA [Steep, 1990, *Biotechnology 8*: 42-46], and the vector pL8PL which fuses the human lysozyme signal to the expressed cDNA [Yamamoto, *Biochem. 28*: 2728-2732)]. In addition, DvLGIC/GluCl is expressed in yeast as a fusion protein conjugated to ubiquitin utilizing the vector pVEP [Ecker, 1989, *J. Biol. Chem. 264:* 7715-7719, Sabin, 1989 *Biotechnology 7*: 705-709, McDonnell, 1989, *Mol. Cell Biol. 9:* 5517-5523 (1989)]. The levels of expressed DvLGIC/GluCl are determined by the assays described herein.

### EXAMPLE 6

### Purification of Recombinant DvLGIC/GluCl

Recombinantly produced DvLGIC/GluCl may be purified by antibody affinity chromatography. DvLGIC/GluCl LGIC/GluCl antibody affinity columns are made by adding the anti-DvLGIC/GluCl LGIC/GluCl antibodies to Affigel-10 (Biorad), a gel support which is pre-activated with N-hydroxysuccinimide esters such that the antibodies form covalent linkages with the agarose gel bead support. The antibodies are then coupled to the gel via amide bonds with the spacer arm. The remaining activated esters are then quenched with 1M ethanolamine HCl (pH 8). The column is washed with water followed by 0.23 M glycine HCl (pH 2.6) to remove any non-conjugated antibody or extraneous protein. The column is then equilibrated in phosphate buffered saline (pH 7.3) together with appropriate membrane solubilizing agents such as detergents and the cell culture supernatants or cell extracts containing solubilized DvLGIC/GluCl are slowly passed through the column. The column is then washed with phosphate- buffered saline together with detergents until the optical density (A280) falls to background, then the protein is eluted with 0.23 M glycine-HCl (pH 2.6) together with detergents. The purified DvLGIC/GluCl protein is then dialyzed against phosphate buffered saline.

## Claims

1. A purified nucleic acid molecule encoding a *D. variabilis* LGIC/GluCl channel protein, wherein said nucleic acid molecule comprises:
(a) a nucleic acid molecule which encodes an amino acid sequence selected from the group consisting of SEQ ID NOs 2, and 5;
(b) a nucleic acid molecule which hybridizes under conditions of moderate to high stringency to the complement of a second nucleic acid molecule which encodes SEQ ID NOs 2, or 5; or,
(c) a nucleic acid molecule which hybridizes under conditions of moderate stringency to the complement of a second nucleic acid molecule as set forth in SEQ ID NOs 1, 3, or 4; wherein said nucleic acid molecule has at least about a . 65% identity to at least one of the second nucleic acid molecules as set forth in SEQ ID NOs 1, 3, and 4.

2. A purified nucleic acid molecule encoding a *D. variabilis* LGIC/GluCl channel protein, wherein said protein comprises an amino acid sequence as set forth in SEQ ID NO:2.

3. An expression vector for expressing a *D. variabilis* LGIC/GluCl channel protein in a recombinant host cell wherein said expression vector comprises a nucleic acid molecule of claim 2.

4. A host cell which expresses a recombinant *D. variabilis* LGIC/GluCl channel protein wherein said host cell contains the expression vector of claim 3.

5. A process for expressing a *D. variabilis* LGIC/GluCl channel protein in a recombinant host cell, comprising:
(a) transfecting the expression vector of claim 3 into a suitable host cell; and,
(b) culturing the host cells of step (a) under conditions which allow expression of said *D. variabilis* LGIC/GluCl channel protein from said expression vector.

6. A purified DNA molecule encoding a *D. variabilis* LGIC/GluCl channel protein which consists of a nucleotide sequence as set forth in SEQ ID NO:1.

7. The DNA molecule of claim 6 which consists of the nucleotide sequence from about nucleotide 170 to about nucleotide 1363.

8. A purified DNA molecule encoding a *D. variabilis* LGIC/GluCl channel protein which consists of a nucleotide sequence as set forth in SEQ ID NO:3.

9. The DNA molecule of claim 8 which consists of the nucleotide sequence from about nucleotide 32 to about nucleotide 1225.

10. A purified nucleic acid molecule encoding a *D*. *variabilis* LGIC/GluCl channel protein, wherein said protein comprises an amino acid sequence as set forth in SEQ ID NO:5.

11. An expression vector for expressing a *D. variabilis* LGIC/GluCl channel protein in a recombinant host cell wherein said expression vector comprises a nucleic acid molecule of claim 10.

12. A host cell which expresses a recombinant *D. variabilis* LGIC/GluCl channel protein wherein said host cell contains the expression vector of claim 11.

13. A process for expressing a *D. variabilis* LGIC/GluCl channel protein in a recombinant host cell, comprising:
(a) transfecting the expression vector of claim 11 into a suitable host cell; and,
(b) culturing the host cells of step (a) under conditions which allow expression of said *D. variabilis* LGIC/GluCl channel protein from said expression vector.

14. A purified DNA molecule encoding a *D. variabilis* LGIC/GluCl channel protein which consists of a nucleotide sequence as set forth in SEQ ID NO:4.

15. The DNA molecule of claim 14 which consists of the nucleotide sequence from about nucleotide 47 to about nucleotide 1315.

16. A *D*. *variabilis* LGIC/GluCl channel protein substantially free from other proteins which comprises an amino acid sequence as set forth in SEQ ID NO:2.

17. A *D. variabilis* LGIC/GluCl channel protein of claim 16 which is a product of a DNA expression vector contained within a recombinant host cell.

18. A substantially pure membrane preparation comprising the *D. variabilis* LGIC/GluCl channel protein purified from the recombinant host cell of claim 17.

19. A *D*. *variabilis* LGIC/GluCl channel protein substantially free from other proteins which comprises an amino acid sequence as set forth in SEQ ID NO:5.

20. A *D. variabilis* LGIC/GluCl channel protein of claim 19 which is a product of a DNA expression vector contained within a recombinant host cell.

21. A substantially pure membrane preparation comprising the *D. variabilis* LGIC/GluCl channel protein purified from the recombinant host cell of claim 20.

22. A *D. variabilis* LGIC/GlnCl channel protein which consists of an amino acid sequence selected from the group consisting of amino acid sequences as set forth in SEQ ID NO:2, and SEQ ID NO:5.

23. A method of identifying a modulator of a LGIC/GluCl channel protein, comprising:
(a) contacting a test compound with a *D*. *variabilis* LGIC/GluCl channel protein selected from the group consisting of SEQ ID NO:2, and SEQ ID NO:5; and,
(b) measuring the effect of the test compound on the LGIC/GluCl channel protein.

24. The method of claim 23 wherein the *D. variabilis* LGIC/GluCl protein of step (a) is a product of a DNA expression vector contained within a recombinant host cell.

25. A method of identifying a compound that modulates glutamate-gated channel protein activity, which comprises:
a) injecting into a host cell solution a population of nucleic acid molecules, at least of portion of which encodes a *D. variabilis* LGIC/GluCl channel protein selected from the group consisting of SEQ ID NO:2, and SEQ ID NO:5, such that expression of said portion of nucleic acid molecules results in an active glutamate-gated channel;
b) adding a test compound into said solution; and,
c) measuring host cell membrane current at a holding potential more positive than the reversal potential for chloride.

26. The method of claim 25 wherein said nucleic acid molecule is selected from the group consisting of complementary DNA, poly A⁺ messenger RNA and complementary RNA.

## Patentansprüche

1. Gereinigtes Nucleinsäuremolekül, das ein *D. variabilis*-LGlC/GluCl-Kanalprotein codiert, wobei das Nucleinsäuremolekül umfasst:
(a) ein Nucleinsäuremolekül, das eine Aminsäuresequenz codiert, die aus der Gruppe bestehend aus den SEQ ID NO:2 und SEQ ID NO:5 ausgewählt ist;
(b) ein Nucleinsäuremolekül, das unter moderaten bis hohen stringenten Bedingungen an das Komplement eines zweiten Nucleinsäuremoleküls hybridisiert, das SEQ ID NO:2 oder SEQ ID NO:5 codiert, oder
(c) ein Nucleinsäuremolekül, das unter moderaten stringenten Bedingungen an das Komplement eines in SEQ ID NO:1, SEQ ID NO:3 oder SEQ ID NO:4 dargestellten zweiten Nucleinsäuremoleküls hybridisiert, wobei das Nucleinsäuremolekül mindestens zu 65% identisch ist mit mindestens einem der in SEQ ID NO:1, SEQ ID NO:3 und SEQ ID NO:4 dargestellten zweiten Nucleinsäuremoleküle.

2. Gereinigtes Nucleinsäuremolekül, das ein *D. variabilis*-LGIC/GluCl-Kanalprotein codiert, wobei das Protein eine in SEQ ID NO:2 dargestellte Aminosäuresequenz umfasst.

3. Expressionsvektor zur Expression des *D. variabilis*-LGIC/GluCl-Kanalproteins in einer rekombinanten Wirtszelle, wobei der Expressionsvektor ein Nucleinsäuremolekül nach Anspruch 2 umfasst.

4. Wirtszelle, die ein rekombinantes *D. variabilis-*LGIC/GluCl-Kanalprotein exprimiert, wobei die Wirtszelle den Expressionsvektor nach Anspruch 3 enthält.

5. Verfahren zur Expression eines *D. variabilis*-LGIC/GluCl-Kanalproteins in einer rekombinanten Wirtszelle, umfassend:
(a) Transfektion des Expressionsvektors nach Anspruch 3 in eine geeignete Wirtszelle; und
(b) Züchten der Wirtszellen aus Schritt (a) unter Bedingungen, die die Expression des *D. variabilis*-LGIC/GluCl-Kanalproteins von dem Expressionsvektor erlauben.

6. Gereinigtes DNA-Molekül, das ein *D. variabilis*-LGIC/GluCl-Kanalprotein codiert, das aus einer in SEQ ID NO:1 dargestellten Nucleotidsequenz besteht.

7. DNA-Molekül nach Anspruch 6, das aus der Nucleotidsequenz von etwa Nucleotid 170 bis etwa Nucleotid 1363 besteht.

8. Gereinigtes DNA-Molekül, das ein *D. variabilis*-LGIC/GluCl-Kanalprotein codiert, das aus einer in SEQ ID NO:3 dargestellten Nucleotidsequenz besteht.

9. DNA-Molekül nach Anspruch 8, das aus der Nucleotidsequenz von etwa Nucleotid 32 bis etwa Nucleotid 1225 besteht.

10. Gereinigtes DNA-Molekül, das ein *D. variabilis-*LGIC/GluCl-Kanalprotein codiert, wobei das Protein eine in SEQ ID NO:5 dargestellte Aminosäuresequenz umfasst.

11. Expressionsvektor zur Expression eines *D. variabilis*-LGIC/GluCl-Kanalproteins in einer rekombinanten Wirtszelle, wobei der Expressionsvektor ein Nucleinsäuremolekül nach Anspruch 10 umfasst.

12. Wirtszelle, die ein *D. variabilis*-LGIC/GluCl-Kanalprotein exprimiert, wobei die Wirtszelle den Expressionsvektor nach Anspruch 11 enthält.

13. Verfahren zur Expression eines *D. variabilis*-LGIC/GluCl-Kanalproteins in einer rekombinanten Wirtszelle, umfassend:
(a) Transfektion des Expressionsvektors nach Anspruch 11 in eine geeignete Wirtszelle; und
(b) Züchten der Wirtszellen aus Schritt (a) unter Bedingungen, die die Expression des *D. variabilis*-LGIC/GluCl-Kanalproteins von dem Expressionsvektor erlauben.

14. Gereinigtes DNA-Molekül, das ein *D. variabilis*-LGIC/GluCl-Kanalprotein codiert, das aus einer in SEQ ID NO:4 dargestellten Nucleotidsequenz besteht.

15. DNA-Molekül nach Anspruch 14, das aus der Nucleotidsequenz von etwa Nucleotid 47 bis etwa Nucleotid 1315 besteht.

16. *D. variabilis*-LGIC/GluCl-Kanalprotein, das im Wesentlichen frei von anderen Proteinen ist und das die in SEQ ID NO:2 dargestellte Aminosäuresequenz umfasst.

17. *D. variabilis*-LGIC/GluCl-Kanalprotein nach Anspruch 16, das ein Produkt eines in einer rekombinanten Wirtszelle enthaltenen DNA-Expressionsvektors ist.

18. Im Wesentlichen reines Membranpräparat, umfassend das *D. variabilis-*LGIC/GluCl-Kanalprotein, das von der rekombinanten Wirtszelle nach Anspruch 17 gereinigt wurde.

19. *D. variabilis*-LGIC/GluCl-Kanalprotein, das im Wesentlichen frei von anderen Proteinen ist und die in SEQ ID NO:5 dargestellte Aminosäuresequenz umfasst.

20. *D. variabilis*-LGIC/GluCl-Kanalprotein nach Anspruch 19, das ein Produkt eines in einer rekombinanten Wirtszelle enthaltenen DNA-Expressionsvektors ist.

21. Im Wesentlichen reines Membranpräparat, umfassend das *D. variabilis-*LGIC/GluCl-Kanalprotein, das von der rekombinanten Wirtszelle nach Anspruch 20 gereinigt wurde.

22. *D. variabilis*-LGIC/GluCl-Kanalprotein, das aus einer Aminosäuresequenz besteht, die ausgewählt ist aus der Gruppe bestehend aus den in SEQ ID NO: 2 und SEQ ID NO: 5 dargestellten Aminosäuresequenzen.

23. Verfahren zur Identifizierung eines Modulators eines LGIC/GluCl-Kanalproteins umfassend:
(a) das Inkontaktbringen einer Testverbindung mit einem *D. variabilis-*LGIC/GluCl-Kanalprotein ausgewählt aus der Gruppe bestehend aus SEQ ID NO:2 und SEQ ID NO:5, und
(b) das Messen der Wirkung der Testverbindung auf das *D. variabilis-*LGIC/GluCl-Kanalprotein.

24. Verfahren nach Anspruch 23, wobei das *D. variabilis*-LGIC/GluCl-Protein aus Schritt (a) ein Produkt eines DNA-Expressionsvektors ist, der in einer rekombinanten Wirtszelle enthalten ist.

25. Verfahren zur Identifizierung einer Verbindung, die die Glutamat-gesteuerte Aktivität des Kanalproteins moduliert, das umfasst:
(a) Injektion, in eine Wirtszelllösung, einer Population von Nucleinsäuremolekülen, von denen zumindest ein Teil das *D. variabilis-*LGIC/GluCl-Kanalprotein ausgewählt aus der Gruppe bestehend aus SEQ ID NO:2 und SEQ ID NO:5 codiert, so dass durch die Expression des Teiles von Nucleinsäuremolekülen ein aktiver Glutamat-gesteuerter Kanal entsteht;
(b) Hinzufügen einer Testverbindung zu der Lösung; und
(c) Messen des Stroms der Wirtszellmembran bei einem Haltepotential, das mehr positiv ist als das Umkehrpotential für Chlorid.

26. Verfahren nach Anspruch 25, wobei das Nucleinsäuremolekül ausgewählt ist aus der Gruppe bestehend aus komplementärer DNA, Poly A⁺ mRNA und komplementärer RNA.

## Revendications

1. Molécule d'acide nucléique purifiée codant une protéine canal LGIC/GluCl de *D. variabilis,* ladite molécule d'acide nucléique comprenant :
(a) une molécule d'acide nucléique qui code une séquence d'acides aminés choisie dans le groupe consistant en SEQ ID N° 2 et 5 ;
(b) une molécule d'acide nucléique qui s'hybride dans des conditions modérément à très stringentes au complément d'une seconde molécule d'acide nucléique qui code SEQ ID N° 2 ou 5 ; ou
(c) une molécule d'acide nucléique qui s'hybride dans des conditions modérément stringentes au complément d'une seconde molécule d'acide nucléique comme décrite par SEQ ID N° 1, 3 ou 4 ; ladite molécule d'acide nucléique ayant au moins environ 65 % d'identité avec au moins l'une des secondes molécules d'acide nucléique comme décrites par SEQ ID N° 1, 3, 4 et 5.

2. Molécule d'acide nucléique purifiée codant une protéine canal LGIC/GluCl de *D. variabilis,* ladite protéine comprenant une séquence d'acides aminés comme décrite par SEQ ID N° 2.

3. Vecteur d'expression pour exprimer une protéine canal LGIC/GluCl de *D. variabilis* dans une cellule hôte recombinante, ledit vecteur d'expression comprenant une molécule d'acide nucléique selon la revendication 2.

4. Cellule hôte qui exprime une protéine canal LGIC/GluCl de *D. variabilis,* ladite cellule hôte contenant le vecteur d'expression selon la revendication 3.

5. Processus pour exprimer une protéine canal LGIC/GluCl de *D. variabilis* dans une cellule hôte recombinante, comprenant :
(a) transfecter le vecteur d'expression de la revendication 3 dans une cellule hôte adaptée ; et
(b) cultiver les cellules hôtes de l'étape (a) dans des conditions qui permettent l'expression de ladite protéine canal LGIC/GluCl de *D. variabilis* à partir dudit vecteur d'expression.

6. Molécule d'ADN purifiée codant une protéine canal LGIC/GluCl de *D. variabilis* qui consiste en une séquence de nucléotides comme décrite par SEQ ID N° 1.

7. Molécule d'ADN selon la revendication 6 qui consiste en la séquence de nucléotides du nucléotide 170 environ au nucléotide 1 363 environ.

8. Molécule d'ADN purifiée codant une protéine canal LGIC/GluCl de *D. variabilis* qui consiste en une séquence de nucléotides comme décrite par SEQ ID N° 3.

9. Molécule d'ADN selon la revendication 8 qui consiste en la séquence de nucléotides du nucléotide 32 environ au nucléotide 1 225 environ.

10. Molécule d'acide nucléique purifiée codant une protéine canal LGIC/GluCl de *D. variabilis,* ladite protéine comprenant une séquence d'acides aminés comme décrite par SEQ ID N° 5.

11. Vecteur d'expression pour exprimer une protéine canal LGIC/GluCl de *D. variabilis* dans une cellule hôte recombinante, ledit vecteur d'expression comprenant une molécule d'acide nucléique selon la revendication 10.

12. Cellule hôte qui exprime une protéine canal LGIC/GluCl de *D. variabilis* recombinante, ladite cellule hôte contenant le vecteur d'expression selon la revendication 11.

13. Processus pour exprimer une protéine canal LGIC/GluCl de *D. variabilis* dans une cellule hôte recombinante, comprenant :
(a) transfecter le vecteur d'expression selon la revendication 11 dans une cellule hôte adaptée ; et
(b) cultiver les cellules hôtes de l'étape (a) dans des conditions qui permettent l'expression de ladite protéine canal LGIC/GluCl de *D. variabilis* depuis ledit vecteur d'expression.

14. Molécule d'ADN purifiée codant une protéine canal LGIC/GluCl de *D. variabilis* qui consiste en une séquence de nucléotides comme décrite par SEQ ID N° 4.

15. Molécule d'ADN selon la revendication 14 qui consiste en la séquence de nucléotide du nucléotide 47 environ au nucléotide 1 315 environ.

16. Protéine canal LGIC/GluCl de *D. variabilis* sensiblement exempte d'autres protéines qui comprend une séquence d'acides aminés comme décrite par SEQ ID N° 2.

17. Protéine canal LGIC/GluCl de *D. variabilis* selon la revendication 16 qui est un produit d'un vecteur d'expression d'ADN contenu dans une cellule hôte recombinante.

18. Préparation de membrane sensiblement pure comprenant la protéine canal LGIC/GluCl de *D. variabilis* purifiée à partir de la cellule hôte recombinante selon la revendication 17.

19. Protéine canal LGIC/GluCl de *D. variabilis* sensiblement exempte d'autres protéines qui comprend une séquence d'acides aminés comme décrite par SEQ ID N° 5.

20. Protéine canal LGIC/GluCl de *D. variabilis* selon la revendication 19 qui est un produit d'un vecteur d'expression d'ADN contenu dans une cellule hôte recombinante.

21. Préparation de membrane sensiblement pure comprenant la protéine canal LGIC/GluCl de *D. variabilis* purifiée à partir de la cellule hôte recombinante de la revendication 20.

22. Protéine canal LGIC/GluCl de *D. variabilis* qui consiste en une séquence d'acides aminés sélectionnée dans le groupe consistant en les séquences d'acides aminés décrites par SEQ ID N° 2 et SEQ ID N° 5.

23. Procédé pour identifier un modulateur d'une protéine canal LGIC/GluCl de *D. variabilis* comprenant les étapes consistant à :
(a) mettre en contact un composé à l'essai avec une protéine canal LGIC/GluCl de *D. variabilis* sélectionnée dans le groupe consistant en SEQ ID N° 2 et SEQ ID N° 5 ; et
(b) mesurer l'effet du composé à l'essai sur la protéine canal LGIC/GluCl de *D. variabilis.*

24. Procédé selon la revendication 23 dans lequel la protéine canal LGIC/GluCl de *D. variabilis* de l'étape (a) est un produit d'un vecteur d'expression de l'ADN contenu dans une cellule hôte recombinante.

25. Procédé d'identification d'un composé qui module l'activité de la protéine canal commandée par le glutamate, qui comprend :
a) injecter dans une solution de cellules hôtes une population de molécules d'acide nucléique, dont au moins une partie code une protéine canal LGIC/GluCl de *D. variabilis* choisie dans le groupe consistant en SEQ ID N° 2 et SEQ ID N° 5, de sorte que l'expression de ladite partie des molécules d'acide nucléique ait pour résultat un canal actif commandé par le glutamate :
b) ajouter un composé à l'essai dans ladite solution ; et
c) mesurer le courant de la membrane des cellules hôtes à un potentiel de référence plus positif que le potentiel d'inversion du chlorure.

26. Procédé selon la revendication 25 dans lequel ladite molécule d'acide nucléique est sélectionnée dans le groupe consistant en de l'ADN complémentaire, de l'ARN messager poly A+ et de l'ARN complémentaire.


### SEQUENCE LISTING

<110> Merck & Co., Inc.
<120> DNA MOLECULES ENCODING LIGAND GATED ION CHANNELS FROM DERMACENTOR VARIABILIS
<130> 20629 PCT
<150> 60/193,935
   <151> 2000-03-31
<160> 13
<170> FastSEQ for Windows Version 4.0
<210> 1
   <211> 3598
   <212> DNA
   <213> Dermacentor variabilis
<220>
   <221> CDS
   <222> (170)...(1363)
<400> 1
   gcgaggctgt cggtggaaag cgcggcgagc acgcgtccgc gcgcctgcgc tccagtccgg    60
   acccgagctg gagcacggcc tggagggata ggtctggtcg accgtggttg cagctccaga   120
   cgcgcagttg gagctcggcg aaggggctgc tgctgcgagc actgtgcgc atg cca ctt   178
                                                         Met Pro Leu
                                                          1
   tca gcg ctg aac gtg tgg cgc gct tgc gtc acg ttg tcc ctc ctc agg     226
   Ser Ala Leu Asn Val Trp Arg Ala Cys Val Thr Leu Ser Leu Leu Arg
        5                  10                  15
   acg acg ctc gcg cag gaa agg cgg tca aac gga gcg ctg gat gac ctg     274
   Thr Thr Leu Ala Gln Glu Arg Arg Ser Asn Gly Ala Leu Asp Asp Leu
    20                  25                  30                  35
   gag aag ctt gac gac tta tta aga acc tat gac cgg cgt gcc ctt ccc     322
   Glu Lys Leu Asp Asp Leu Leu Arg Thr Tyr Asp Arg Arg Ala Leu Pro
                    40                  45                  50
   acg aca cac ttg gga acg cca aca aaa gtg gct tgc gaa atc tac ata     370
   Thr Thr His Leu Gly Thr Pro Thr Lys Val Ala Cys Glu Ile Tyr Ile
                55                  60                  65
   cgc agc ttc ggg tcc ata aat cca gcc aca atg gac tat gag gtt gat     418
   Arg Ser Phe Gly Ser Ile Asn Pro Ala Thr Met Asp Tyr Glu Val Asp
            70                  75                  80
   ctt tat ttg cgg cag act tgg caa gat gat cgc ttg acg agc ccc aac     466
   Leu Tyr Leu Arg Gln Thr Trp Gln Asp Asp Arg Leu Thr Ser Pro Asn
        85                  90                  95
   gta tcc agg ccc ctg gac ctc aat gat cca aag ctg gtg cag cgt ata     514
   Val Ser Arg Pro Leu Asp Leu Asn Asp Pro Lys Leu Val Gln Arg Ile
   100                 105                 110                 115
   tgg aaa ccg gaa gta ttc ttc gca aat gcc aaa cac gca gag ttc caa      562
   Trp Lys Pro Glu Val Phe Phe Ala Asn Ala Lys His Ala Glu Phe Gln
                   120                 125                 130
   tat gtc aca gta cct aat gta ctg gtc cgc gtt aac ccg aac gga aag      610
   Tyr Val Thr Val Pro Asn Val Leu Val Arg Val Asn Pro Asn Gly Lys
               135                 140                 145
   att cta tac atg ctc agg ctc aag cta agg ttt gca tgt atg atg gat      658
   Ile Leu Tyr Met Leu Arg Leu Lys Leu Arg Phe Ala Cys Met Met Asp
           150                 155                 160
   tta tat cgc ttt cct atg gac tcc caa gtt tgc agc atc gaa ctc gcc      706
   Leu Tyr Arg Phe Pro Met Asp Ser Gln Val Cys Ser Ile Glu Leu Ala
       165                 170                 175
   tca ttc tcg aaa aca acc gaa gaa ctg cat ctg gag tgg tct gat acc      754
   Ser Phe Ser Lys Thr Thr Glu Glu Leu His Leu Glu Trp Ser Asp Thr
   180                 185                 190                 195
   aat ccg ata ata cta ttc gaa ggc ctg aag tta cca caa ttc gag att      802
   Asn Pro Ile Ile Leu Phe Glu Gly Leu Lys Leu Pro Gln Phe Glu Ile
                   200                 205                 210
   cag aat ata aat acg tca atc tgc atg gag aaa ttt cac atc gga gag      850
   Gln Asn Ile Asn Thr Ser Ile Cys Met Glu Lys Phe His Ile Gly Glu
               215                 220                 225
   tac agc tgc ctg aag gcc gac ttc cac ttg cag cgg tca ctg ggc tac      898
   Tyr Ser Cys Leu Lys Ala Asp Phe His Leu Gln Arg Ser Leu Gly Tyr
           230                 235                 240
   cac atg gtg cag tcg tat ctg cct aca gtg ctc atc gtg gtc atc tcg      946
   His Met Val Gln Ser Tyr Leu Pro Thr Val Leu Ile Val Val Ile Ser
       245                 250                 255
   tgg gtg tcc ttc tgg ctc gac gtt gag tcc att ccg gcg cgc acc aca      994
   Trp Val Ser Phe Trp Leu Asp Val Glu Ser Ile Pro Ala Arg Thr Thr
   260                 265                 270                 275
   ctg ggc gtc acg acg ctg ctc act att tct tcc aag ggc tcc ggt ata     1042
   Leu Gly Val Thr Thr Leu Leu Thr Ile Ser Ser Lys Gly Ser Gly Ile
                   280                 285                 290
   cag tcc aac ttg cct ccg gtc tca tac gtg aag gca atc gat gtg tgg     1090
   Gln Ser Asn Leu Pro Pro Val Ser Tyr Val Lys Ala Ile Asp Val Trp
               295                 300                 305
   atg gga gcc tgc acg ggc ttc gtg ttc tcg gca cta ctg gag ttc acc     1138
   Met Gly Ala Cys Thr Gly Phe Val Phe Ser Ala Leu Leu Glu Phe Thr
           310                 315                 320
   gtc gtc agc tgc ctg gcc agg atg cag gca cga gac aag gag tca agt     1186
   Val Val Ser Cys Leu Ala Arg Met Gln Ala Arg Asp Lys Glu Ser Ser
       325                 330                 335
   atg gtt aca aca aag cac gga gtg gcg att gtc aac gct gtt cct gat     1234
   Met Val Thr Thr Lys His Gly Val Ala Ile Val Asn Ala Val Pro Asp
   340                 345                 350                 355
   aac cag gcg tcg gtt cct tgc act gtc cgg gcg aaa act att gac cag    1282
   Asn Gln Ala Ser Val Pro Cys Thr Val Arg Ala Lys Thr Ile Asp Gln
                   360                 365                 370
   gtc tgc cgc gta gcg ttt ccg gcc atc ttc ctc gtg ttt aac gcc att    1330
   Val Cys Arg Val Ala Phe Pro Ala Ile Phe Leu Val Phe Asn Ala Ile
               375                 380                 385
   tac tgg ccg tat ttt atg tgc ttt aca gag tag aacatcaccg aacaacgcaa  1383
   Tyr Trp Pro Tyr Phe Met Cys Phe Thr Glu  *
           390                 395
   aagttctgcg gaaaaagtgt ccgtataacg tgtcttgagg ctcattgtca cgtatttaca  1443
   ccggcatgaa aggttcgtta aatcaaccaa tatagcgtcc tcagccaatt acgcacacta  1503
   gtttagagca gccagtcgca tttcctttac tactatcgag agaggttgga ctaagtcatg  1563
   agttcattcc cttcggtagc ttctgtcaat tgtctcaggg aaggataggt tggtgcttcg  1623
   agctctttag cgcatgcaaa ctctgttggg atgcttaggt acgcgcaggg aacgtgacga  1683
   tctataatgt tttttggagt agtaatggaa cacggcactg acggtcgata aatttgatag  1743
   catgaggaag tgaactaatt actataaaat gcacaacggc tttattgtgg agtattgcgc  1803
   gttttctttt tataatgtag gagggataga atataagtgc caagaagcag atacctaaaa  1863
   tcgtaaaaca gcgccgccat gtagatgtct gatttagaag ataccgttgc actgcatcac  1923
   aggcgtagca tacaacaaat ttaagctctt ctataggaaa tagaaatatt gagtattact  1983
   tcgttaatgc gggaatcgta tttgttaaat gtatctttcg attaacaatt gggactttcg  2043
   ctgtttcaat acagactttg ttgagccttc gtataacatt acgaaaaaaa aagaaaatct  2103
   gaaaagaata atatctacgt tttcaatacc agccattcta gtccagaagg caagcgtgct  2163
   gcaaaatccg aaagcaaaat ttatttatgt taaatataac atcccggtca tttgccctaa  2223
   ctttgtggcg acaattgaca gcgtcaacta aactgcgtat tccatgttgt cgcttaatgg  2283
   ctttgccatg atgccatctt agtcattttc agctgttcaa agttttaagg aataagctat  2343
   gcttaagcta caattgattg ttaatgaagt gtcagcgcga agacttgcga gtttgatttc  2403
   gtacatatga gtgttcttta tacaccctga cactaccttt ttggaggcga tgagccgaga  2463
   attcagaaaa cgtcatggcc agttttaaca gaacagtgac cctgttaaaa atgtctgtat  2523
   gaatactgtt gttatttatg gtagttttga aatcgtttaa tatatgttat gttacgtgat  2583
   caagtgtcaa tggctataca ttatcgacct cccattaact tgatcaatcc aatcgtccag  2643
   acatttaatg tccgaggaac ttcaggttta ttaactgtag gttaaaactc tgatgtatat  2703
   ataacagcat ggaatgcaag atctcgtcat atttcatgca atttcactag atgcagcgat  2763
   gttttcgatg gagattattc gtctcctgaa aaaaaaaatt gacattcacc ggcatgtagg  2823
   ctgaagctat gaagaaaacc cagctgggtt tcctttgtag cttcgttttt ttcctagata  2883
   aggttaatat cttgatctct gtgctacagt aagagtgaaa ctgaactcgg cctgaaaaac  2943
   ttgcgttttc ttatcgcact accgtcattg aaacgctcag tactaggtct tggtgaaaca  3003
   catgactaaa atttgaaagc tttagaatga atttatttat ttttatttat ttacaaatac  3063
   tgcaatcccg ttacgggatt gcagtatttg cattatgaaa gaaacacatt atgaaagaaa  3123
   cgagaaacgc aatcttcgca ttatgaaaga aacgagcaga agacagatgg ctaattttat  3183
   ttgctgattg tagcccattt tcctcttact agagagttat gggtgacagc agaattctca  3243
   gaatagtgca ttctcttaaa ataacttgac atcgtgtggt aatttcccta aatctcatgt  3303
   aggtagatgc tttatttatg taatttgagg agacataccc atgaaaacga aaagatgacg  3363
   ggcgctaatg gttatagaag tccttcctgc cactgttggc tgaaatgtat ttgtatgttt  3423
   tttggtcagt cactgtgtcc caaagcttct tcgtgctgaa gcttaagtga gtctatgctg  3483
   ttcaacacca ttgtatattt ttgtaataaa atagtttatt aaatgacctg gttctacttg  3543
   aaaaaaaaaa aaaaaaaaaa aaaaaaaaaa aaaaaaaaaa aaaaaaaaaa aaaaa       3598
<210> 2
   <211> 397
   <212> PRT
   <213> Dermacentor variabilis
<400> 2
   Met Pro Leu Ser Ala Leu Asn Val Trp Arg Ala Cys Val Thr Leu Ser
    1               5                  10                  15
   Leu Leu Arg Thr Thr Leu Ala Gln Glu Arg Arg Ser Asn Gly Ala Leu
               20                  25                  30
   Asp Asp Leu Glu Lys Leu Asp Asp Leu Leu Arg Thr Tyr Asp Arg Arg
           35                  40                  45
   Ala Leu Pro Thr Thr His Leu Gly Thr Pro Thr Lys Val Ala Cys Glu
       50                  55                  60
   Ile Tyr Ile Arg Ser Phe Gly Ser Ile Asn Pro Ala Thr Met Asp Tyr
   65                  70                  75                  80
   Glu Val Asp Leu Tyr Leu Arg Gln Thr Trp Gln Asp Asp Arg Leu Thr
                   85                  90                  95
   Ser Pro Asn Val Ser Arg Pro Leu Asp Leu Asn Asp Pro Lys Leu Val
               100                 105                 110
   Gln Arg Ile Trp Lys Pro Glu Val Phe Phe Ala Asn Ala Lys His Ala
           115                 120                 125
   Glu Phe Gln Tyr Val Thr Val Pro Asn Val Leu Val Arg Val Asn Pro
       130                 135                 140
   Asn Gly Lys Ile Leu Tyr Met Leu Arg Leu Lys Leu Arg Phe Ala Cys
   145                 150                 155                 160
   Met Met Asp Leu Tyr Arg Phe Pro Met Asp Ser Gln Val Cys Ser Ile
                   165                 170                 175
   Glu Leu Ala Ser Phe Ser Lys Thr Thr Glu Glu Leu His Leu Glu Trp
               180                 185                 190
   Ser Asp Thr Asn Pro Ile Ile Leu Phe Glu Gly Leu Lys Leu Pro Gln
           195                 200                 205
   Phe Glu Ile Gln Asn Ile Asn Thr Ser Ile Cys Met Glu Lys Phe His
       210                 215                 220
   Ile Gly Glu Tyr Ser Cys Leu Lys Ala Asp Phe His Leu Gln Arg Ser
   225                 230                 235                 240
   Leu Gly Tyr His Met Val Gln Ser Tyr Leu Pro Thr Val Leu Ile Val
                   245                 250                 255
   Val Ile Ser Trp Val Ser Phe Trp Leu Asp Val Glu Ser Ile Pro Ala
               260                 265                 270
   Arg Thr Thr Leu Gly Val Thr Thr Leu Leu Thr Ile Ser Ser Lys Gly
           275                 280                 285
   Ser Gly Ile Gln Ser Asn Leu Pro Pro Val Ser Tyr Val Lys Ala Ile
       290                 295                 300
   Asp Val Trp Met Gly Ala Cys Thr Gly Phe Val Phe Ser Ala Leu Leu
   305                 310                 315                 320
   Glu Phe Thr Val Val Ser Cys Leu Ala Arg Met Gln Ala Arg Asp Lys
                   325                 330                 335
   Glu Ser Ser Met Val Thr Thr Lys His Gly Val Ala Ile Val Asn Ala
               340                 345                 350
   Val Pro Asp Asn Gln Ala Ser Val Pro Cys Thr Val Arg Ala Lys Thr
           355                 360                 365
   Ile Asp Gln Val Cys Arg Val Ala Phe Pro Ala Ile Phe Leu Val Phe
       370                 375                 380
   Asn Ala Ile Tyr Trp Pro Tyr Phe Met Cys Phe Thr Glu
   385                 390                 395
<210> 3
   <211> 3442
   <212> DNA
   <213> Dermacentor variabilis
<220>
   <221> CDS
   <222> (32)...(1225)
<400> 3
   cgaaggggct gctgctgcga gcactgtgcg c atg cca ctt tca gcg ctg aac          52
                                      Met Pro Leu Ser Ala Leu Asn
                                      1               5
   gtg tgg cgc gct tgc gtc acg ttg tcc ctc ctc agg acg acg ctc gcg        100
   Val Trp Arg Ala Cys Val Thr Leu Ser Leu Leu Arg Thr Thr Leu Ala
           10                  15                  20
   cag gaa agg cgg tca aac gga gcg ctg gat gac ctg gag aag ctt gac      148
   Gln Glu Arg Arg Ser Asn Gly Ala Leu Asp Asp Leu Glu Lys Leu Asp
        25                  30                  35
   gac tta tta aga acc tat gac cgg cgt gcc ctt ccc acg aca cac ttg      196
   Asp Leu Leu Arg Thr Tyr Asp Arg Arg Ala Leu Pro Thr Thr His Leu
    40                  45                  50                  55
   gga acg cca aca aaa gtg gct tgc gaa atc tac ata cgc agc ttc ggg      244
   Gly Thr Pro Thr Lys Val Ala Cys Glu Ile Tyr Ile Arg Ser Phe Gly
                    60                  65                  70
   tcc ata aat cca gcc aca atg gac tat gag gtt gat ctt tat ttg cgg      292
   Ser Ile Asn Pro Ala Thr Met Asp Tyr Glu Val Asp Leu Tyr Leu Arg
                75                  80                  85
   cag act tgg caa gat gat cgc ttg acg agc ccc aac gta tcc agg ccc      340
   Gln Thr Trp Gln Asp Asp Arg Leu Thr Ser Pro Asn Val Ser Arg Pro
            90                  95                 100
   ctg gac ctc aat gat cca aag ctg gtg cag cgt ata tgg aaa cca gaa      388
   Leu Asp Leu Asn Asp Pro Lys Leu Val Gln Arg Ile Trp Lys Pro Glu
       105                 110                 115
   gta ttc ttc gca aat gca aaa cac gca gag ttc caa tat gtc aca gta      436
   Val Phe Phe Ala Asn Ala Lys His Ala Glu Phe Gln Tyr Val Thr Val
   120                 125                 130                 135
   cct aat gta ctg gtc cgc gtt aac ccg aac gga aag att cta tac atg      484
   Pro Asn Val Leu Val Arg Val Asn Pro Asn Gly Lys Ile Leu Tyr Met
                   140                 145                 150
   ctc agg ctc aag cta agg ttt gca tgt atg atg gat cta tat cgc ttt      532
   Leu Arg Leu Lys Leu Arg Phe Ala Cys Met Met Asp Leu Tyr Arg Phe
               155                 160                 165
   cct atg gac tcc caa gtt tgc agc atc gaa ctc gcc tca ttc tcg aaa      580
   Pro Met Asp Ser Gln Val Cys Ser Ile Glu Leu Ala Ser Phe Ser Lys
           170                 175                 180
   aca acc gaa gaa ctg cat ctg gag tgg tcc gat acc aat ccg ata ata      628
   Thr Thr Glu Glu Leu His Leu Glu Trp Ser Asp Thr Asn Pro Ile Ile
       185                 190                 195
   cta ttc gaa ggc ctg aag tta cca cag ttc gag att cag aat ata aat      676
   Leu Phe Glu Gly Leu Lys Leu Pro Gln Phe Glu Ile Gln Asn Ile Asn
   200                 205                 210                 215
   acg tca atc tgc atg gag aaa ttt cac atc gga gag tac agc tgc ctg      724
   Thr Ser Ile Cys Met Glu Lys Phe His Ile Gly Glu Tyr Ser Cys Leu
                   220                 225                 230
   aag gcc gac ttc cac ttg cag cgg tca ctg ggc tac cac atg gtg cag      772
   Lys Ala Asp Phe His Leu Gln Arg Ser Leu Gly Tyr His Met Val Gln
               235                 240                 245
   tcg tat ctg cct aca gtg ctc atc gtg gtc atc tcg tgg gtg tcc ttc      820
   Ser Tyr Leu Pro Thr Val Leu Ile Val Val Ile Ser Trp Val Ser Phe
           250                 255                 260
   tgg ctc gac gtt gag tcc att ccg gcg cgc acc aca ctg ggc gtc acg       868
   Trp Leu Asp Val Glu Ser Ile Pro Ala Arg Thr Thr Leu Gly Val Thr
       265                 270                 275
   acg ctg ctc act att tct tcc aag ggc tcc ggt ata cag tcc aac ttg       916
   Thr Leu Leu Thr Ile Ser Ser Lys Gly Ser Gly Ile Gln Ser Asn Leu
   280                 285                 290                 295
   cct ccg gtc tca tac gtg aag gca atc gat gtg tgg atg gga gcc tgc       964
   Pro Pro Val Ser Tyr Val Lys Ala Ile Asp Val Trp Met Gly Ala Cys
                   300                 305                 310
   acg ggc ttc gtg ttc tcg gca cta ctg gag ttc acc gtc gtc agc tgc      1012
   Thr Gly Phe Val Phe Ser Ala Leu Leu Glu Phe Thr Val Val Ser Cys
               315                 320                 325
   ctg gcc agg atg cag gca cga gac aag gag tca agc atg gtt aca aca      1060
   Leu Ala Arg Met Gln Ala Arg Asp Lys Glu Ser Ser Met Val Thr Thr
           330                 335                 340
   aag cac gga gtg gcg att gtc aac gct gtt cct gat aac caa gcg tcg      1108
   Lys His Gly Val Ala Ile Val Asn Ala Val Pro Asp Asn Gln Ala Ser
       345                 350                 355
   gtt cct tgc act gtc cgg gcg aaa act att gac cag gtc tgc cgc gta      1156
   Val Pro Cys Thr Val Arg Ala Lys Thr Ile Asp Gln Val Cys Arg Val
   360                 365                 370                 375
   gcg ttt ccg gcc atc ttc ctc gtg ttt aac gcc att tac tgg ccg tac      1204
   Ala Phe Pro Ala Ile Phe Leu Val Phe Asn Ala Ile Tyr Trp Pro Tyr
                   380                 385                 390
   ttt atg tgc ttt act gag tag aacatcaccg aacaaggcaa tagttctgcg         1255
   Phe Met Cys Phe Thr Glu  *
               395
   gaaaaagtgt ccgtataacg tgtcttgagg ctcattgtca cgtatttaca ccggcatgaa   1315
   aggtaggtca agggagcgtt cgttaaatca accaatatag cgtcctcagc caattacgca   1375
   cactagttta gagcagccag tcgaatttcc tttactacta tcgagagagg ttggactaag   1435
   tcatgagttc attcccttcg gtagcttctg tcaattgtct cagggaagga taggttggtg   1495
   cttcgagctc tttagcgcat gcaaactctg ttgggatgct taggtacgcg cagggaacgt   1555
   gacgatctat aatgtttttt ggagtagtaa tggaacacgg cactgacggt cgataaattt   1615
   gatggtatga ggaagtgcac tgattactat aaaatgcaca acggctttat tgtggagtat   1675
   ggctcgtttt ctttttataa tgtaggaggg atagaatata agtgccaaga agcagatact   1735
   taaaatccta aaacagcgcc gccatgtaga tgtctgattt agaagatacc gttgcactgc   1795
   atcacaagcg tagcatacaa caaatttaag ctcttctata ggaaatagaa atattgagta   1855
   ttacttcgtt aatgcgggaa tcgtatttgt taaatgtatc tttcgattaa caattgggac   1915
   tttcgctgtt tcaatacaga cttttttgag ccttcgtata acattacgaa aaaaaaagaa   1975
   aatctgaaaa gaataatatc tacgttttca ataccagcca ttctagtcca gaaggcaagc   2035
   gtgctgcaaa atccgaaagc aaaatttatt tatgttaaat ataacatccc ggtcatttgc   2095
   cctaactttg tggcgacaat tgacagcgtc aactaaactg cgtattccat gttgtcgctt   2155
   aatggctttg ccatgatgcc atcttagtca ttttcagctg ttcaaagttt taaggaataa   2215
   gctatgctta agctacaatt gattgttaat gaagtgtcag cgcgaagact tgcgagtttg   2275
   atttcgtaca tatgagtgtt ctttatacaa cctgacacta cctttttgga ggcgatgagc   2335
   cgagaattca gaaaacgtca tggccagttt taacagaaca gtgaccctgt taaaatgtct   2395
   gtataaatac tgttgttatt tatggtagtt ttgaaatcgt ttaatatatg ttatgttacg   2455
   tgatcaagtg tcaatggcta tacattatcg acctcccatt aacttgatca atccaatcgt   2515
   ccagacattt aatgtccgag gaacttcagg tttattaact gtaggttaaa actctgatgt   2575
   atatataaca gcatggaatg caagatctcg tcatatttca tgcaatttca ctagatgcag   2635
   cgatgttttc gatggagatt attcgtctcc tgaaaaaaaa aattgacatt caccggcatg   2695
   taggctgaag ctatgaagga aacccagctg ggtttccttt gtagcttcgt ttttttccta    2755
   gataaggtta atatcttgat ctctgtgcta cagtaagagt gaaactgaac taggcctgaa   2815
   aaacttgcgt tttcttatcg cactaccttc attgaaacgc tcagtactag gtcttggtga   2875
   aacacatgac taaaatttga aagctttaga atgaatttat ttatttttat ttatttacaa   2935
   atactgcaat cccgttacgg gattgcagta tttgcattat gaaagaaaca cattatgaaa   2995
   gaaacgagaa acgcaatctt cgcattatga aagaaacgag cagaagacag atggctaatt   3055
   ttatttgctg attgtagccc atttttctct tactagagag ttatgggtga cagcagaatt   3115
   ctcagaatag tgcattctct taaaataact tgacatcgtg tggtaatttc cctaaatctc   3175
   atgtaggtag ctgctttatt tatgtaattt gaggagacat acccatgaaa acgaaaagac   3235
   gacgggcgct aatgattata gaagtccttc ctgccactgt tggctgaaat gtatttgtat   3295
   gttttttggt cagtcactgt gtcccaaagc ttcttcgtgc tgaagcttaa gtgagtctat   3355
   gctgttcaac accattgtat atttttgtaa taaaatagtt tattaaatga cctggttcta   3415
   cttgaaaaaa aaaaaaaaaa aaaaaaa                                       3442
<210> 4
   <211> 2194
   <212> DNA
   <213> Dermacentor variabilis
<220>
   <221> CDS
   <222> (47)...(1315)
<400> 4
   ctcggtcgcg cgcgcacaca gcaagtgctc cggtgaggcg gctgat atg atc ccg        55
                                                      Met Ile Pro
                                                       1
   gcg tcc gtg gct ctc ggc cga agg atg tgc tct ctg ctg ctc gct gtc      103
   Ala Ser Val Ala Leu Gly Arg Arg Met Cys Ser Leu Leu Leu Ala Val
        5                  10                  15
   gga tgc gcc acg act agc gcc tgg ttc gct cag gct gtc gac cac atc      151
   Gly Cys Ala Thr Thr Ser Ala Trp Phe Ala Gln Ala Val Asp His Ile
    20                  25                  30                  35
   gac aaa gga tac cca gca cca gga ctc ttc gat gat gtc gac ctt caa      199
   Asp Lys Gly Tyr Pro Ala Pro Gly Leu Phe Asp Asp Val Asp Leu Gln
                    40                  45                  50
   ata ttg gac aac atc tta tgg agc tac gac cga cgc atc acc cct ggt      247
   Ile Leu Asp Asn Ile Leu Trp Ser Tyr Asp Arg Arg Ile Thr Pro Gly
                55                  60                  65
   cat cat tta aac gtt cct aca gtt gtt aag tgc gag ata tat ctc agg      295
   His His Leu Asn Val Pro Thr Val Val Lys Cys Glu Ile Tyr Leu Arg
            70                  75                  80
   agt ttt gga gct gtg aac cct gca aca atg gac tac gac gta gac ctg      343
   Ser Phe Gly Ala Val Asn Pro Ala Thr Met Asp Tyr Asp Val Asp Leu
        85                  90                  95
   tac ctg cgt cag acg tgg acg gac ttg cgg atg aag aac gcc aac ctg      391
   Tyr Leu Arg Gln Thr Trp Thr Asp Leu Arg Met Lys Asn Ala Asn Leu
   100                 105                 110                 115
   acc cgg tcc cta gac tta aac gac ccc aac ctc ctc aag aaa gtg tgg      439
   Thr Arg Ser Leu Asp Leu Asn Asp Pro Asn Leu Leu Lys Lys Val Trp
                   120                 125                 130
   aaa cct gac gtc tac ttt ccc aat gcc aag cac ggg gag ttc cag ttc      487
   Lys Pro Asp Val Tyr Phe Pro Asn Ala Lys His Gly Glu Phe Gln Phe
               135                 140                 145
   gtc act gtt ccc aac gtt ctc ttg agg ata tac cct acc ggc gat ata      535
   Val Thr Val Pro Asn Val Leu Leu Arg Ile Tyr Pro Thr Gly Asp Ile
           150                 155                 160
   ctc tac atg tta agg cta aag cta aca ttc tcc tgc atg atg aac atg      583
   Leu Tyr Met Leu Arg Leu Lys Leu Thr Phe Ser Cys Met Met Asn Met
       165                 170                 175
   gag cgg tac ccc ctg gac cga cag gtc tgc agc atc gag ctt gcc tca      631
   Glu Arg Tyr Pro Leu Asp Arg Gln Val Cys Ser Ile Glu Leu Ala Ser
   180                 185                 190                 195
   ttt tcc aag acg aca aag gag gtt gag ctc caa tgg gga aac gct gag      679
   Phe Ser Lys Thr Thr Lys Glu Val Glu Leu Gln Trp Gly Asn Ala Glu
                   200                 205                 210
   gct gtc acc atg tac agt ggt ctg aag atg gca caa ttc gag ctt caa      727
   Ala Val Thr Met Tyr Ser Gly Leu Lys Met Ala Gln Phe Glu Leu Gln
               215                 220                 225
   caa atc agc ctg acg aag tgc agc ggc gcc ttt cag ata ggc gag tac      775
   Gln Ile Ser Leu Thr Lys Cys Ser Gly Ala Phe Gln Ile Gly Glu Tyr
           230                 235                 240
   agc tgc ctg cgc gcg gag ctc aac ttg aag cgt tcc att ggc cac cac      823
   Ser Cys Leu Arg Ala Glu Leu Asn Leu Lys Arg Ser Ile Gly His His
       245                 250                 255
   cta gtg cag tct tac ctg ccg tcc aca ctc atc gtg gtc gtg tcg tgg      871
   Leu Val Gln Ser Tyr Leu Pro Ser Thr Leu Ile Val Val Val Ser Trp
   260                 265                 270                 275
   gtg tcc ttc tgg ctc gac gtg gac gcc ata ccg gcg cgc atc acg ctg      919
   Val Ser Phe Trp Leu Asp Val Asp Ala Ile Pro Ala Arg Ile Thr Leu
                   280                 285                 290
   ggt gtc acc acg ctc ctc act att tcg tcg gag agc tcc gac cac cag      967
   Gly Val Thr Thr Leu Leu Thr Ile Ser Ser Glu Ser Ser Asp His Gln
               295                 300                 305
   gcc aac cta gcg ccg gtg tcg tac gtg aaa gcg ctc gac gtg tgg atg     1015
   Ala Asn Leu Ala Pro Val Ser Tyr Val Lys Ala Leu Asp Val Trp Met
           310                 315                 320
   ggc acg tgc acc atg ttc gtg ttc gcc gcg gtg ctc gag ttc acc ttc     1063
   Gly Thr Cys Thr Met Phe Val Phe Ala Ala Val Leu Glu Phe Thr Phe
       325                 330                 335
   gtc tcc tac ctc gct cgc aga aag cag atc gtg ccc gcc tct atc gcg     1111
   Val Ser Tyr Leu Ala Arg Arg Lys Gln Ile Val Pro Ala Ser Ile Ala
   340                 345                 350                 355
   gac gtc gag gct tcc caa gat ctc gtt ctt gtc gtg gga aac aag gac     1159
   Asp Val Glu Ala Ser Gln Asp Leu Val Leu Val Val Gly Asn Lys Asp
                   360                 365                 370
   aaa aat cga ccc ccg tca ccg tcc atc ccg acg tcc acc cac gtg gtc     1207
   Lys Asn Arg Pro Pro Ser Pro Ser Ile Pro Thr Ser Thr His Val Val
               375                 380                 385
   ttg gct tac aga cac cgt gcc aag cag atc gac caa gtg agc cgg gtc      1255
   Leu Ala Tyr Arg His Arg Ala Lys Gln Ile Asp Gln Val Ser Arg Val
           390                 395                 400
   gct ttc cca atc ggc ttt gtt ctc ttc aac gca ctc tac tgg ccc tat      1303
   Ala Phe Pro Ile Gly Phe Val Leu Phe Asn Ala Leu Tyr Trp Pro Tyr
       405                 410                 415
   tac ttg ctc tag ttggccatgg tctcagtgcc tacagctgct gctcccaacg          1355
   Tyr Leu Leu  *
   420
   tgcagccata cgccgggaaa cgggtggctg cgtaccccag ggaaacggtc ggccgctgga    1415
   ttgaaaagga ctgccatcac cgacgcacgc tctggtggaa gagaaagcta cactctttgc    1475
   tctgccgcat tcattctttt cttaccgtga tcctctttgt ctcttatctt ttcttttgtg    1535
   tgtgtgtagc cgttggcgct gtcttcaggg cattccgctc ttaagcgggt gctgacacat    1595
   tgaccatcgc ttcagacttc ctcgttgtac ggatgttgcc atcataatcc caaagagcat    1655
   catggttaaa actgtccata cgcacatttg taaataagaa ttgattcaca catcagaaac    1715
   atggttgtac ttaggggtgc ccaaaaatat ttttgccctt ttttgaataa tgtatgaaag    1775
   acaacttaac tttcaccaaa ataaactaga aagctcagcg tgtttgtctt tattcgctgc    1835
   tacactaact tcgagaccaa cggataagaa agttaacgga ataagagagc ggtaccttta    1895
   ttacctctct ttaaaagaag ttagcagcga tgaatttgtt gctcttttct ctaaggcatt    1955
   caataattta taaggcgtcg ggtatttcag ttactcaatt attcaatgaa acaatgtatc    2015
   ctacatgacg agtactggtc agtcgagatg cgttgttttc ccgacagttc tcattcaggg    2075
   ttctttccga gcgaagactg attgcgtgct gccagactga ttcgttcttg gcgatttggt    2135
   cgaaacgttt gcgcttcctc attcagcgtc cggcgtcagc aatatttgcg cgtaatccc     2194
<210> 5
   <211> 422
   <212> PRT
   <213> Dermacentor variabilis
<400> 5
   Met Ile Pro Ala Ser Val Ala Leu Gly Arg Arg Met Cys Ser Leu Leu
    1               5                  10                  15
   Leu Ala Val Gly Cys Ala Thr Thr Ser Ala Trp Phe Ala Gln Ala Val
               20                  25                  30
   Asp His Ile Asp Lys Gly Tyr Pro Ala Pro Gly Leu Phe Asp Asp Val
           35                  40                  45
   Asp Leu Gln Ile Leu Asp Asn Ile Leu Trp Ser Tyr Asp Arg Arg Ile
       50                  55                  60
   Thr Pro Gly His His Leu Asn Val Pro Thr Val Val Lys Cys Glu Ile
   65                  70                  75                  80
   Tyr Leu Arg Ser Phe Gly Ala Val Asn Pro Ala Thr Met Asp Tyr Asp
                   85                  90                  95
   Val Asp Leu Tyr Leu Arg Gln Thr Trp Thr Asp Leu Arg Met Lys Asn
               100                 105                 110
   Ala Asn Leu Thr Arg Ser Leu Asp Leu Asn Asp Pro Asn Leu Leu Lys
           115                 120                 125
   Lys Val Trp Lys Pro Asp Val Tyr Phe Pro Asn Ala Lys His Gly Glu
       130                 135                 140
   Phe Gln Phe Val Thr Val Pro Asn Val Leu Leu Arg Ile Tyr Pro Thr
   145                 150                 155                 160
   Gly Asp Ile Leu Tyr Met Leu Arg Leu Lys Leu Thr Phe Ser Cys Met
                   165                 170                 175
   Met Asn Met Glu Arg Tyr Pro Leu Asp Arg Gln Val Cys Ser Ile Glu
               180                 185                 190
   Leu Ala Ser Phe Ser Lys Thr Thr Lys Glu Val Glu Leu Gln Trp Gly
           195                 200                 205
   Asn Ala Glu Ala Val Thr Met Tyr Ser Gly Leu Lys Met Ala Gln Phe
       210                 215                 220
   Glu Leu Gln Gln Ile Ser Leu Thr Lys Cys Ser Gly Ala Phe Gln Ile
   225                 230                 235                 240
   Gly Glu Tyr Ser Cys Leu Arg Ala Glu Leu Asn Leu Lys Arg Ser Ile
                   245                 250                 255
   Gly His His Leu Val Gln Ser Tyr Leu Pro Ser Thr Leu Ile Val Val
               260                 265                 270
   Val Ser Trp Val Ser Phe Trp Leu Asp Val Asp Ala Ile Pro Ala Arg
           275                 280                 285
   Ile Thr Leu Gly Val Thr Thr Leu Leu Thr Ile Ser Ser Glu Ser Ser
       290                 295                 300
   Asp His Gln Ala Asn Leu Ala Pro Val Ser Tyr Val Lys Ala Leu Asp
   305                 310                 315                 320
   Val Trp Met Gly Thr Cys Thr Met Phe Val Phe Ala Ala Val Leu Glu
                   325                 330                 335
   Phe Thr Phe Val Ser Tyr Leu Ala Arg Arg Lys Gln Ile Val Pro Ala
               340                 345                 350
   Ser Ile Ala Asp Val Glu Ala Ser Gln Asp Leu Val Leu Val Val Gly
           355                 360                 365
   Asn Lys Asp Lys Asn Arg Pro Pro Ser Pro Ser Ile Pro Thr Ser Thr
       370                 375                 380
   His Val Val Leu Ala Tyr Arg His Arg Ala Lys Gln Ile Asp Gln Val
   385                 390                 395                 400
   Ser Arg Val Ala Phe Pro Ile Gly Phe Val Leu Phe Asn Ala Leu Tyr
                   405                 410                 415
   Trp Pro Tyr Tyr Leu Leu
               420
<210> 6
   <211> 4077
   <212> DNA
   <213> Dermacentor variabilis
<220>
   <221> CDS
   <222> (360)...(1331)
<400> 6
   cggaccggtc ggcccacttt ctcctttcat gacgcgccgt gatcacgcgg cgtgacaccc      60
   agcgtcgcct ctacgtttca ttcatttcgt gtctccgcct gcggtgcgcc tgccgcgtga     120
   cgcaaccggg cgcatgacac cgccgaaccc tctgtcgtcg gcgcatcgcg tcctggcgct     180
   gctcctgctg gtgacagtgc cggcttctct ggggcagagg agacatggaa ctgtcggcga     240
   tttggacaag ttggacaaac tcctgagcaa atatgacaga agggcgttgc caacggggca     300
   catgagatta cgaagtggac ctctacctgc gacaacgatg gcatgatgac cgctttgag      359
   atg agc ggc att agt gga ccc ctc gac ctg aac gat ccc aaa ctg gtg       407
   Met Ser Gly Ile Ser Gly Pro Leu Asp Leu Asn Asp Pro Lys Leu Val
    1               5                  10                  15
   caa cgt ata tgg aaa ccc gaa gtc ttt ttt gcc aac gca aag cat gcg       455
   Gln Arg Ile Trp Lys Pro Glu Val Phe Phe Ala Asn Ala Lys His Ala
               20                  25                  30
   gag ttc cag tac gtg acg gtg ccc aac gtc cta gta cgc atc agt cct       503
   Glu Phe Gln Tyr Val Thr Val Pro Asn Val Leu Val Arg Ile Ser Pro
           35                  40                  45
   acg ggg gac att ctc tac atg ctc agg ttg aag ctg act ttt tct tgc       551
   Thr Gly Asp Ile Leu Tyr Met Leu Arg Leu Lys Leu Thr Phe Ser Cys
       50                  55                  60
   atg atg gac ctt tac cgg tac ccc cta gac gct caa gtt tgc agc att       599
   Met Met Asp Leu Tyr Arg Tyr Pro Leu Asp Ala Gln Val Cys Ser Ile
   65                  70                  75                  80
   gaa ctc gct tcg ttc tcg aag acg acg gac gag cta cag ctg cac tgg      647
   Glu Leu Ala Ser Phe Ser Lys Thr Thr Asp Glu Leu Gln Leu His Trp
                    85                  90                  95
   tct aag gca tcg cct gtg atc ctc tat gaa aac atg aag ctc cca caa      695
   Ser Lys Ala Ser Pro Val Ile Leu Tyr Glu Asn Met Lys Leu Pro Gln
               100                 105                 110
   ttt gaa att caa aac gtg aac acg tcc ctg tgc aat gag aca ttc cac      743
   Phe Glu Ile Gln Asn Val Asn Thr Ser Leu Cys Asn Glu Thr Phe His
           115                 120                 125
   att gga gag tac agc tgc ctg aaa gcc gag ttc aac cta cag cgc tct      791
   Ile Gly Glu Tyr Ser Cys Leu Lys Ala Glu Phe Asn Leu Gln Arg Ser
       130                 135                 140
   att ggc tac cac ctc gtc caa tcg tat ctg ccc acc atc ttg atc gtg      839
   Ile Gly Tyr His Leu Val Gln Ser Tyr Leu Pro Thr Ile Leu Ile Val
   145                 150                 155                 160
   gtc atc tct tgg gtc tcc ttc tgg ctc gac gtg gaa gcg att cca gcc      887
   Val Ile Ser Trp Val Ser Phe Trp Leu Asp Val Glu Ala Ile Pro Ala
                   165                 170                 175
   cga att aca ttg gga gtc acc acg ctt ctt acc atc tca tcc aag ggt      935
   Arg Ile Thr Leu Gly Val Thr Thr Leu Leu Thr Ile Ser Ser Lys Gly
               180                 185                 190
   gcc ggt ata caa gga aac ctg ccg ccc gtc tcg tac gtc aag gca atc      983
   Ala Gly Ile Gln Gly Asn Leu Pro Pro Val Ser Tyr Val Lys Ala Ile
           195                 200                 205
   gac gtc tgg atg ggc gcc tgc acc atg ttc gtg ttt gcc gca ctg ctt     1031
   Asp Val Trp Met Gly Ala Cys Thr Met Phe Val Phe Ala Ala Leu Leu
       210                 215                 220
   gag ttc acc ttt gtc aac tac ctg tgg agg aag cgg ccc gcg act gcc     1079
   Glu Phe Thr Phe Val Asn Tyr Leu Trp Arg Lys Arg Pro Ala Thr Ala
   225                 230                 235                 240
   aag tca cca cct ccg gtg gtc gca gcc att ccc gag agc aaa gtg gct     1127
   Lys Ser Pro Pro Pro Val Val Ala Ala Ile Pro Glu Ser Lys Val Ala
                   245                 250                 255
   gtg ctc ctc cca tgc aac gga aac ttg ggg cca tgc agc ccc atc act     1175
   Val Leu Leu Pro Cys Asn Gly Asn Leu Gly Pro Cys Ser Pro Ile Thr
               260                 265                 270
   ggc ggt aca gac atc agc cct tcg ccc aca ggt cct gaa gct gtc aga     1223
   Gly Gly Thr Asp Ile Ser Pro Ser Pro Thr Gly Pro Glu Ala Val Arg
           275                 280                 285
   aac aga cac aag gtt cag gcc aag aga att gac cag acc tgc agg ata     1271
   Asn Arg His Lys Val Gln Ala Lys Arg Ile Asp Gln Thr Cys Arg Ile
       290                 295                 300
   gca ttt ccc atg gct ttc ctg gcg ttt agc gtc gca tac tgg cca tac     1319
   Ala Phe Pro Met Ala Phe Leu Ala Phe Ser Val Ala Tyr Trp Pro Tyr
   305                 310                 315                 320
   tat ctt ttg tga ggccgcggta ccccgagcta atgtcaggaa cggagaggcg        1371
   Tyr Leu Leu  *
   ggtaccacga agtcgggggg gggggggagg ggggagagtg cttgtggcta tcacaatccc   1431
   gttggttctc tgtaagaacg cttttgtttt gcacagaagc tcactgcatc acattttgcg   1491
   tctccctagt gtttaattat ttgtttctgc acttgtgttc ccgtgtgcat tctgactgaa   1551
   tatcactcca acccttcagt gtgtataagt cccaaagtga attggatatt tcctcttcgc   1611
   gatcctcttg agggcacctc tagtcactaa tctaacacgt aggagagttt aaggatgcgt   1671
   taggcagcac ttttcttgtg ctttaagtgg atctcatcat attctggtag agaatataaa   1731
   cttcaacact gaagtagtat ttacaaggca gactaacatg ttgctagaaa cagtattttt   1791
   gcaggaggga agatgcaatg attatacagg gtgttcaaaa ttaagcttta tggttttata   1851
   ggaattaggc actgcgaggg gaagggcaac cgttatcgtc tttgtctatg cctccgccct   1911
   attgtcagac taaatgccgc acacaacagc ctcgtcacat cagggaagat ctttgtgcca   1971
   atcctcactc tcttgcgtgc gtaatcacgt aaacgacaat taaaatttgg agccagctat   2031
   ctcgaagcaa agatatgctg gaagaattct tctaagtgta actgtgtaga aacttttcaa   2091
   tacacaaata cacacttact gcagtcaata aaaagttaat tactcgattt tatttaattg   2151
   ggctgctgac agcaataact ctcatctcac tttgtgtccc cctggccaca taacttattt   2211
   gcacaggtgg tcttcgcgtg catcccagtg gctaaattta agaaaaccat aaagcttaat   2271
   tttgaacacc tggtatatca tgatgctttc aatgctttat tgttgtatta taaaaaaaga   2331
   tatactatca acgactcagg ccggagaatc atgttggaaa aaaaatgttt cattgtttcc   2391
   tttcgtcatc gcgcccttag gttaatttgc cctgtacagt tcctgaggga acgcattagt   2451
   gcacaaaaaa agtatttcgg cttccacatc gcaacgaaaa cgggcgtcgc ctcctgtctc   2511
   tacaagacaa tgagatgcgc aggccgcacg ctttttcggg gtccgcaatt attaaacatg   2571
   gcgtatattt tgataacccg caccttcttc ctacgcagca tttttctgtt agacccactg   2631
   ggttcattta accaatccta ggcctaaaac cgtattcaag cccagcacaa agtccgcttt   2691
   tgcgaactcc cgttcagatg tggatgagcc gttggcttac aggactctga cctaagtatg   2751
   ggcctgtgtc aaacggcgtc agaaagatga gcacaacagc cccttattgc gtaacgctgc   2811
   cggcaatgct cgccatttta agctgtcccg aactgcgaaa ttattccacg gtagcgcttt   2871
   tgtagatgtg gaagacttgc ctaatcactt caaaggtgtc gccacttaca atactatacg   2931
   tacagttccg cctggagaat ttggcgcacg catacttgta gtaccatgag gcggagttat   2991
   tacttcggga ggaattgcgc aggcagctaa tccccatcta cgcaactctg gacagtcgga   3051
   tgttatgcat ggtaggagaa tggactatag aagggtggag tctgcaagtc aggcgaggat   3111
   acagcggcgt agcgaaaacg tagccatgct tgtggagtac acgacccgac tcttgtgaaa   3171
   cacggatcca tctatgtcgg aaacaaaaat ttaagcactt catgcgcgca gtaaagaaag   3231
   aaccctttgg gggcctgata ccaaacttgc ccaagaacct cccagagtac ctcgcagagg   3291
   ccatgtcaaa ggaaaagacg atctagcagt aggatcctga tttggctttg gacaacgtcg   3351
   ctgtaatgcg agtgcttata aagttctttg ttctggaaga ggttaaatgc tccatctaac   3411
   tccaggctct gtactgcgga cttcgccggc tgaggtcgtt cgttagaaga tggggcgtgc   3471
   tgcccgaacc tcagaatatt tcggagcgcc actgtacgag gtgcggcagc tggcactttg   3531
   aatcacctat gcggaagctg cgcgaggttc tccacactag gactcccaca atgtgcgcgc   3591
   ccttgaacaa gcgattgcca acttcagagc ccgcggcgac caatcaaagc tgaagtatgt   3651
   catcgcaaaa cttatattta tcgaacctca attggaaaga ccatgtattt tcactgcgct   3711
   gtggaacatg aaatttatgc gttacatatt cgctccgggg aatagcaaaa atattgcaaa   3771
   aatattggtg acacagaaag cagtcgcata tcaagcccat tatatgcgtt gacgctgtag   3831
   tttgtaaagg gcacttgaat gtggacgcct gtttagaatc gcggagagat ttcattttcg   3891
   cggagcttat accactctca aatgtgctgg ggcacggcag aatcgtggat ccagtttttt   3951
   taacttccgt caaaacagat tagcagtagt tcacagcggc gaaacactca caagtgtagt   4011
   tataaaaacc taacagtttg aatcaataaa tatttgacat caaaaaaaaa aaaaaaaaaa   4071
   aaaaaa                                                               4077
<210> 7
   <211> 323
   <212> PRT
   <213> Dermacentor variabilis
<400> 7
   Met Ser Gly Ile Ser Gly Pro Leu Asp Leu Asn Asp Pro Lys Leu Val
    1               5                  10                  15
   Gln Arg Ile Trp Lys Pro Glu Val Phe Phe Ala Asn Ala Lys His Ala
               20                  25                  30
   Glu Phe Gln Tyr Val Thr Val Pro Asn Val Leu Val Arg Ile Ser Pro
           35                  40                  45
   Thr Gly Asp Ile Leu Tyr Met Leu Arg Leu Lys Leu Thr Phe Ser Cys
       50                  55                  60
   Met Met Asp Leu Tyr Arg Tyr Pro Leu Asp Ala Gln Val Cys Ser Ile
   65                  70                  75                  80
   Glu Leu Ala Ser Phe Ser Lys Thr Thr Asp Glu Leu Gln Leu His Trp
                   85                  90                  95
   Ser Lys Ala Ser Pro Val Ile Leu Tyr Glu Asn Met Lys Leu Pro Gln
               100                 105                 110
   Phe Glu Ile Gln Asn Val Asn Thr Ser Leu Cys Asn Glu Thr Phe His
           115                 120                 125
   Ile Gly Glu Tyr Ser Cys Leu Lys Ala Glu Phe Asn Leu Gln Arg Ser
       130                 135                 140
   Ile Gly Tyr His Leu Val Gln Ser Tyr Leu Pro Thr Ile Leu Ile Val
   145                 150                 155                 160
   Val Ile Ser Trp Val Ser Phe Trp Leu Asp Val Glu Ala Ile Pro Ala
                   165                 170                 175
   Arg Ile Thr Leu Gly Val Thr Thr Leu Leu Thr Ile Ser Ser Lys Gly
               180                 185                 190
   Ala Gly Ile Gln Gly Asn Leu Pro Pro Val Ser Tyr Val Lys Ala Ile
           195                 200                 205
   Asp Val Trp Met Gly Ala Cys Thr Met Phe Val Phe Ala Ala Leu Leu
       210                 215                 220
   Glu Phe Thr Phe Val Asn Tyr Leu Trp Arg Lys Arg Pro Ala Thr Ala
   225                 230                 235                 240
   Lys Ser Pro Pro Pro Val Val Ala Ala Ile Pro Glu Ser Lys Val Ala
                   245                 250                 255
   Val Leu Leu Pro Cys Asn Gly Asn Leu Gly Pro Cys Ser Pro Ile Thr
               260                 265                 270
   Gly Gly Thr Asp Ile Ser Pro Ser Pro Thr Gly Pro Glu Ala Val Arg
           275                 280                 285
   Asn Arg His Lys Val Gln Ala Lys Arg Ile Asp Gln Thr Cys Arg Ile
       290                 295                 300
   Ala Phe Pro Met Ala Phe Leu Ala Phe Ser Val Ala Tyr Trp Pro Tyr
   305                 310                 315                 320
   Tyr Leu Leu
<210> 8
   <211> 18
   <212> DNA
   <213> Artificial Sequence
<220>
   <223> oligonucleotide
<400> 8
   cggatattgg acagcatc 18
<210> 9
   <211> 22
   <212> DNA
   <213> Artificial Sequence
<220>
   <223> oligonucleotide
<400> 9
   ccagtagacg aggttgaaga gg 22
<210> 10
   <211> 1197
   <212> DNA
   <213> Rhipicephalus sanguineus
<400> 10
   cggatattgg acagcatcat tggccagggt cgttatgact gcaggatccg gcccatggga      60
   attaacaaca cagacgggcc ggctcttgta cgcgttaaca tctttgtaag aagtatcggc     120
   agaattgatg acgtcaccat ggagtacaca gtgcaaatga cgttcagaga gcagtggcgg     180
   gacgagagac tccagtacga cgacttgggc ggccaggttc gctacctgac gctcaccgaa     240
   ccggacaagc tttggaagcc ggacctgttt ttctccaacg agaaagaggg acacttccac     300
   aacatcatca tgcccaacgt gcttctacgc atacatccca acggcgacgt tctcttcagc     360
   atcagaatat ccttggtgct ttcatgtccg atgaacctga aattttatcc tttggataaa     420
   caaatctgct ctatcgtcat ggtgagctat gggtatacaa cagaggacct ggtgtttcta     480
   tggaaagagg gggatcctgt acaggtcaca aaaaatctcc acttgccacg tttcacgctg     540
   gaaaggtttc aaaccgacta ctgcaccagt cggaccaaca ctggcgagta cagctgcttg     600
   cgcgtggacc tggtgttcaa gcgcgagttc agctactacc tgatccagat ctacatcccg     660
   tgctgcatgc tggtcatcgt gtcctgggtg tcgttctggc tcgaccccac ctcgatcccg     720
   gcgcgagtgt cgctgggcgt caccaccctg ctcaccatgg ccacgcagat atcgggcatc     780
   aacgcctcgc tgcctcccgt ttcctacacc aaggccattg acgtgtggac cggcgtctgt     840
   ctgaccttcg tattcggcgc gctcctcgag ttcgccctgg tcaactacgc ctcgcggtca     900
   gattcacgcc ggcagaacat gcagaagcag aagcagagga aatgggagct cgagccgccc     960
   ctggactcgg accacctgga ggacggcgcc accacgttcg ccatgaggcc gctggtgcac    1020
   caccacggag agctgcatgc cgacaagttg cggcagtgcg aagtccacat gaagaccccc    1080
   aagacgaacc tttgcaaggc ctggctttcc aggtttccca cgcgatccaa acgcatcgac    1140
   gtcgtctcgc ggatcttctt tccgctcatg ttcgccctct tcaacctcgt ctactgg       1197
<210> 11
   <211> 18
   <212> DNA
   <213> Artificial Sequence
<220>
   <223> oligonucleotide
<400> 11
   tgtggtggtg atagctgc 18
<210> 12
   <211> 20
   <212> DNA
   <213> Artificial Sequence
<220>
   <223> oligonucleotide
<400> 12
   gagttgatca atctgcttgg 20
<210> 13
   <211> 1150
   <212> DNA
   <213> Dermacentor variabilis
<400> 13
   tgtggtggtg atagctgcgt tctgctggcc gcccgctctg ccgctcgtac ccgggggagt      60
   ttcctccaga gcaaacgatc tggacattct ggacgagctc ctcaaaaact acgatcgaag     120
   ggccctgccg agcagtcacc tcggaaatgc aactattgtg tcatgcgaaa tttacatacg     180
   aagttttgga tcaataaatc cttcgaacat ggactacgaa gtcgacctct acttccggca     240
   gtcgtggctc gacgagcggt tacgcaaatc cacgctatct cgtccgctcg accttaatga     300
   cccaaagctg gtacaaatga tatggaagcc agaagttttc tttgcgaacg cgaaacacgc     360
   cgagttccaa tatgtgactg tacctaacgt cctcgttagg atcaacccga ctggaataat     420
   cttgtacatg ttgcggttaa aactgaggtt ctcctgcatg atggacctgt accggtaccc     480
   catggattcc caagtctgca gcatcgaaat tgcctctttt tccaaaacca ccgaagagct     540
   gctgctgaaa tggtccgaga gtcagcctgt cgttctcttc gataacctca agttgcccca     600
   gtttgaaata gagaaggtga acacgtcctt atgcaaagaa aagtttcaca taggggaata     660
   cagttgcctg aaagccgact tctatctgca gcgttccctc ggttatcaca tggtgcagac     720
   ctatcttccg accacgctta tcgtggtcat ctcatgggtg tcattctggc tcgacgtaga     780
   cgccataccc gcccgtgtca ccctgggcgt aaccacgctg ctcaccatct catccaaggg     840
   tgccggtatc cagggaaacc tgcctcccgt ctcgtacatc aaggccatgg acgtctggat     900
   aggatcctgt acttcgtttg tctttgcggc ccttctagag ttcacattcg tcaactatct     960
   ctggaggcgg ctgcccaata agcgcccatc ttctgacgta ccggtgacgg atataccaag    1020
   cgacggctca aagcatgaca ttgcggcaca gctcgtactc gacaagaatg gacacaccga    1080
   agttcgcacg ttggtccaag cgatgccacg cagcgtcgga aaagtgaagg ccaagcagat    1140
   tgatcaactc                                                            1150